# EUROPEAN PATENT APPLICATION

(11) **EP 4 174 084 A1**
(43) Date of publication of application: **03.05.2023**
(21) Application number: 21828260.6
(22) Date of filing: 07.05.2021
(51) Int. Cl.: C07K 16/10, A61P 31/14, A61K 39/00

(54) **ANTI-RSV ANTIBODY AND PHARMACEUTICAL COMPOSITION COMPRISING SAME**

(30) Priority: 25.06.2020 KR 20200077914
(71) Applicant: Mogam Institute for Biomedical Research, Yongin-si, Gyeonggi-do 16924 (KR)
(72) Inventor: KIM, Dong Sik, Yongin-si Gyeonggi-do 16924 (KR); JANG, Shin A, Yongin-si Gyeonggi-do 16924 (KR); HAN, Young Woo, Yongin-si Gyeonggi-do 16924 (KR); LEE, Su A, Yongin-si Gyeonggi-do 16924 (KR); KIM, Woo Hyun, Yongin-si Gyeonggi-do 16924 (KR)
(74) Representative: Dehns
(86) International application number: PCT/KR2021/005742
(87) International publication number: WO 2021/261762

(57) **Abstract**

The present invention relates to an anti-respiratory syncytial virus (RSV) antibody and a pharmaceutical composition comprising the same, and particularly, to an anti-RSV antibody specifically binding to an F-protein of RSV, and a pharmaceutical composition for use in preventing or treating an RSV infection. The anti-RSV antibody according to the present invention can effectively prevent RSV infection and has excellent efficacy for alleviating and treating symptoms of RSV infection.

## Description

### [Technical Field]

The present invention relates to an anti-respiratory syncytial virus (RSV) antibody and a pharmaceutical composition comprising the same, and particularly, to an anti-RSV antibody specifically binding to an F-protein of RSV, and a pharmaceutical composition for use in preventing or treating an RSV infection.

### [Background Art]

Respiratory Syncytial Virus (RSV) is a virus that belongs to the paramyxovirus family and causes acute respiratory infections. RSV is highly toxic and easily spreadable, and causes a runny nose, sore throat, cough and sputum as main symptoms, accompanied by nasal congestion, hoarseness, wheezing, and vomiting. RSV infections show mild symptoms (mainly upper respiratory tract infection), like a cold, in adults, but they can cause lower respiratory tract diseases such as bronchiolitis and pneumonia in infants of one year old or younger and often cause pneumonia in newborns.

Currently, no RSV prevention vaccines or specific antiviral agents have been developed, and except special cases, a method of alleviating infectious symptoms has been used as a treatment method. However, for a high-risk group including premature infants with a lung or heart disease, a product, named Synagis, for injecting mass-produced anti-RSV antibody into the body is commonly used, but has disadvantages of monthly administration during the RSV epidemic period since the half-life of an antibody is about one month, and its high cost.

RSV includes two major surface glycoproteins, the G-protein and F-protein. While the F-protein facilitated the fusion of the virus and the cell membrane, the G-protein mediates the binding of the virus to a cell receptor and allows the penetration of a ribonucleoprotein of the virus into the cell cytoplasm. Particularly, the F-protein has several sites involved in infection.

It was demonstrated that antibodies produced against the RSV F or G-protein neutralize RSV with high efficiency *in vitro* and have a preventive effect *in vivo* (refer to Walsh et al., (1986) J. Gen. Microbiol. 67:505; Beeler et al., (1989) J. Virol. 63:2941-2950, Garcia-Borenno et al., (1989) J. Virol. 63:925-932, Taylor et al., (1984) Immunology 52:137-142). Antibodies against the RSV F-protein are also effective in inhibiting the fusion of RSV-infected cells and neighboring uninfected cells.

However, to prevent RSV infections, it is essential to simultaneously block all sites and proteins related to the infections. When all sites are not completely blocked, cell infections may occur through unblocked sites.

Accordingly, the present inventors have endeavored to develop anti-RSV antibodies for effectively preventing and treating an RSV infection and thus developed a novel anti-RSV antibody having excellent efficacy.

### [Disclosure of Invention]

### [Technical Problem]

The present invention is directed to providing an antibody or an antigen-binding fragment thereof specifically binding to respiratory syncytial virus (RSV) selected from the group consisting of the following (i) to (vii):
(i) an antibody specifically binding to RSV, comprising a heavy chain variable domain which comprises heavy chain CDR1 comprising the amino acid sequence of SEQ ID NO: 1, heavy chain CDR2 comprising the amino acid sequence of SEQ ID NO: 2, and heavy chain CDR3 comprising the amino acid sequence of SEQ ID NO: 3, and a light chain variable domain which comprises light chain CDR1 comprising the amino acid sequence of SEQ ID NO: 4, light chain CDR2 comprising the amino acid sequence of SEQ ID NO: 5, and light chain CDR3 comprising the amino acid sequence of SEQ ID NO: 6;
(ii) an antibody specifically binding to RSV, comprising a heavy chain variable domain which comprises heavy chain CDR1 comprising the amino acid sequence of SEQ ID NO: 1, heavy chain CDR2 comprising the amino acid sequence of SEQ ID NO: 2, and heavy chain CDR3 comprising the amino acid sequence of SEQ ID NO: 3, and a light chain variable domain which comprises light chain CDR1 comprising the amino acid sequence of SEQ ID NO: 7, light chain CDR2 comprising the amino acid sequence of SEQ ID NO: 5, and light chain CDR3 comprising the amino acid sequence of SEQ ID NO: 6;
(iii) an antibody specifically binding to RSV, comprising a heavy chain variable domain which comprises heavy chain CDR1 comprising the amino acid sequence of SEQ ID NO: 8, heavy chain CDR2 comprising the amino acid sequence of SEQ ID NO: 9, and heavy chain CDR3 comprising the amino acid sequence of SEQ ID NO: 10, and a light chain variable domain which comprises light chain CDR1 comprising the amino acid sequence of SEQ ID NO: 7, light chain CDR2 comprising the amino acid sequence of SEQ ID NO: 5, and light chain CDR3 comprising the amino acid sequence of SEQ ID NO: 6;
(iv) an antibody specifically binding to RSV, comprising a heavy chain variable domain which comprises heavy chain CDR1 comprising the amino acid sequence of SEQ ID NO: 11, heavy chain CDR2 comprising the amino acid sequence of SEQ ID NO: 12, and heavy chain CDR3 comprising the amino acid sequence of SEQ ID NO: 3, and a light chain variable domain which comprises light chain CDR1 comprising the amino acid sequence of SEQ ID NO: 13, light chain CDR2 comprising the amino acid sequence of SEQ ID NO: 5, and light chain CDR3 comprising the amino acid sequence of SEQ ID NO: 6;
(v) an antibody specifically binding to RSV, comprising a heavy chain variable domain which comprises heavy chain CDR1 comprising the amino acid sequence of SEQ ID NO: 1, heavy chain CDR2 comprising the amino acid sequence of SEQ ID NO: 2, and heavy chain CDR3 comprising the amino acid sequence of SEQ ID NO: 3, and a light chain variable domain which comprises light chain CDR1 comprising the amino acid sequence of SEQ ID NO: 14, light chain CDR2 comprising the amino acid sequence of SEQ ID NO: 15, and light chain CDR3 comprising the amino acid sequence of SEQ ID NO: 6;
(vi) an antibody specifically binding to RSV, comprising a heavy chain variable domain which comprises heavy chain CDR1 comprising the amino acid sequence of SEQ ID NO: 8, heavy chain CDR2 comprising the amino acid sequence of SEQ ID NO: 9, and heavy chain CDR3 comprising the amino acid sequence of SEQ ID NO: 3, and a light chain variable domain which comprises light chain CDR1 comprising the amino acid sequence of SEQ ID NO: 14, light chain CDR2 comprising the amino acid sequence of SEQ ID NO: 5, and light chain CDR3 comprising the amino acid sequence of SEQ ID NO: 6; and
(vii) an antibody specifically binding to RSV, comprising a heavy chain variable domain which comprises heavy chain CDR1 comprising the amino acid sequence of SEQ ID NO: 1, heavy chain CDR2 comprising the amino acid sequence of SEQ ID NO: 16, and heavy chain CDR3 comprising the amino acid sequence of SEQ ID NO: 17, and a light chain variable domain which comprises light chain CDR1 comprising the amino acid sequence of SEQ ID NO: 14, light chain CDR2 comprising the amino acid sequence of SEQ ID NO: 18, and light chain CDR3 comprising the amino acid sequence of SEQ ID NO: 6.

The present invention is also directed to providing an antibody or an antigen-binding fragment thereof specifically binding to RSV, selected from the group consisting of the following (viii) to (xv):
(viii) an antibody specifically binding to RSV, comprising a heavy chain variable domain comprising the amino acid sequence of SEQ ID NO: 19 and a light chain variable domain comprising the amino acid sequence of SEQ ID NO: 20;
(ix) an antibody specifically binding to RSV, comprising a heavy chain variable domain comprising the amino acid sequence of SEQ ID NO: 21 and a light chain variable domain comprising the amino acid sequence of SEQ ID NO: 22;
(x) an antibody specifically binding to RSV, comprising a heavy chain variable domain comprising the amino acid sequence of SEQ ID NO: 23 and a light chain variable domain comprising the amino acid sequence of SEQ ID NO: 24;
(xi) an antibody specifically binding to RSV, comprising a heavy chain variable domain comprising the amino acid sequence of SEQ ID NO: 25 and a light chain variable domain comprising the amino acid sequence of SEQ ID NO: 26;
(xii) an antibody specifically binding to RSV, comprising a heavy chain variable domain comprising the amino acid sequence of SEQ ID NO: 27 and a light chain variable domain comprising the amino acid sequence of SEQ ID NO: 28;
(xiii) an antibody specifically binding to RSV, comprising a heavy chain variable domain comprising the amino acid sequence of SEQ ID NO: 29 and a light chain variable domain comprising the amino acid sequence of SEQ ID NO: 30;
(xiv) an antibody specifically binding to RSV, comprising a heavy chain variable domain comprising the amino acid sequence of SEQ ID NO: 31 and a light chain variable domain comprising the amino acid sequence of SEQ ID NO: 24; and
(xv) an antibody specifically binding to RSV, comprising a heavy chain variable domain comprising the amino acid sequence of SEQ ID NO: 32 and a light chain variable domain comprising the amino acid sequence of SEQ ID NO: 33.

The present invention is also directed to providing an antibody or an antigen-binding fragment thereof specifically binding to RSV, selected from the group consisting of the following (xvi) to (xxiii):
(xvi) an antibody specifically binding to RSV, comprising a heavy chain variable domain encoded by the nucleotide sequence of SEQ ID NO: 34 and a light chain variable domain encoded by the nucleotide sequence of SEQ ID NO: 35;
(xvii) an antibody specifically binding to RSV, comprising a heavy chain variable domain encoded by the nucleotide sequence of SEQ ID NO: 36 and a light chain variable domain encoded by the nucleotide sequence of SEQ ID NO: 37;
(xviii) an antibody specifically binding to RSV, comprising a heavy chain variable domain encoded by the nucleotide sequence of SEQ ID NO: 38 and a light chain variable domain encoded by the nucleotide sequence of SEQ ID NO: 39;
(xix) an antibody specifically binding to RSV, comprising a heavy chain variable domain encoded by the nucleotide sequence of SEQ ID NO: 40 and a light chain variable domain encoded by the nucleotide sequence of SEQ ID NO: 41;
(xx) an antibody specifically binding to RSV, comprising a heavy chain variable domain encoded by the nucleotide sequence of SEQ ID NO: 42 and a light chain variable domain encoded by the nucleotide sequence of SEQ ID NO: 43;
(xxi) an antibody specifically binding to RSV, comprising a heavy chain variable domain encoded by the nucleotide sequence of SEQ ID NO: 44 and a light chain variable domain encoded by the nucleotide sequence of SEQ ID NO: 45;
(xxii) an antibody specifically binding to RSV, comprising a heavy chain variable domain encoded by the nucleotide sequence of SEQ ID NO: 46 and a light chain variable domain encoded by the nucleotide sequence of SEQ ID NO: 39; and
(xxiii) an antibody specifically binding to RSV, comprising a heavy chain variable domain encoded by the nucleotide sequence of SEQ ID NO: 47 and a light chain variable domain encoded by the nucleotide sequence of SEQ ID NO: 48.

The present invention is also directed to providing a polynucleotide encoding a light chain variable domain and a light chain variable domain of the aforementioned antibody.

The present invention is also directed to providing an expression vector comprising the polynucleotide.

The present invention is also directed to providing a host cell transformed with the expression vector.

The present invention is also directed to providing a method of preparing an antibody or an antigen-binding fragment thereof specifically binding to respiratory syncytial virus (RSV), comprising the step of culturing the host cell.

The present invention is also directed to providing a pharmaceutical composition for use in preventing or treating respiratory syncytial virus (RSV) infection, comprising the aforementioned antibody or antigen-binding fragment thereof.

### [Technical Solution]

To achieve the above-described purposes, the present invention provides an antibody or an antigen-binding fragment thereof specifically binding to respiratory syncytial virus (RSV) selected from the group consisting of the following (i) to (vii):
(i) an antibody specifically binding to RSV, comprising a heavy chain variable domain which comprises heavy chain CDR1 comprising the amino acid sequence of SEQ ID NO: 1, heavy chain CDR2 comprising the amino acid sequence of SEQ ID NO: 2, and heavy chain CDR3 comprising the amino acid sequence of SEQ ID NO: 3, and a light chain variable domain which comprises light chain CDR1 comprising the amino acid sequence of SEQ ID NO: 4, light chain CDR2 comprising the amino acid sequence of SEQ ID NO: 5, and light chain CDR3 comprising the amino acid sequence of SEQ ID NO: 6;
(ii) an antibody specifically binding to RSV, comprising a heavy chain variable domain which comprises heavy chain CDR1 comprising the amino acid sequence of SEQ ID NO: 1, heavy chain CDR2 comprising the amino acid sequence of SEQ ID NO: 2, and heavy chain CDR3 comprising the amino acid sequence of SEQ ID NO: 3, and a light chain variable domain which comprises light chain CDR1 comprising the amino acid sequence of SEQ ID NO: 7, light chain CDR2 comprising the amino acid sequence of SEQ ID NO: 5, and light chain CDR3 comprising the amino acid sequence of SEQ ID NO: 6;
(iii) an antibody specifically binding to RSV, comprising a heavy chain variable domain which comprises heavy chain CDR1 comprising the amino acid sequence of SEQ ID NO: 8, heavy chain CDR2 comprising the amino acid sequence of SEQ ID NO: 9, and heavy chain CDR3 comprising the amino acid sequence of SEQ ID NO: 10, and a light chain variable domain which comprises light chain CDR1 comprising the amino acid sequence of SEQ ID NO: 7, light chain CDR2 comprising the amino acid sequence of SEQ ID NO: 5, and light chain CDR3 comprising the amino acid sequence of SEQ ID NO: 6;
(iv) an antibody specifically binding to RSV, comprising a heavy chain variable domain which comprises heavy chain CDR1 comprising the amino acid sequence of SEQ ID NO: 11, heavy chain CDR2 comprising the amino acid sequence of SEQ ID NO: 12, and heavy chain CDR3 comprising the amino acid sequence of SEQ ID NO: 3, and a light chain variable domain which comprises light chain CDR1 comprising the amino acid sequence of SEQ ID NO: 13, light chain CDR2 comprising the amino acid sequence of SEQ ID NO: 5, and light chain CDR3 comprising the amino acid sequence of SEQ ID NO: 6;
(v) an antibody specifically binding to RSV, comprising a heavy chain variable domain which comprises heavy chain CDR1 comprising the amino acid sequence of SEQ ID NO: 1, heavy chain CDR2 comprising the amino acid sequence of SEQ ID NO: 2, and heavy chain CDR3 comprising the amino acid sequence of SEQ ID NO: 3, and a light chain variable domain which comprises light chain CDR1 comprising the amino acid sequence of SEQ ID NO: 14, light chain CDR2 comprising the amino acid sequence of SEQ ID NO: 15, and light chain CDR3 comprising the amino acid sequence of SEQ ID NO: 6;
(vi) an antibody specifically binding to RSV, comprising a heavy chain variable domain which comprises heavy chain CDR1 comprising the amino acid sequence of SEQ ID NO: 8, heavy chain CDR2 comprising the amino acid sequence of SEQ ID NO: 9, and heavy chain CDR3 comprising the amino acid sequence of SEQ ID NO: 3, and a light chain variable domain which comprises light chain CDR1 comprising the amino acid sequence of SEQ ID NO: 14, light chain CDR2 comprising the amino acid sequence of SEQ ID NO: 5, and light chain CDR3 comprising the amino acid sequence of SEQ ID NO: 6; and
(vii) an antibody specifically binding to RSV, comprising a heavy chain variable domain which comprises heavy chain CDR1 comprising the amino acid sequence of SEQ ID NO: 1, heavy chain CDR2 comprising the amino acid sequence of SEQ ID NO: 16, and heavy chain CDR3 comprising the amino acid sequence of SEQ ID NO: 17, and a light chain variable domain which comprises light chain CDR1 comprising the amino acid sequence of SEQ ID NO: 14, light chain CDR2 comprising the amino acid sequence of SEQ ID NO: 18, and light chain CDR3 comprising the amino acid sequence of SEQ ID NO: 6.

The present invention also provides an antibody or an antigen-binding fragment thereof specifically binding to RSV, selected from the group consisting of the following (viii) to (xv):
(viii) an antibody specifically binding to RSV, comprising a heavy chain variable domain comprising the amino acid sequence of SEQ ID NO: 19 and a light chain variable domain comprising the amino acid sequence of SEQ ID NO: 20;
(ix) an antibody specifically binding to RSV, comprising a heavy chain variable domain comprising the amino acid sequence of SEQ ID NO: 21 and a light chain variable domain comprising the amino acid sequence of SEQ ID NO: 22;
(x) an antibody specifically binding to RSV, comprising a heavy chain variable domain comprising the amino acid sequence of SEQ ID NO: 23 and a light chain variable domain comprising the amino acid sequence of SEQ ID NO: 24;
(xi) an antibody specifically binding to RSV, comprising a heavy chain variable domain comprising the amino acid sequence of SEQ ID NO: 25 and a light chain variable domain comprising the amino acid sequence of SEQ ID NO: 26;
(xii) an antibody specifically binding to RSV, comprising a heavy chain variable domain comprising the amino acid sequence of SEQ ID NO: 27 and a light chain variable domain comprising the amino acid sequence of SEQ ID NO: 28;
(xiii) an antibody specifically binding to RSV, comprising a heavy chain variable domain comprising the amino acid sequence of SEQ ID NO: 29 and a light chain variable domain comprising the amino acid sequence of SEQ ID NO: 30;
(xiv) an antibody specifically binding to RSV, comprising a heavy chain variable domain comprising the amino acid sequence of SEQ ID NO: 31 and a light chain variable domain comprising the amino acid sequence of SEQ ID NO: 24; and
(xv) an antibody specifically binding to RSV, comprising a heavy chain variable domain comprising the amino acid sequence of SEQ ID NO: 32 and a light chain variable domain comprising the amino acid sequence of SEQ ID NO: 33.

The present invention also provides an antibody or an antigen-binding fragment thereof specifically binding to RSV, selected from the group consisting of the following (xvi) to (xxiii):
(xvi) an antibody specifically binding to RSV, comprising a heavy chain variable domain encoded by the nucleotide sequence of SEQ ID NO: 34 and a light chain variable domain encoded by the nucleotide sequence of SEQ ID NO: 35;
(xvii) an antibody specifically binding to RSV, comprising a heavy chain variable domain encoded by the nucleotide sequence of SEQ ID NO: 36 and a light chain variable domain encoded by the nucleotide sequence of SEQ ID NO: 37;
(xviii) an antibody specifically binding to RSV, comprising a heavy chain variable domain encoded by the nucleotide sequence of SEQ ID NO: 38 and a light chain variable domain encoded by the nucleotide sequence of SEQ ID NO: 39;
(xix) an antibody specifically binding to RSV, comprising a heavy chain variable domain encoded by the nucleotide sequence of SEQ ID NO: 40 and a light chain variable domain encoded by the nucleotide sequence of SEQ ID NO: 41;
(xx) an antibody specifically binding to RSV, comprising a heavy chain variable domain encoded by the nucleotide sequence of SEQ ID NO: 42 and a light chain variable domain encoded by the nucleotide sequence of SEQ ID NO: 43;
(xxi) an antibody specifically binding to RSV, comprising a heavy chain variable domain encoded by the nucleotide sequence of SEQ ID NO: 44 and a light chain variable domain encoded by the nucleotide sequence of SEQ ID NO: 45;
(xxii) an antibody specifically binding to RSV, comprising a heavy chain variable domain encoded by the nucleotide sequence of SEQ ID NO: 46 and a light chain variable domain encoded by the nucleotide sequence of SEQ ID NO: 39; and
(xxiii) an antibody specifically binding to RSV, comprising a heavy chain variable domain encoded by the nucleotide sequence of SEQ ID NO: 47 and a light chain variable domain encoded by the nucleotide sequence of SEQ ID NO: 48.

The present invention also provides a polynucleotide encoding a light chain variable domain and a light chain variable domain of the aforementioned antibody.

The present invention also provides an expression vector comprising the polynucleotide.

The present invention also provides a host cell transformed with the expression vector.

The present invention also provides a method of preparing an antibody or an antigen-binding fragment thereof specifically binding to respiratory syncytial virus (RSV), comprising the step of culturing the host cell.

The present invention also provides a pharmaceutical composition for use in preventing or treating respiratory syncytial virus (RSV) infection, comprising the aforementioned antibody or antigen-binding fragment thereof.

### [Advantageous Effects]

The anti-RSV antibody according to the present invention can effectively prevent RSV infection and has excellent efficacy for alleviating and treating symptoms of RSV infection.

### [Brief Description of Drawings]

The above and other objects, features and advantages of the present invention will become more apparent to those of ordinary skill in the art by describing in detail exemplary embodiments thereof with reference to the accompanying drawings, in which:
FIG. 1 shows the SDS-PAGE results for DS-Cav1 (lane 1), sc9-10 (A-type; lane 2), DS-Cav1 (B-type), sc9-10 variants (sc9-10 A149C Y458C and sc9-10 N138GC N428C), and post-F-protein;
FIG. 2 is a result of analyzing the expression level of an RSV F-protein;
FIG. 3A shows a B cell isolation process, FIG. 3B shows a ratio of isolated cells with respect to the total cell number, and FIG. 3C shows a process of isolating cells, which are double positive for RSV F;
FIG. 4 is a result of constructing a chromium single sample V(D)J library;
FIG. 5 shows the results of VH/Vκ/V_{λ}, PCR and scFv overlapping PCR;
FIGS. 6 to 10 show test results for the neutralizing ability of selected RSV antibodies;
FIG. 11 shows the RSV neutralizing antibody IC₅₀ result for an MG9112A-screening antibody; and
FIGS. 12 to 17 show the results of measuring the affinity for a recombinant F-protein with respect to a selected anti-F antibody.

### [Best Mode for Carrying Out the Invention]

The term "antibody" used herein refers to an immunoglobulin and an immunoglobulin fragment, which includes any fragment including more than a part of a variable domain of an immunoglobulin molecule retaining the specific binding ability of a full-length immunoglobulin which is naturally occurring, or partially or entirely synthesized, e.g., recombinantly prepared. Therefore, an antibody includes any protein having a binding domain, which is homologous to or substantially homologous to an immunoglobulin antigen-binding domain (antibody binding site). An antibody includes antibody fragments, e.g., anti-RSV antibody fragments. Therefore, the antibodies provided in the present invention include synthetic antibodies, recombinantly prepared antibodies, multispecific antibodies (e.g., bispecific antibodies), human antibodies, non-human antibodies, humanized antibodies, chimeric antibodies, intrabodies, and antibody fragments, but the present invention is not limited thereto. For example, the antibodies provided in the present invention include a Fab fragment, a Fab' fragment, a F(ab')2 fragment, a Fv fragment, a disulfide-stabilized Fv (dsFv), a Fd fragment, a Fd' fragment, a single-chain Fv (scFv), a single-chain Fab (scFab), a diabody, an anti-idiotype (anti-Id) antibody, or any antigen-binding fragment thereof. The antibodies provided in the present invention include components of any immunoglobulin types (e.g., IgG, IgM, IgD, IgE, IgA, and IgY), any categories (e.g., IgG1, IgG2, IgG3, IgG4, IgA1, and IgA2), or subclasses (e.g., IgG2a, and IgG2b).

The term "antibody fragment" or "antigen-binding fragment" of an antibody, used herein, includes at least a part (e.g., one or more CDRs and/or one or more antibody-binding sites) of a variable domain of an antibody binding to an antigen, smaller than a full-length antibody, and accordingly, it refers to any part of a full-length antibody, retaining at least some of the binding specificity and the specific binding ability of the full-length antibody. Therefore, the antigen-binding fragment refers to an antibody fragment containing an antigen-binding region that binds to the same antigen as an antibody form which the antibody fragment is derived. The antibody fragment includes not only an antibody derivative prepared by enzymatic treatment of a full-length antibody, but also a derivative prepared by synthesis, for example, recombination. The antibody fragment is included in an antibody. Examples of the antibody fragments include, but not limited to, Fab, Fab', F(ab')2, single-chain Fv (scFv), Fv, dsFv, a diabody, and Fd and Fd' fragments, and other fragments, such as modified fragments. The fragment may include, for example, multiple chains linked together by a disulfide linkage and/or peptide linker. Antibody fragments generally include approximately 50 or more amino acids, and typically approximately 200 or more amino acids.

The antigen-binding fragment includes any antibody fragment that generates an antibody (that is, with a Ka of approximately 10⁷ - 10⁸ M⁻¹ or more) immunospecifically binding to an antigen during insertion into an antibody framework (like that prepared by substitution of a corresponding region).

The term "neutralizing antibody" used herein is any antibody that binds to a pathogen to hinder the ability of the pathogen to infect cells/ cause a disease in a subject, or an antigen-binding fragment thereof. Examples of the neutralizing antibodies are neutralizing antibodies binding to a viral, bacterial, and fungal pathogens. Typically, the neutralizing antibody provided in the present invention binds to the surface of a pathogen. When a pathogen is a virus, a neutralizing antibody binding to the virus binds to a surface protein of the virus. According to the classification of viruses, the surface protein may be a capsid protein (e.g., a capsid protein of a non-enveloped virus) or a viral envelope protein (e.g., viral envelope protein of an enveloped virus). In some examples, the protein may be a glycoprotein. The ability to inhibit viral infection may be measured by *in vitro* neutralization analysis, for example, plaque reduction analysis using Vero host cells.

The term "surface protein" of a pathogen, used herein, is any protein located on the outer surface of the pathogen. The surface protein may be partially or entirely exposed to an external environment (that is, an outer surface). An example of the surface protein is a membrane protein, such as a protein located on a viral envelope surface or a bacterial outer membrane (e.g., a membrane glycoprotein). The membrane protein may be a transmembrane protein (i.e., proteins that passes through a lipid bilayer) or a non-transmembrane cell surface-associated protein (e.g., anchored or covalently attached to a membrane surface, like attachment of other proteins to the surface of a pathogen). An example of other surface proteins includes a viral capsid protein of a non-enveloped virus at least partially exposed to an external environment.

The term "epitope" used herein refers to any antigenic determinant on an antigen to which a paratope of the antibody binds. The epitope determinant generally includes a chemically active surface grouping of a molecule, for example, an amino acid or sugar side chain, and generally has a specific charge property, as well as a specific 3D structural property.

The term "phage display" used herein refers to the expression of a polypeptide on the surface of a filamentous bacteriophage.

The term "panning" used herein refers to an affinity-based selection for separating a molecule having specificity for a binding partner, for example, a captured molecule (e.g., an antigen), or a phage indicating the region, part or location of an amino acid or nucleotide.

The "specifically binding" or "immunospecifically binding" related to an antibody used herein or an antigen-binding fragment thereof is interchangeably used, and refers to the ability of an antibody or antigen-binding fragment to form one or more non-covalent bonds with a cognate antigen by non-covalent interaction between the antibody-binding site(s) of an antibody and an antigen.

The term "polypeptide" used herein refers to two or more amino acids which are covalently bonded. The term "polypeptide" and "protein" are interchangeably used in the present invention. The term "peptide" used herein refers to a polypeptide having a length of 2 to approximately 40 amino acids. In addition, the term "amino acid" used herein is an organic compound with an amino group and a carboxyl group. The polypeptide includes two or more amino acids.

The term "polynucleotide" and "nucleic acid molecule" used herein refers to an oligomer or polymer including two or more linked nucleotides or nucleotide derivatives, which are generally bound with each other by a phosphodiester bond, such as deoxyribonucleic acid (DNA) and ribonucleic acid (RNA).

The term "expression" used herein refers to a process of generating a polypeptide by the transcription and translation of a polynucleotide. The expression level of a polypeptide may be assessed by any method known in the art, for example, a method of determining a polypeptide amount from host cells. The method may include, but is not limited to, the quantification of a polypeptide in a cell lysate by ELISA, staining with Coomassie blue after electrophoresis, Lowry protein assay, and Bradford protein assay.

The term "host cell" used herein is a cell used to accommodate, maintain, reproduce, and amplify a vector. Host cells may also be used to express a polypeptide encoded by a vector. A nucleic acid included in the vector is replicated when the host cell divides and the nucleic acid is amplified. In one example, a host cell is a gene package, which expresses various polypeptides on its surface. In another example, a host cell is infected with a gene package. For example, a host cell may be a phage-displayed compatible host cell, which is transformed with a phage or phagemid vector to accommodate a phage package expressing fusion proteins including variant polypeptides.

The term "vector" used herein is a replicable nucleic acid capable of expressing one or more exogenous proteins upon transformation into a suitable host cell. The description on a vector includes vectors into which a nucleic acid encoding a polypeptide or a fragment thereof can be generally introduced by restriction enzyme digestion and ligation. The description on a vector also includes vectors having a nucleic acid encoding a polypeptide. A vector is used to introduce a nucleic acid encoding a polypeptide into a host cell for the amplification of a nucleic acid, or the expression/display of a polypeptide encoded by a nucleic acid. A vector generally remains in an episomal state, and may be designed to accomplish the integration of a gene or a part thereof into the chromosome of a genome. In addition, the present invention considers vectors, which are artificial chromosomes, for example, a yeast artificial chromosome and a mammalian artificial chromosome. The selection and use of such a vehicle is widely known in the art.

The vector includes a "virus vector" or "viral vector." The viral vector is an engineered virus operably linked to a foreign gene to transport (as a vehicle or shuttle) the foreign gene into cells.

The "expression vector" used herein includes a vector that can express DNA operably linked to a regulatory sequence capable of performing the expression of a DNA fragment, for example, a promoter region. The additional segments may include promoter and terminator sequences and arbitrarily include one or more replication origins, one or more selection markers, an enhancer, and a polyadenylation sequence. An expression vector may be generally derived from plasmid or viral DNA, or include both. Therefore, an expression vector means a recombinant DNA or RNA construct, for example, such as a plasmid, phage, recombinant virus, or other vectors causing the expression of cloned DNA upon introduction into a suitable host cell. An appropriate expression vector is well known to those of ordinary skill in the art, and includes those replicable in eukaryotic cells and/or prokaryotic cells and those remaining episomal, or those integrated into the host cell genome.

The term "modification" used herein refers to modification of an amino acid sequence of a polypeptide or a nucleotide sequence in a nucleic acid molecule, and includes deletion, insertion and substitution of each of amino acids or nucleotides. A method of modifying a polypeptide is that common used by those of ordinary skill in the art, may be, for example, recombinant DNA technology.

The term "infection" and "RSV infection" used herein refer to a pathological state derived from not only all stages of the RSV life cycle in a host (invasion and replication by RSV in cells or living tissues, but the present invention is not limited thereto), but also infiltration and replication by RSV. The infiltration and proliferation by RSV include, but not limited to, the following steps: docking of RSV particles to cells, fusion of the cell membrane and the virus, introduction of viral genetic information into cells, expression of RSV proteins, generation of new RSV particles, and release of RSV particles from cells. RSV infection may be upper respiratory tract RSV infection (URI), lower respiratory tract RSV infection (LRI), or a combination thereof. In some examples, a pathological condition resulting from the infiltration and replication by RSV is an acute RSV disease.

The "acute RSV disease" used herein refers to a clinically serious disease occurring in the lungs or lower respiratory tract as a result of RSV infection, and it may be manifested as pneumonia and/or bronchitis. Here, the signs of this disease may include, for example, hypoxia, suffocation, dyspnea, difficulty in breathing, gasping respiration, stridor, and cyanosis. An acute RSV disease requires an affected subject to obtain medical intervention, for example, hospitalization, oxygenation, endotracheal insertion and/or ventilation.

The "treatment" of a subject with a disease or condition, used herein, means that a subject's symptoms are partially or entirely alleviated, or remain in a static state after treatment. Accordingly, the treatment includes prevention, therapy and/or curing. The prevention means the prevention of a potential disease, and/or the prevention of the aggravation of symptoms or disease progression. The treatment also includes any pharmaceutical use of any antibody provided herein or an antigen-binding fragment thereof, or a composition provided herein.

The term "prevention" used herein refers to a method of reducing the risk of developing a disease or state.

The term "pharmaceutically effective drug (preparation)" used herein includes a conventional therapeutic drug including any medicine or bioactive agent, for example, an anesthetic, a vasoconstrictor, a dispersant, a small molecule drug and a therapeutic protein, but the present invention is not limited thereto.

The term "subject" used herein refers to mammals, for example, mammals including a human. The animals of the present invention include any animals, for example, primates including a human, a gorilla and a monkey; rodents including a mouse and a rat; poultry such as chicken; ruminants such as goats, cattle, deer and sheep; sheep and other animals such as a pig. Non-human animals exclude humans as a considered animal.

One aspect of the present invention provides an antibody or an antigen-binding fragment thereof specifically binding to respiratory syncytial virus (RSV) selected from the group consisting of the following (i) to (vii):
(i) an antibody specifically binding to RSV, comprising a heavy chain variable domain which comprises heavy chain CDR1 comprising the amino acid sequence of SEQ ID NO: 1, heavy chain CDR2 comprising the amino acid sequence of SEQ ID NO: 2, and heavy chain CDR3 comprising the amino acid sequence of SEQ ID NO: 3, and a light chain variable domain which comprises light chain CDR1 comprising the amino acid sequence of SEQ ID NO: 4, light chain CDR2 comprising the amino acid sequence of SEQ ID NO: 5, and light chain CDR3 comprising the amino acid sequence of SEQ ID NO: 6;
(ii) an antibody specifically binding to RSV, comprising a heavy chain variable domain which comprises heavy chain CDR1 comprising the amino acid sequence of SEQ ID NO: 1, heavy chain CDR2 comprising the amino acid sequence of SEQ ID NO: 2, and heavy chain CDR3 comprising the amino acid sequence of SEQ ID NO: 3, and a light chain variable domain which comprises light chain CDR1 comprising the amino acid sequence of SEQ ID NO: 7, light chain CDR2 comprising the amino acid sequence of SEQ ID NO: 5, and light chain CDR3 comprising the amino acid sequence of SEQ ID NO: 6;
(iii) an antibody specifically binding to RSV, comprising a heavy chain variable domain which comprises heavy chain CDR1 comprising the amino acid sequence of SEQ ID NO: 8, heavy chain CDR2 comprising the amino acid sequence of SEQ ID NO: 9, and heavy chain CDR3 comprising the amino acid sequence of SEQ ID NO: 10, and a light chain variable domain which comprises light chain CDR1 comprising the amino acid sequence of SEQ ID NO: 7, light chain CDR2 comprising the amino acid sequence of SEQ ID NO: 5, and light chain CDR3 comprising the amino acid sequence of SEQ ID NO: 6;
(iv) an antibody specifically binding to RSV, comprising a heavy chain variable domain which comprises heavy chain CDR1 comprising the amino acid sequence of SEQ ID NO: 11, heavy chain CDR2 comprising the amino acid sequence of SEQ ID NO: 12, and heavy chain CDR3 comprising the amino acid sequence of SEQ ID NO: 3, and a light chain variable domain which comprises light chain CDR1 comprising the amino acid sequence of SEQ ID NO: 13, light chain CDR2 comprising the amino acid sequence of SEQ ID NO: 5, and light chain CDR3 comprising the amino acid sequence of SEQ ID NO: 6;
(v) an antibody specifically binding to RSV, comprising a heavy chain variable domain which comprises heavy chain CDR1 comprising the amino acid sequence of SEQ ID NO: 1, heavy chain CDR2 comprising the amino acid sequence of SEQ ID NO: 2, and heavy chain CDR3 comprising the amino acid sequence of SEQ ID NO: 3, and a light chain variable domain which comprises light chain CDR1 comprising the amino acid sequence of SEQ ID NO: 14, light chain CDR2 comprising the amino acid sequence of SEQ ID NO: 15, and light chain CDR3 comprising the amino acid sequence of SEQ ID NO: 6;
(vi) an antibody specifically binding to RSV, comprising a heavy chain variable domain which comprises heavy chain CDR1 comprising the amino acid sequence of SEQ ID NO: 8, heavy chain CDR2 comprising the amino acid sequence of SEQ ID NO: 9, and heavy chain CDR3 comprising the amino acid sequence of SEQ ID NO: 3, and a light chain variable domain which comprises light chain CDR1 comprising the amino acid sequence of SEQ ID NO: 14, light chain CDR2 comprising the amino acid sequence of SEQ ID NO: 5, and light chain CDR3 comprising the amino acid sequence of SEQ ID NO: 6; and
(vii) an antibody specifically binding to RSV, comprising a heavy chain variable domain which comprises heavy chain CDR1 comprising the amino acid sequence of SEQ ID NO: 1, heavy chain CDR2 comprising the amino acid sequence of SEQ ID NO: 16, and heavy chain CDR3 comprising the amino acid sequence of SEQ ID NO: 17, and a light chain variable domain which comprises light chain CDR1 comprising the amino acid sequence of SEQ ID NO: 14, light chain CDR2 comprising the amino acid sequence of SEQ ID NO: 18, and light chain CDR3 comprising the amino acid sequence of SEQ ID NO: 6.

Another aspect of the present invention also provides an antibody or an antigen-binding fragment thereof specifically binding to RSV, selected from the group consisting of the following (viii) to (xv):
(viii) an antibody specifically binding to RSV, comprising a heavy chain variable domain comprising the amino acid sequence of SEQ ID NO: 19 and a light chain variable domain comprising the amino acid sequence of SEQ ID NO: 20;
(ix) an antibody specifically binding to RSV, comprising a heavy chain variable domain comprising the amino acid sequence of SEQ ID NO: 21 and a light chain variable domain comprising the amino acid sequence of SEQ ID NO: 22;
(x) an antibody specifically binding to RSV, comprising a heavy chain variable domain comprising the amino acid sequence of SEQ ID NO: 23 and a light chain variable domain comprising the amino acid sequence of SEQ ID NO: 24;
(xi) an antibody specifically binding to RSV, comprising a heavy chain variable domain comprising the amino acid sequence of SEQ ID NO: 25 and a light chain variable domain comprising the amino acid sequence of SEQ ID NO: 26;
(xii) an antibody specifically binding to RSV, comprising a heavy chain variable domain comprising the amino acid sequence of SEQ ID NO: 27 and a light chain variable domain comprising the amino acid sequence of SEQ ID NO: 28;
(xiii) an antibody specifically binding to RSV, comprising a heavy chain variable domain comprising the amino acid sequence of SEQ ID NO: 29 and a light chain variable domain comprising the amino acid sequence of SEQ ID NO: 30;
(xiv) an antibody specifically binding to RSV, comprising a heavy chain variable domain comprising the amino acid sequence of SEQ ID NO: 31 and a light chain variable domain comprising the amino acid sequence of SEQ ID NO: 24; and
(xv) an antibody specifically binding to RSV, comprising a heavy chain variable domain comprising the amino acid sequence of SEQ ID NO: 32 and a light chain variable domain comprising the amino acid sequence of SEQ ID NO: 33.

Still another aspect of the present invention provides an antibody or an antigen-binding fragment thereof specifically binding to RSV, selected from the group consisting of the following (xvi) to (xxiii):
(xvi) an antibody specifically binding to RSV, comprising a heavy chain variable domain encoded by the nucleotide sequence of SEQ ID NO: 34 and a light chain variable domain encoded by the nucleotide sequence of SEQ ID NO: 35;
(xvii) an antibody specifically binding to RSV, comprising a heavy chain variable domain encoded by the nucleotide sequence of SEQ ID NO: 36 and a light chain variable domain encoded by the nucleotide sequence of SEQ ID NO: 37;
(xviii) an antibody specifically binding to RSV, comprising a heavy chain variable domain encoded by the nucleotide sequence of SEQ ID NO: 38 and a light chain variable domain encoded by the nucleotide sequence of SEQ ID NO: 39;
(xix) an antibody specifically binding to RSV, comprising a heavy chain variable domain encoded by the nucleotide sequence of SEQ ID NO: 40 and a light chain variable domain encoded by the nucleotide sequence of SEQ ID NO: 41;
(xx) an antibody specifically binding to RSV, comprising a heavy chain variable domain encoded by the nucleotide sequence of SEQ ID NO: 42 and a light chain variable domain encoded by the nucleotide sequence of SEQ ID NO: 43;
(xxi) an antibody specifically binding to RSV, comprising a heavy chain variable domain encoded by the nucleotide sequence of SEQ ID NO: 44 and a light chain variable domain encoded by the nucleotide sequence of SEQ ID NO: 45;
(xxii) an antibody specifically binding to RSV, comprising a heavy chain variable domain encoded by the nucleotide sequence of SEQ ID NO: 46 and a light chain variable domain encoded by the nucleotide sequence of SEQ ID NO: 39; and
(xxiii) an antibody specifically binding to RSV, comprising a heavy chain variable domain encoded by the nucleotide sequence of SEQ ID NO: 47 and a light chain variable domain encoded by the nucleotide sequence of SEQ ID NO: 48.

The antibody according to the present invention may include an amino acid sequence having a 80% or more, preferably, 90% or more, more preferably 95% or more, and most preferably 99% or more homology with the amino acid sequence(s) of a heavy chain, a light chain, a heavy chain variable domain and/or a light chain variable domain of any one antibody selected from the group consisting of i) to xv).

The antibody or antigen-binding fragment of the present invention may specifically bind to RSV. Here, RSV to which the antibody or antigen-binding fragment binds may be a surface protein of RSV, such as the F-protein.

In the light chain and heavy chain variable domains, as long as the characteristics consistent with the purpose of the present invention, for example, the affinity to and specificity for RSV, are maintained, some amino acids can be substituted, inserted and/or deleted. For example, in the light chain and/or heavy chain variable domain(s), the conservative substitution of an amino acid may occur. The conservative substitution refers to the substitution with another amino acid residue having similar characteristics to the original amino acid sequence.

For example, lysine, arginine, and histidine have similar characteristics due to having basic side chains, and aspartic acid and glutamic acid have similar characteristics due to having acidic side chains. In addition, glycine, asparagine, glutamine, serine, threonine, tyrosine, cysteine, and tryptophan have similar properties due to having uncharged polar side chains, and alanine, valine, leucine, threonine, isoleucine, proline, phenylalanine, and methionine have similar characteristics due to having non-polar side chains, whereas tyrosine, phenylalanine, tryptophan, and histidine have similar characteristics due to having aromatic side chains. Therefore, it is obvious to those of ordinary skill in the art that, even if amino acid substitution occurs in groups having similar characteristics to those described above, there will be no significant change in characteristics. For this reason, as long as the characteristics of the antibody are maintained, the antibody in which a modification by conservative substitution occurs in a variable domain is also included in the scope of the present invention.

The antibody or antigen-binding fragment of the present invention may be a humanized antibody. The term "humanized antibody" used herein refers to a chimera antibody containing the minimal sequence derived from an immunoglobulin of a non-human antibody such as a mouse, and may mean an antibody in which all parts except the sequence corresponding to the hypervariable domain are substituted with the sequence of a human antibody. Here, the term "hypervariable domain (HVR)" refers to a variable domain showing hypervariability in an antibody sequence, or forming a structurally defined loop. Among the explanatory methods describing it, the Kabat's complementarity-determining region (CDR) is most commonly used as a method of classifying domains based on sequence variability.

An antibody fragment may be used as the antibody as long as the antibody's function is maintained. The antibody or antibody fragment may be a single-chain antibody, a diabody, a triabody, a tetrabody, a Fab fragment, a Fab' fragment, a F(ab')₂ fragment, Fd, scFv, a domain antibody, a minibody, a scab, an IgD antibody, an IgE antibody, an IgM antibody, an IgG1 antibody, an IgG2 antibody, an IgG3 antibody, an IgG4 antibody, a derivative of an antibody constant domain, or an artificial antibody based on a protein scaffold, maintaining the binding function to RSV, but the present invention is not limited thereto.

The antibody or antigen-binding site of the present invention may be a neutralizing antibody. The neutralizing antibody refers to any antibody that binds to a pathogen, thereby hindering the ability of a pathogen to infect cells or cause a disease in a subject, or an antigen-binding fragment thereof.

Another aspect of the present invention provides a polynucleotide encoding a light chain variable domain and a light chain variable domain of the antibody or antigen-binding fragment, and an expression vector comprising the same.

Specifically, a polynucleotide encoding the amino acid sequence of the anti-RSV antibody may be used separately or in the form of being inserted into one vector and a polynucleotide encoding a heavy chain or its variable domain may be used separately or in the form of being inserted into one vector.

An expression vector suitable for the production of the anti-RSV antibody may include a signal sequence for membrane targeting or secretion, in addition to expression regulatory elements such as a promoter, an initiation codon, a termination codon, a polyadenylation signal, and an enhancer. The initiation codon and termination codon are generally considered as parts of a polynucleotide sequence encoding an immunogenic target protein, and necessarily act in an individual when a gene construct is administered and must be in-frame with the coding sequence. A general promoter may be constitutive or inducible. As a promoter, a lac, tac, T3, or T7 promoter for prokaryotic cells, simian virus 40 (SV40), or a mouse mammary tumor virus (MMTV) promoter may be used, but the present invention is not limited thereto.

The expression vector may include a selective marker for selecting host cells containing the same. The selective marker is for selecting cells transformed with a vector, and may be a marker that imparts a selectable phenotype such as drug resistance, auxotrophy, resistance to a cytotoxic agent, or the expression of a surface protein. In an environment treated with a selective agent, since only cells expressing a selection marker survive, transformed cells may be selected. In addition, when the vector is a replicable expression vector, it may include a replication origin, which is a specific nucleic acid sequence from which replication is initiated.

As a recombinant expression vector for inserting a foreign gene, various types of vectors such as a plasmid, a virus, and a cosmid may be used. While the type of recombinant vector is not particularly limited as long as it serves to express a desired gene and produce a desired protein in various types of host cells of prokaryotic and/or eukaryotic cells, it is preferable to use a vector capable of possessing a promoter exhibiting potent activity and strong expression ability and massively producing a foreign protein in a form similar to the natural state.

Still another aspect of the present invention provides a host cell transformed with the expression vector. The expression vector may be inserted into a host cell to form a transformant.

The term "transformation into a host cell" used herein may include any method for introducing a nucleic acid into an organism, a cell, tissue or an organ, and may be performed by selecting suitable standard technology according to host cells as known in the art. Specifically, electroporation, protoplast fusion, calcium phosphate (CaPO₄) precipitation, calcium chloride (CaCh) precipitation, agitation with a silicon carbide fiber, agrobacterium-mediated transformation, PEG, dextran sulfate, Lipofectamine, and drying/suppression-mediated transformation may be used, but the present invention is not limited thereto.

Yet another aspect of the present invention provides a method of preparing an antibody or an antigen-binding fragment thereof specifically binding to respiratory syncytial virus (RSV), comprising the step of culturing the host cell. Specifically, the method of preparing an antibody may include preparing a recombinant vector by inserting a nucleotide sequence encoding the anti-RSV antibody; transforming host cells with the recombinant vector and culturing the resulting product; and isolating and purifying a humanized antibody from the cultured transformant.

Yet another aspect of the present invention provides a pharmaceutical composition for use in preventing or treating RSV infection, comprising the aforementioned antibody or antigen-binding fragment thereof.

The pharmaceutical composition may further include a pharmaceutically acceptable carrier. For oral administration, a binder, a lubricant, a disintegrant, an excipient, a solubilizer, a dispersant, a stabilizer, a suspending agent, a coloring agent, or a fragrance may be used, for injectables, a buffer, a preservative, a pain reliver, a solubilizer, an isotonic agent, and a stabilizer may be mixed and used, and for topical administration, a base material, an excipient, a lubricant, or a preservative may be used.

The pharmaceutical composition of the present invention may be prepared in various forms by being mixed with the above-described pharmaceutically acceptable carrier. For example, for oral administration, the pharmaceutical composition of the present invention may be prepared in various dosage forms such as a tablet, a troche, a capsule, an elixir, a suspension, a syrup and a wafer, and for injectables, the pharmaceutical composition or vaccine composition of the present invention may be prepared in a unit dose ampoule or multiple dose forms.

In addition, the pharmaceutical composition may include a surfactant that can improve membrane permeability. Such a surfactant may be derived from a steroid, a cationic lipid such as N-[1-(2,3-dioleyloxy)propyl-N,N,N-trimethylammonium chloride (DOTMA), or various types of compounds such as cholesterol hemisuccinate or phosphatidyl glycerol, but the present invention is not limited thereto.

The pharmaceutical composition may be administered together or sequentially with the above-described pharmaceutical or physiological component. Alternatively, the pharmaceutical composition may be administered in combination with an additional conventional therapeutic agent, or sequentially or simultaneously administered with a conventional therapeutic agent. Such administration may be performed once or multiple times. Taking all the factors into consideration, it is important to administer the pharmaceutical composition in an amount capable of obtaining the maximum effect in the minimum amount without side effects, and the amount may be easily determined by those of ordinary skill in the art.

The term "individual" used herein refers to a mammal suffering from a condition or disease that can be alleviated, suppressed or treated by administering the pharmaceutical composition, or having a risk thereof, and preferably, a human.

The term "administration" used herein refers to the introduction of a predetermined material into an individual by an appropriate method, and the pharmaceutical composition may be administered through any route that can reach target tissue. Such an administration method may be oral administration, intraperitoneal administration, intravenous administration, intramuscular administration, subcutaneous administration, intradermal administration, oral administration, topical administration, intranasal administration, intrapulmonary administration, or intrarectal administration, but the present invention is not limited thereto. However, when orally administered, proteins are digested, so it may be preferable that an oral composition must be formulated to coat an active ingredient or protect it from being degraded in the stomach. In addition, the pharmaceutical composition may be administered by any device to allow the active ingredient to move to target cells.

Hereinafter, the present invention will be described in further detail with reference to examples. The examples are merely provided to more specifically explain the present invention, and it will be obvious to those of ordinary skill in the art that the scope of the present invention is not limited to the examples according to the gist of the present invention.

### [Experimental materials: reagents and instruments]

### 1. Preparation of F-protein

**[Table 1]**

| Product | Supplier | Cat. # |
|---|---|---|
| PBS 1×, w/o Ca⁺⁺, Mg⁺⁺ 1 L | Lonza | 17-516Q |
| Molecular Grade Water | Corning | 46-000-CM |
| XL 1-Blue | Stratagene | 200228 |
| LB Broth | Amresco | J833-1L |
| Carbenicillin | Sigma | C3416 |
| Kanamycin Sulfate | Amresco | A20219 |
| NotI | New England Biolabs | R0189S |
| HindIII | New England Biolabs | R0104S |
| In-fusion HD Cloning Kit | Clontech | 639650 |
| T4 Ligase | New England Biolabs | M0202L |
| LB Agar LOP^{™} Plate + Carbenicilin75 | Narae Biotech | LN004CA |
| LB Agar LOP^{™} Plate + Kanamycin 50 | Narae Biotech | LN004K |
| Expi293F^{™} Cells | Gibco^{®} | A14524 |
| ExpiFectamine^{™} 293 Transfection Kits | Gibco^{®} | 14524 |
| Opti-MEMIReduced Serum Medium | Life Technologies | 31985-070 |
| Bacto Tryptone | DIFCO | 211705 |
| Bacto Yeast Extract | DIFCO | 212750 |
| MOPS | Sigma | M3183 |
| NuPAGE MOPS SDS Running Buffer (20X) | NOVEX | NP0001 |
| Tris Base | Merck Millipore | CAS 77-86-1 |
| Imidazole | Sigma | 12399 |
| Sodium Chloride | Sigma | S7653 |
| Ni-NTA Agarose | QIAGEN | 30210 |
| MabSelect^{™}Xtra | GE Healthcare | 17-5269-02 |
| Protein A IgG binding buffer | Pierce | 21007 |
| IgG Elution buffer | Pierce | 21009 |
| PageBlue Protein Staining Solution | Pierce | 24620 |
| Tween-20 | Sigma Aldrich | P1379 |
| TMB Microwell Peroxidase Substrate | KPL | 52-00-03 |
| TMB Stop Solution | KPL | 50-85-06 |
| Reagent / Kinetics buffer(10X) | FORTEBIO | 18-1092 |
| EDC | GE Healthcare | BR-1000-50 |
| NHS | GE Healthcare | BR-1000-50 |
| 10mM Acetate buffer | GE Healthcare | BR-1003-51 |
| 1.0 M Ethanolamine-HCl pH 8.5 | GE Healthcare | BR-1000-50 |
| HBS-EP buffer | GE Healthcare | BR-1001-88 |

**[Table 2]**

| Product | Supplier | Cat. No. |
|---|---|---|
| Qiaprep Spin Miniprep Kit | Qiagen | 27106 |
| Qiaprep Plasmid Midi Kit | Corning | 12945 |
| Gel Extraction Kit | Qiagen | 28706 |
| PCR Purification Kit | Qiagen | 28106 |
| 1L Erlenmeyer Culture Flask | Corning | 431147 |
| 225 mL Graduated Conical Tube | Corning | 352075 |
| Disposable 10 mL Polypropylene Columns | Pierce | 29924 |
| NuPAGE^{®} Novex 4-12% Bis-Tris Gel | Invitrogen | NP0321BOX |
| 1000 mL Vacuum Filter/Bottle, 0.22 µm (PES) | Corning | 431098 |
| Zeba^{™} Spin Desalting Columns | Thermo Scientific | 89891 |
| Vivaspin20 membrane 50.000 MWCO | Sartorus | VS2032 |
| 37°C Incubator | N-Biotek | SJP-250MI |
| 37°C Shaking Incubator | Vision Scientific Co., Ltd. | VS-8489SR |
| Clean Bench | Nok Woo Industry | N/A |
| CO₂ Incubator | N-Biotek | NB-206CXXL |
| Electrophoration Cuvette 0.2 cm | BTX | 620 |
| 50 mL Cornical tube | SPL | 50050 |
| Micro centrifugation Tube | SPL | 60115 |
| 15 mL Cornical tube | SPL | 50015 |
| pH meter (SevenEasy) | Mettler Toledo | N/A |
| Biosensors / Anti-Human Fc Capture (AHC) | FORTEBIO | 18-5064 |
| Greiner 96well plates | GREINER BIO-ONE | 655209 |

### 2. Construction and screening of human B cell-derived library

**[Table 3]**

| Product | Supplier | Cat. # |
|---|---|---|
| Anti-Human CD3 FITC (OKT3) | eBioscience | 11-0037 |
| ANTI-HUMAN CD8 ANTIBODY FITC | TONBO BIOS | 35-0087-T100 |
| Anti-human CD14 FITC | TONBO BIOS | 35-0149-T100 |
| Streptavidin, R-phycoerythrin conjugate (SAPE) | Invitrogen | S866 |
| Anti-Human CD19, PerCP-cyanine5.5 | eBioscience | 45-0199 |
| Anti-human CD20 PerCP-Cy5.5 | BD | 332781 |
| Mouse Anti-human CD27, PE-Cy7 | BD | 560609 |
| Streptavidin-APC | eBioscience | 17-4317-82 |
| RPMI-1640 Medium (ATCC Modification) | GIBCO | A10491-01 |
| Antibiotic-Antimyotic (100X) | GIBCO | 15240-062 |
| 55mM 2-Mercaptoethanol (1,000X) | Thermo Fisher | 21985023 |
| Fetal Bovine Serum, certified, US origin | GIBCO | 16000044 |
| GlutaMAX Supplement | Invitrogen | 35050-061 |
| Ficoll-Paque PLUS | GE | 17-1440-03 |
| SPRIselect Reagent | Beckman Coulter | B23318 |
| T4 DNA Ligase | Invitrogen | 15224017 |
| SepMate-50 | STEMCELL Technologies Inc | ST86450 |
| Dimethyl sulfoxide | Sigma Aldrich | D2650 |
| SuperScript^{®}IV First-Strand Synthesis S | Invitrogen | 18091050 |
| Expand High FidelityPLUS PCR System | Roche | 03300226001 |
| Deoxynucleotide (dNTP) Solution Mix | NEB | N0447L |
| Sheath Fluid | BD | 342003 |
| 21343EZ-Link^{®}NHS-Biotin Reagents | Thermo Fisher | 21336 |
| SPRIselect Reagent | Beckman Coulter | B23318 |
| Chromium Single Cell 5 prime Library Gel | 10X Genomics | 1000014 |
| Chromium Single Cell A Chip Kit 16 rxns | 10X Genomics | 1000008 |
| PBS 1×, w/o Ca⁺⁺, Mg⁺⁺ 1 L | Lonza | 17-516Q |
| Molecular Grade Water | Corning | 46-000-CM |
| XL1-Blue | Stratagene | 200228 |
| T7 Express Strains | New England Biolabs | C3010 |
| LB Broth | Amresco | J833-1L |
| Carbenicillin | Sigma | C3416 |
| Kanamycin Sulfate | Amresco | A20219 |
| NotI | New England Biolabs | R0189S |
| Nco I | New England Biolabs | R0193S |
| T4 Ligase | New England Biolabs | M0202L |
| LB Agar LOP^{™} Plate + Carbenicilin75 | Narae Biotech | LN004CA |
| LB Agar LOP^{™} Plate + Kanamycin 50 | Narae Biotech | LN004K |
| Bacto Tryptone | DIFCO | 211705 |
| Bacto Yeast Extract | DIFCO | 212750 |
| MOPS | Sigma | M3183 |
| NuPAGE MOPS SDS Running Buffer (20X) | NOVEX | NP0001 |
| Tris Base | Merck Millipore | CAS 77-86-1 |
| Boric acid | Sigma | B6768 |
| Sodium Chloride | Sigma | S7653 |
| Sodium Azide | Sigma | S8032 |
| Bovine Serum Albumins | Sigma | A2058 |
| Ethanol | VWR Lifescience | E193-500ML |
| 0.5 M EDTA (pH 8.0) | Ambion | AM9260G |
| Glycerol | Sigma Aldrich | G7893 |
| MabSelect^{™}Xtra | GE Healthcare | 17-5269-02 |
| Protein A IgG binding buffer | Pierce | 21007 |
| IgG Elution buffer | Pierce | 21009 |
| PageBlue Protein Staining Solution | Pierce | 24620 |
| Tween-20 | Sigma Aldrich | P1379 |
| TMB Microwell Peroxidase Substrate | KPL | 52-00-03 |
| TMB Stop Solution | KPL | 50-85-06 |
| Dithiothreitol (DTT), 0.1M Solution | USB | 707265ML |

**[Table 4]**

| Product | Supplier | Cat. No. |
|---|---|---|
| PCR cleanup kit | MN | 740609.10 |
| QuickExtract^{™}RNA Extraction Kit | Lucigen | QER090150 |
| SMARTer^{®}RACE 5'/3' Kit | Takara Bio USA Inc | 634859 |
| Chromium Single Cell V(D)J Enrichment Kit, Human B Cell, 96 rxns | 10X Genomics | 1000016 |
| Chromium i7 Multiplex Kit 96 rxns | 10X Genomics | 120262 |
| Chromium Single Cell A Chip Kit 16 rxns | 10X Genomics | 1000009 |
| Qubit^{™}dsDNA HS Assay Kit | Thermo Fisher | Q32854 |
| Eppendorf PCR Tubes 0.2ml | Eppendorf | 0030124359 |
| High Sensitivity DNA Kit | Agilent | 5067-4626 |
| In-fusion HD Cloning Kit | Clontech | 639650 |
| Qiaprep Spin Miniprep Kit | Qiagen | 27106 |
| Gel Extraction Kit | Qiagen | 28706 |
| PCR Purification Kit | Qiagen | 28106 |
| 1L Erlenmeyer Culture Flask | Corning | 431147 |
| 225 mL Graduated Conical Tube | Corning | 352075 |
| Disposable 10 mL Polypropylene Columns | Pierce | 29924 |
| NuPAGE^{®} Novex 4-12% Bis-Tris Gel | Invitrogen | NP0321BOX |
| 1000 mL Vacuum Filter/Bottle, 0.22 µm (PES) | Corning | 431098 |
| Vivaspin20 membrane 50.000 MWCO | Sartorus | VS2032 |
| 37°C Incubator | N-Biotek | SJP-250MI |
| 37°C Shaking Incubator | Vision Scientific Co., Ltd. | VS-8489SR |
| Clean Bench | Nok Woo Industry | N/A |
| CO₂ Incubator | N-Biotek | NB-206CXXL |
| Electrophoration Cuvette 0.2 cm | BTX | 620 |
| 50 mL Cornical tube | SPL | 50050 |
| Micro centrifugation Tube | SPL | 60115 |
| 15 mL Cornical tube | SPL | 50015 |
| PCR tube | Bioneer | T1C-028-R |
| Internal cryogenic vial | Corning | 431417 |
| pH meter (SevenEasy) | Mettler Toledo | N/A |
| BD LSRFIRTESSA | BD | 649225 |
| NanoDrop^{™}2000 Spectrophotometer | Thermo Fisher | ND-2000 |

### 3. Preparation and selection of anti-RSV antibody

**[Table 5]**

| Product | Supplier | Cat. # |
|---|---|---|
| PBS 1×, w/o Ca⁺⁺, Mg⁺⁺ 1 L | Lonza | 17-516Q |
| Molecular Grade Water | Corning | 46-000-CM |
| XL 1-Blue | Stratagene | 200228 |
| LB Broth | Amresco | J833-1L |
| Carbenicillin | Sigma | C3416 |
| Kanamycin Sulfate | Amresco | A20219 |
| LB Agar LOP^{™} Plate + Carbenicilin75 | Narae Biotech | LN004CA |
| Bacto Tryptone | DIFCO | 211705 |
| Bacto Yeast Extract | DIFCO | 212750 |
| MOPS | Sigma | M3183 |
| Tris Base | Merck Millipore | CAS 77-86-1 |
| Sodium Chloride | Sigma | S7653 |
| NuPAGE MOPS SDS Running Buffer (20X) | NOVEX | NP0001 |
| Protein A IgG binding buffer | Pierce | 21007 |
| IgG Elution buffer | Pierce | 21009 |
| PageBlue Protein Staining Solution | Pierce | 24620 |
| Protein A IgG binding buffer | Pierce | 21007 |
| SuperScript^{®}IV First-Strand Synthesis S | Invitrogen | 18091050 |
| Expand High FidelityPLUS PCR System | Roche | 03300226001 |
| Deoxynucleotide (dNTP) Solution Mix | NEB | N0447L |
| Tween-20 | Sigma Aldrich | P1379 |
| TMB Microwell Peroxidase Substrate | KPL | 52-00-03 |
| TMB Stop Solution | KPL | 50-85-06 |

**[Table 6]**

| Product | Supplier | Cat. No. |
|---|---|---|
| Qiaprep Spin Miniprep Kit | Qiagen | 27106 |
| Qiaprep Plasmid Midi Kit | Corning | 12945 |
| Gel Extraction Kit | Qiagen | 28706 |
| PCR Purification Kit | Qiagen | 28106 |
| 1L Erlenmeyer Culture Flask | Corning | 431147 |
| 225 mL Graduated Conical Tube | Corning | 352075 |
| Disposable 10 mL Polypropylene Columns | Pierce | 29924 |
| NuPAGE^{®} Novex 4-12% Bis-Tris Gel | Invitrogen | NP0321BOX |
| 1000 mL Vacuum Filter/Bottle, 0.22 µm (PES) | Corning | 431098 |
| Zeba^{™} Spin Desalting Columns | Thermo Scientific | 89891 |
| Vivaspin20 membrane 50.000 MWCO | Sartorus | VS2032 |
| 37°C Incubator | N-Biotek | SJP-250MI |
| 37°C Shaking Incubator | Vision Scientific Co., Ltd. | VS-8489SR |
| Clean Bench | Nok Woo Industry | N/A |
| CO₂ Incubator | N-Biotek | NB-206CXXL |
| Electrophoration Cuvette 0.2 cm | BTX | 620 |
| 50 mL Cornical tube | SPL | 50050 |
| Micro Centrifuge Tube | SPL | 60115 |
| 15 mL Cornical tube | SPL | 50015 |
| pH meter (SevenEasy) | Mettler Toledo | N/A |

### <Experimental method>

### 1. Preparation of RSV F-protein antigen

### 1.1. Transformation and protein expression

100 ng of a F-protein subtype gene expression vector (pcIW) was added to competent cells (competent sample) and mixed. The resulting mixture was transferred to a cuvette for electroporation, and then an electric shock was applied with a gap of 2 mm and a voltage of 2.5 kV. The cells were recovered in a shaking incubator with 1 mL of LB media for 1 hour at 37 °C. The resulting cells were spread on an LB plate containing carbenicillin (75 µg/mL) for transformation. The obtained plasmid was sequenced to confirm whether a desired gene was cloned in a vector.

The day before expression, Expi293 cells (host cells) were sub-cultured at 2 × 10⁶ samples/mL. After confirming that the number of cells reached 3 × 10⁶ samples/mL or more on the day of expression, a mixture containing 30 µg of prepared DNA in 1.5 mL of Opti-MEM medium and a mixture containing 80 µL of ExpiFectamine in 1.5 mL of a medium were prepared and incubated for 5 minutes at room temperature. Afterward, the two mixtures were mixed and incubated for 20 to 30 minutes, and then the mixture was added to the prepared cells (25.5 mL). Enhancer 1 and Enhancer 2 were added at 150 µL and 1.5 mL 16 to 20 hours after expression, respectively, and expressed for 5 to 7 days at 37 °C in a shaking incubator.

### 1.2. Protein purification and concentration

1 mL of a Ni-NTA agarose resin washed with PBS was added to a protein-expressing supernatant, and inverted at 4 °C for 2 hours and at room temperature for 30 minutes. After transferring to a column for purification, the resulting mixture was sufficiently washed with an over20-fold amount of a washing buffer (His-tag). Subsequently, 500 µL of an eluent was acquired six or more times using an elution buffer, and then the concentration of each sample was measured at a wavelength of 280 nm.

For buffer concentration and concentration, 10 mL of a PBS buffer was added to a Vivaspin20 membrane, centrifuged at 6500 rpm for 5 minutes to wash the membrane. After adding 10 mL of the PBS buffer, the sample was centrifuged at 6500 rpm for 10 minutes (repeated three times to contain a total of 30 mL of PBS). After centrifugation until approximately 500 µL of the sample remained, the resulting filtrate was collected to measure a concentration.

The prepared protein was confirmed using SDS-PAGE. The sample and a 4X non-reducing sample buffer were mixed in a volume ratio of 3:1, heated at 90 °C for 3 minutes and then cooled, followed by loading 3 µg of the prepared sample to each well of a 4 to 12% Bis-Tris gel and performing electrophoresis. The gel was isolated and stained with a PageBlue Protein Staining Solution for 30 minutes or more, followed by destaining with distilled water (DW) and confirming the size and purity of the protein.

### 2. Construction of human B cell-derived library

### 2.1. Isolation of human PBMCs

Peripheral blood mononuclear cells (PBMCs) were isolated by collecting blood samples from 40 healthy adults, 30 RSV convalescent infants, and 10 healthy infants.

After 35 mL each of the blood samples collected from the healthy adult donors was mixed with 35 mL of 2% FBS/PBS in a 1:1 ratio, the resulting mixture was dispensed into two 50-mL conical tubes (SPL, 50050). 15 mL of Ficoll-Paque Plus (GE, 17-1440-03) was added to each of four SepMate tubes (STEMCELL Technologies Inc, ST86450), a mixture of the blood and PBS (Lonza, 17-516Q) was overlaid by 17.5 mL, and centrifuged at 1200 g for 10 minutes (25 °C, Acc5/Dec 5). After removing a plasma, the supernatant was collected in a new 50 mL conical tube (SPL, 50050) and centrifuged at 300 × g for 8 minutes (25 °C, Acc5/Dec 5). The resulting product was washed with 20 mL of 2% FBS/PBS, and centrifuged at 300 × g for 8 minutes (25 °C, Acc5/Dec 5). A supernatant was removed. Cells were resuspended with 10 mL of complete media (RPMI GIBCO A10491-01, 10% FBS GIBCO 16000044, 1% GlutaMAX Invitrogen 35050-061, 1% antibiotic-antimyotic GIBCO 15240-062, 1000x 2-mercaptoethanol Thermo Fisher 21985023), and transferred to a new 15-mL conical tube (SPL, 50015). 50 µL of the resuspended cells was counted, and the number of stored samples was determined (1 × 10⁷ cells/mL, 1 mL/vial). Remaining cells were centrifuged at 300 × g for 8 minutes (25 °C, Acc5/Dec 5). After resuspending the cells with FBS (GIBCO, 16000044) in a volume half the vial volume, the other half of the volume was filled with 20% DMSO/FBS and dispensed into an internal cryogenic vial (Corning, 431417). The vial was cryopreserved using CRF and then stored in liquid nitrogen.

4 mL of PBS (Lonza, 17-516Q) was added to 2 mL of the blood samples collected from the RSV convalescent and healthy infants and then gently inverted. 3 mL of Ficoll-Paque Plus (GE, 17-1440-03) was added to a 15 mL tube, and 6 mL of PBS-diluted blood was layered. After centrifugation (Brake: off) at room temperature and 1500 rpm for 25 minutes, a plasma and approximately 2 mL of a buffy coat of the Ficoll-Paque Plus interface were transferred to a new 15-mL tube (SPL, 50015). 10 mL of PBS (Lonza, 17-516Q) was added to the buffy coat and centrifuged (Brake on [9]) at 1200 rpm for 10 minutes, and a washing process for removing the supernatant was repeated twice. The supernatant-removed PBMCs were suspended in 1 mL of Complete RPMI (RPMI GIBCO A10491-01, 10% FBS GIBCO 16000044, 1% GlutaMAX Invitrogen 35050-061, 1% antibiotic-antimyotic GIBCO 15240-062, 1000x 2-mercaptoethanol Thermo Fisher 21985023), and the cells were counted using an Adam counter. After the supernatant was removed by centrifugation, the cells were suspended with FBS (GIBCO, 16000044) to be 2~4 × 10⁷ cell/mL, 20% DMSO/FBS was carefully mixed with FBS in the same volume (1:1) ratio, and then the mixture was transferred to an internal cryogenic vial (Corning, 431417). The vial was cryopreserved using CRF and then stored in liquid nitrogen.

### 2.2. Isolation of B cells specific for RSV F-protein

7 PBMC vials (donor #2, 4, 11, 23, 29, 39, 43, total cell number of 4.47 × 10⁷ cells) were resuspended in RPMI (GIBCO, A10491-01), and centrifuged at 1500 rpm for 10 minutes (25 °C) to discard the supernatant. The remaining cells were resuspended with 1 mL of 2% FBS/PBS, and centrifuged at 1,000 rpm for 3 minutes to discard the supernatant. PBMC cells were resuspended with 0.5 mL of 2% FBS/PBS such that a biotinylated RSV F (DS-Cav1) protein became 200 nM. The cells were reacted by tapping at 4 °C for 1 hour to prevent them from settling. After centrifugation at 1000 rpm for 3 minutes, the supernatant was discarded, and a process of washing the cells with 1 mL of 2% FBS/PBS and centrifuging the cells was repeated twice. 2.5 µL each of labeled antibodies (anti-human CD3 FITC eBioscience 11-0037, anti-human CD8 FITC TONBO BIOS 35-0087-T100, anti-human CD14 FITC TONBO BIOS 35-0149-T100, Streptavidin R-PE conjugate Invitrogen S866, anti-human CD19 Per-CP-cyanine5.5 eBioscience 45-0199, anti-human 20 PerCP-Cy5.5 BD 332781, mouse anti-human CD27 PE-Cy7 BD 560609, Streptavidin-APC eBioscience 17-4317-82) were added to 0.5 mL of 2% FBS/PBS and well mixed to manufacture an antibody mixture, followed by resuspension of the supernatant-removed cells and reaction at 4 °C for 20 minutes. After centrifugation at 1000 rpm for 3 minutes, the supernatant was discarded, and a process of washing the cells with 1 mL of 2% FBS/PBS and performing centrifugation was repeated twice, followed by resuspension with 0.5 mL of 2% FBS/PBS and sorting with FACSAriaII. Here, as a sorting gate, SSC/FSC → PBMC → CD3⁻CD8⁻CD14⁻ /CD19⁺CD20⁺ → CD27⁺ → PE⁺/APC⁺ (for the control B cells, PE⁻/APC⁻) was selected, and approximately 1,000 cells were collected at a level of approximately 0.1 to 0.5% of CD27⁺ B cells in 0.5 mL of 2% FBS/PBS.

### 2.3. Construction of B cell-derived V(D)J library

Using a SMARTer RACE 5' Kit (Takara Bio USA Inc, 634859), RT-PCR was performed. Isolated B cells were centrifuged at 1,500 rpm for 10 minutes, resuspended with 60 µL of Quick extraction buffer (Lucigen, QER090150), and reacted at -80 °C overnight. 4 µL of 5× RT buffer (Invitrogen, 18091050), 0.5 µL of DTT (USB, 707265ML, 100 mM), and dNTPs (NEB, N0447L, 20 mM) were mixed to prepare a cDNA synthesis reaction mixture, and then the mixture was left at room temperature. 10 µL of cells lysed with Quick extraction buffer (Lucigen, QER090150) and 1 µL of a 5'-CDC primer were transferred to a PCR tube (Bioneer, T1C-028-R) to allow a reaction at 72 °C for 3 minutes and at 42 °C for 2 minutes, followed by spinning down. 0.5 µL of an RNase inhibitor and 2 µL of SMARTScribe Reverse Transcriptase were mixed with 5.5 µL of a prepared cDNA synthesis reaction mixture, and left at room temperature. 1 µL of SMARTer II A oligonucleotide was put into the tube in which the reaction had been completed, and 8 µL of the prepared mixture was added. After spin-down, RT-PCR was performed. RT-PCR was performed at 42 °C for 90 minutes and at 70 °C for 10 minutes. 10 µL of Tricine-EDTA buffer was added to the tube in which the reaction had been completed and diluted.

After 15.5 µL of PCR Grade H₂O, 25 µL of 2X SeqAmp Buffer, and 1 µL of SeqAmp DNA polymerase were mixed to prepare 41.5 µL of a mixture, 2.5 µL of 5'-RACE-finished cDNA, 5 µL of 10X UPM, and 1 µL of 5'-GSP, that is, RT-IgGKLA (10 µM) were added and gently mixed, followed by performing PCR. PCR was performed by repeating 25 cycles at 94 °C for 30 seconds, at 68 °C for 30 seconds, and 72 °C for 3 minutes. An RT-IgGKLA primer was prepared by mixing four types of sequences shown in Table 7 in a 1:1:1:1 ratio.

**[Table 7]**

| Name | Sequences (5'-3') | SEQ ID NO: |
|---|---|---|
| RT-IgGKLA | GTGTGCACGCCGCTGGTC | 334 |
| | GTGGGAAGTTTCTGGCGGTCAC | 335 |
| | TGGAGGGCGTTATCCACCTTCC | 336 |
| | GTGCTCCCTTCATGCGTGACC | 337 |

The PCR-finished reaction product was subjected to PCR cleanup. 100 µL of NTI buffer (MN, 740609.10) was added and loaded on a PCR clean-up column (MN, 740609.10), followed by centrifugation. After the removal of the flow-through, a washing process of adding 700 µL of buffer NT3 (MN, 740609.10) and performing centrifugation again was repeated twice, followed by centrifugation for 1 minute. Afterward, the column was transferred to a new tube, incubated for 1 minute at room temperature with 20 µL of Buffer NE and then centrifuged, thereby obtaining cDNA.

### 2.4. Construction chromium single cell V(D)J library

A Chromium Single Cell V(D)J Enrichment Human B cell Kit (10X Genomics, 1000016) was used. Sorted cells were centrifuged at 1500 rpm for 10 minutes, and resuspended with 60 µL of PBSF (1×PBS, 2% FBS). The resuspension volume was determined so that the concentration became 100 to 2000 cells/µL and a minimum of 40 µL (volume used in counting (30 ml) + volume used in experiment (10 µL)) was obtained. In this experiment, unsorted PBMCs and the control B cell sample were resuspended in 60 µL, but in the case of RSV positive B cells, only 1,000 cells were sorted and diluted to 10 µL, followed by carrying out an experiment immediately without counting.

Counting of B cells specific for the RSV F-protein was sampled enough to perform three times. Counting was performed three times for each sample, and the average value of the results of counting a total of 9 times was obtained. Since the number of sorted cells was approximately 1,000, 31.7 µL of cells not mixed with nuclease-free water (Corning, 46-000-CM) were prepared. In the case of the control B cells, a target cell recovery number was set to 2,000 (since targeted cell recovery efficiency is approximately 50%, the target cell recovery number was set to approximately 1,000, twice the level of sorted cells) at a concentration of 600 cells/µL, and 5.8 µL of cells and 25.9 µL of nuclease-free water (Corning, 46-000-CM) were mixed. For unsorted control PBMCs, as the concentration of a cell stock was 1,400 cells/µL and a targeted cell recovery number was 20000 (for PBMCs, since a B cell population is approximately 10%, 10-fold the target cell recovery number of the B cells was taken), the cells were mixed with 24.8 µL of a cell stock and 7.9 µL of nuclease-free water (Corning, 46-000-CM). The amount of cells to be mixed may be changed according to the number of sorted cells and the desired degree of target cell recovery, and the kit guide was referred for each volume. For each sample, 50 µL of RT reagent mix, 5.9 µL of Poly-dT RT primer, 2.4µL of Additive A, and 10 µL of RT enzyme Mix B were mixed by pipetting 15 times, allowed to settle and then stored on ice.

After binding of Chromium Chip A (10X Genomics, 100009) to a 10x chip holder, 90 µL, 40 µL, and 270 µL of a 50% glycerol solution (Glycerol, Sigma Aldrich G5516) were added to wells that would not be used, marked 1, 2 and 3 on their bottom, respectively. Cells mixed with nuclease-free water (Corning, 46-000-CM) were mixed with the RT-reaction mixture prepared on ice, and then 90 µL of the cell mixture was added to the No. 1 well. 40 µL of gel beads vortexed for 30 seconds were added to the No. 2 well, and 135 µL of partitioning oil was added to the No. 3 well twice (a total of 270 µL). After installing a 10x gasket, GEMs were formed by performing the Single Cell program of the Chromium controller.

After storing the PCR tube on ice, 100 µL of GEMs formed by the Single Cell program were transferred to a PCR tube strip (Eppendorf, 0030124359), and GEM-RT incubation was performed. GEM-RT was performed in a thermal cycler in which a lid temperature was set to 53 °C at 53 °C for 45 minutes, at 85°C for 5 minutes and at 4°C hold.

After GEM-RT, 125 µL of a recovery agent was added to each sample and then incubated for 60 seconds. After the layers of the mixture were separated, a tip was inserted into the bottom of the tube to remove 125 µL of the recovery agent/partitioning oil (pink) layers. 200 µL of a Dynabeads cleanup mix prepared by mixing 9 µL of nuclease-free water (Corning, 46-000-CM), 182 µL of Buffer Sample Clean Up 1, 4 µL of Dynabeads MyOne SILANE, and 5 µL of Additive A was added to each sample. After mixing by pipetting, the resulting mixture was incubated for 10 minutes at room temperature.

After incubation, the tube was located in a 10x Magnetic Separator/High position, and the supernatant of the mixture was removed. 300 µL of 80% ethanol (VWR, E193-500mL) was added to the pellet, and then the ethanol was removed. 200 µL of 80% ethanol was added, and after 30 seconds, the ethanol was removed. After centrifugation, the tube was placed in the low position of the magnet, and the remaining ethanol was removed. After performing air drying for 1 minute, the magnet was removed. 35.5 µL of Elution Solution I (98 µL of Buffer EB, 1 µL of 10% Tween 20 (Sigma Aldrich, P1379), and 1 µL of Additive A) was added and mixed by pipetting. After incubation at room temperature for 1 minute, the tube was located in the low position of the magnet, and the supernatant was transferred to a new tube.

65 µL of cDNA Amplification Mix was added to the GEM-RT Cleanup-finished sample. The cDNA Amplification Mix was prepared by mixing 8 µL of nuclease-free water, 50 µL of an amplification master mix, 5 µL of cDNA Additive, and 2 µL of cDNA Primer Mix. The sample was mixed using a pipette, allowed to settle, and subjected to cDNA amplification PCR. PCR was carried out repeatedly in a thermal cycler in which a lid temperature was set to 105 °C for 14 cycles of 98 °C for 45 seconds, 98 °C for 20 seconds, 67 °C for 30 seconds, and 72 °C for 1 minute, and then at 72 °C for 1 minute and at 4 °C hold.

60 µL of a vortexed SPRIselect reagent (Beckman Coulter, B23318) was added to each of the PCR-completed samples and mixed with a pipette. After incubation at room temperature for 5 minutes, the tube was placed in the high position of the magnet, and the supernatant was removed. 30 seconds after putting 200 µL of 80% ethanol into the pellet, a washing process of removing ethanol was performed a total of three times. After centrifugation, the tube was placed in the low position of the magnet, the remaining ethanol was removed, and air drying was carried out for 1 minute. After the removal of the magnet, 45.5 µL of Buffer EB was added and mixed using a pipette for 15 seconds, and incubated at room temperature for 2 minutes. After placing the tube in the high position of the magnet, 45 µL of the supernatant was transferred to a new tube. 1 µL of the sample transferred to the new tube was taken to perform QC and quantification using Agilent Tapestation.

2 µL of the sample was transferred to a new tube and mixed with 33 µL of nuclease-free water (Corning, 46-000-CM). 65 µL of Target Enrichment 1 Reaction Mix prepared by mixing 5 µL of nuclease-free water, 50 µL an amplification master mix, 5 µL of cDNA Additive, and 1 5 µL of B Cell Mix was added to each tube, thereby preparing 100 µL of a PCR reaction mixture, and then PCR was carried out. PCR was performed in a thermal cycler in which a lid temperature was set to 105 °C at 98 °C for 45 seconds, performed repeatedly for 8 cycles of 98 °C for 20 seconds, 67 °C for 30 seconds, and 72 °C for 1 minute (6 cycles for the control B cells), and then performed at 72 °C for 1 minute and at 4 °C hold.

80 µL of a vortexed SPRIselect reagent (Beckman Coulter, B23318) was added to each of the PCR-completed samples and mixed with a pipette. After incubation at room temperature for 5 minutes, the tube was placed in the high position of the magnet, and the supernatant was removed. 30 seconds after putting 200 µL of 80% ethanol (Ethanol, VWR, E193-500 mL) into the pellet, a washing process of removing ethanol was performed a total of three times. After centrifugation, the tube was placed in the low position of the magnet, the remaining ethanol was removed, and air drying was carried out for 1 minute. After the removal of the magnet, 35.5 µL of Buffer EB was added and mixed using a pipette for 15 seconds, and incubated at room temperature for 2 minutes. After placing the tube in the low position of the magnet, 35 µL of the supernatant was transferred to a new tube.

65 µL of Target Enrichment 2 Reaction Mix prepared by mixing 5 µL of nuclease-free water (Corning, 46-000-CM), 50 µL of an amplification master mix, 5 µL of cDNA Additive, and 25 µL of B cell Mix was added to 35 µL of the sample and mixed using a pipette, followed by performing 2nd PCR. PCR was performed in a thermal cycler in which a lid temperature was set to 105 °C at 98 °C for 45 seconds, repeated for 10 cycles of 98 °C for 20 seconds, 67 °C for 30 seconds, and 72 °C for 1 minute (8 cycles for the control B cells), and performed at 72 °C for 1 minute and at 4 °C hold.

50 µL of a vortexed SPRIselect reagent (Beckman Coulter, B23318) was added to each of the PCR-completed samples and mixed with a pipette. After incubation at room temperature for 5 minutes, the tube was placed in the low position of the magnet, and the supernatant was removed. 30 µL of a SPRIselect reagent (Beckman Coulter, B23318) was added to the sample transferred to the new tube, mixed and reacted at room temperature for 5 minutes. The tube was placed in the high position of the magnet, and 170 µL of the supernatant was removed. 30 seconds after putting 200 µL of 80% ethanol (VWR, E193-500 mL) into the pellet, a washing process of removing ethanol was performed a total of three times. After centrifugation, the tube was placed in the low position of the magnet, the remaining ethanol was removed. After removing the magnet, 45.5 µL, of Buffer EB was added and mixed using a pipette for 15 seconds, and incubated at room temperature for 2 minutes. After placing the tube in the low position of the magnet, 35 µL of the supernatant was transferred to a new tube. 1 µL of the sample contained in the new tube was taken and diluted 1:5, followed by performing QC and quantification using Agilent Tapestation. The sample for which the above procedure had been completed was used to construct a phage-displayed scFv library.

After 50 ng of the sample was transferred to a new tube and diluted with nuclease-free water to 20 µL, a Fragmentation Mix prepared by mixing 5 µL of fragmentation buffer, 10 µL of a fragmentation enzyme blend, and 15 µL of nuclease-free water was added to each sample, and a fragmentation reaction was carried out using a pre-chilled thermal cycler. The reaction was performed in a thermal cycler in which a lid temperature was set to 65 °C under conditions of 4 °C hold (skip immediately after the addition of the sample), 32 °C for 2 minutes, 65 °C for 30 minutes, and 4 °C hold. 50 µL of Adaptor Ligation Mix prepared by mixing 17.5 µL of nuclease-free water, 20 µL of a ligation buffer, 10 µL of a DNA ligase, and 2.5 µL of Adaptor Mix was added to 50 µL of the reaction-completed sample, mixed using a pipette and incubated using a thermal cycler. The incubation was performed in a thermal cycler in which a lid temperature was set to 30 °C under conditions of 20 °C for 15 minutes and 4 °C hold.

After a ligation reaction, a cleanup was carried out. 80 µL of the vortexed SPRIselect reagent (Beckman Coulter, B23318) was added to each sample and mixed using a pipette. After incubation at room temperature for 5 minutes, the tube was placed in a high position of the magnet and the supernatant was removed. 30 seconds after putting 200 µL of 80% ethanol into the pellet, a washing process of removing ethanol was performed a total of three times. After centrifugation, the tube was placed in the low position of the magnet, the remaining ethanol was removed and air drying was carried out for 1 minute. After the removal of the magnet, 30.5 µL of Buffer EB was added and mixed using a pipette for 15 seconds, followed by incubation at room temperature for 2 minutes. After placing the tube in the low position of the magnet, 30 µL of the supernatant was transferred to a new tube, and then sample index PCR was carried out.

30 µL of a sample index PCR mix prepared by mixing 8 µL of nuclease-free water, 50 µL of an amplification master mix, and 2 µL of SI-PCR Primer was added to 60 µL of each sample, and 10 µL of individual Chromium i7 Sample Index was added to each tube. After mixing using a pipette, spin down was performed and sample index PCR was performed. PCR was performed in a thermal cycler in which a lid temperature was set to 105 °C at 98 °C for 45 seconds, repeated for 9 cycles under conditions of 98 °C for 20 seconds, 54 °C for 30 seconds and 72 °C for 20 seconds, and then performed at 72 °C for 1 minute and at 4 °C hold.

80 µL of a SPRIselect reagent (Beckman Coulter, B23318) was added to the sample index PCR-completed sample, and mixed using a pipette. After incubation at room temperature for 5 minutes, the tube was placed in the high position of the magnet and the supernatant was removed. 30 seconds after putting 200 µL of 80% ethanol (VWR, E193-500mL) into the pellet, a washing process of removing ethanol was performed a total of three times. After centrifugation, the tube was placed in the low position of the magnet, the remaining ethanol was removed and air drying was carried out for 1 minute. After the removal of the magnet, 35.5 µL of Buffer EB was added and mixed using a pipette for 15 seconds, followed by incubation at room temperature for 2 minutes. After placing the tube in the low position of the magnet, 35 µL of the supernatant was transferred to a new tube. 1 µL of the sample contained in the new tube was prepared as a1:10 dilution, and QC and quantification were carried out using Agilent Tapestation. Samples for which the production of a single cell V(D)J enriched library was completed were subjected to HT-sequencing.

### 2.5. Construction of phage displayed scFv library

1st PCR and nested PCR were performed using Roche PLUS #03300226001. Primers used for PCR were prepared by mixing the sequences in Table 8 in the same ratio.

**[Table 8]**

| Name | Sequences (5'-3') | SEQ ID NO: | Sequences (5'-3') | SEQ ID NO: |
|---|---|---|---|---|
| 1^{st} For | CCATGGACTGGACCTGGAG | 338 | GCTTCCTCCTCCTTTGGATCTCTG | 339 |
| | ATGGACATACTTTGTTCCACG | 340 | CTSCTGCTCTGGGYTCC | 341 |
| | ATGGAGTTTGGGCTGAGCTGG | 342 | GTCCTGGGCCCAGTCTG | 343 |
| | ATGGAATTGGGGCTGAGCTG | 344 | CCTGGGCTCTGCTSCTCCTC | 345 |
| | ATGGAGTTGGGACTGAGCTG | 346 | GTTCTGTGGTTTCTTCTGAGCTG | 347 |
| | ATGGAACTGGGGCTCCG | 348 | GTGGCCTCCTATGWGCTGAC | 349 |
| | ATGAAACACCTGTGGTTCTTCC | 350 | ACAGGGTCTCTCTCCCAG | 351 |
| | ATGGGGTCAACCGCCATC | 352 | ACAGGTCTCTGTGCTCTGC | 353 |
| | ATGCAAGTGGGGGCCTC | 354 | ATTCYCAGRCTGTGGTGAC | 355 |
| | ATGTCTGTCTCCTTCCTCATC | 356 | GCTCACTGCACAGGTTCTTGG | 357 |
| | GCTCAGCTCCTGGGGCT | 358 | TCCCTCTCSCAGSCTGTG | 359 |
| | CTTCCTCCTGCTACTCTGGCTC | 360 | CAGTGGTCCAGGCAGGG | 361 |
| | TTTCTCTGTTGCTCTGGATCTCTG | 362 | | |
| 1^{st} Rev | GCCTGAGTTCCACGACACC | 363 | CTGTACTTTGGCCTCTCTGGGATAG | 364 |
| | CTGTCCGCTTTCGCTCCAG | 365 | TTCCACTGCTCRGGCGTCAG | 366 |
| Nested For (VH) | CAGGTSCAGCTGGTGCAGTCTGG | 367 | CAGGTGCAGCTGCAGGAGTCGG | 368 |
| | CAGGTCACCTTGAAGGAGTCTGGTCC | 369 | CAGGTGCAGCTACAGCAGTGGG | 370 |
| | GAGGTGCAGCTGGTGGAGTCTGG | 371 | GARGTGCAGCTGGTGCAGTCTGG | 372 |
| | GAGGTGCAGCTGTTGGAGTCTGG | 373 | CAGGTACAGCTGCAGCAGTCAGG | 374 |
| Nested For (VL) | CAGTCTGTGYTGACKCAGCC | 375 | CAGGCAGGGCTGACTCAGC | 376 |
| | CAGTCTGCCCTGACTCAGCC | 377 | GACATCCAGWTGACCCAGTCTCC | 378 |
| | TCCTATGAGCTGACWCAGCCA | 379 | GATATTGTGATGACCCAGWCTCCACTC | 380 |
| | TCTTCTGAGCTGACTCAGGACC | 381 | GAAATTGTGTTGACRCAGTCTCCAG | 382 |
| | CAGCYTGTGCTGACTCAATC | 383 | GACATCGTGATGACCCAGTCTCC | 384 |
| | CWGSCTGTGCTGACTCAGCC | 385 | GAAACGACACTCACGCAGTCTCC | 386 |
| | AATTTTATGCTGACTCAGCCCCAC | 387 | GAAATTGTGCTGACWCAGTCTCCAG | 388 |
| | CAGRCTGTGGTGACYCAGGAG | 389 | GACATTGTGCTGACCCAGTCTC | 390 |
| Nested Rev | GGGAAGTAGTCCTTGACCAGGC | 391 | GCACACAACAGAGGCAGTTCCAG | 392 |
| | TCACACTGAGTGGCTCCTGG | 393 | TGCTGGCCGCRTACTTGTTGTTG | 394 |

1st PCR was performed by mixing 10 µL of Expand High Fidelity^{PLUS} Reaction Buffer(5X) with 7.5 mM MgCl₂, 1 µL of dNTP (NEB, 10 mM), 2 µL of 1st For primer (10 µM), 2 µL of 1st Rev primer (10 µM), 30.5 µL of water, 0.5 µL of Expand High Fidelity^{PLUS} Enzyme blend, and 4 µL of cDNA. PCR was carried out repeatedly for 40 cycles under conditions of 94 °C for 2 minutes, 94 °C for 30 seconds, 53 °C for 1 minute, and 72 °C for 1 minute, and performed at 72 °C for 7 minutes. After PCR was completed, PCR cleanup was performed. 100 µL of NTI buffer was added, and then the resulting sample was loaded on a PCR clean-up column (MN, 740609.10) to run centrifugation. After removing the flow-through, a washing process of adding 700 µL of Buffer NT3(MN, 740609.10) and performing re-centrifugation was repeated twice, and centrifugation was then performed 1 minute to dry the membrane. Subsequently, the column was transferred to a new tube, 20 µL of Buffer NE (MN, 740609.10) was added to the column, and the column was incubated for 1 minute at room temperature and then centrifuged, thereby obtaining cDNA.

For amplification of heavy chain cDNA, 10 µL of Expand High Fidelity^{PLUS} Reaction Buffer(5X) with 7.5 mM MgCl₂, 1 µL of dNTP (NEB, 10 mM), 2 µL of nested For VH primer (10 µM), 2 µL of nested Rev primer (10 µM), 30.5 µL of water, 0.5 µL of Expand High Fidelity^{PLUS} Enzyme blend, and 4 µL of 1st PCR product were mixed to perform 1st PCR. PCR was performed at 94 °C for 2 minutes, repeated for 40 cycles of 94 °C for 30 seconds, 53°C for 1 minute and 72 °C for 1 minute, and then at 72 °C for 7 minutes. For amplification of light chain cDNA, in the same manner as above, 10 µL of Expand High Fidelity^{PLUS} Reaction Buffer(5X) with 7.5 mM MgCl₂, 1 µL of dNTP (NEB, 10 mM), 2 µL of nested For VH primer (10 µM), 2 µL of nested Rev primer (10 µM), 30.5 µL of water, 0.5 µL of Expand High Fidelity^{PLUS} Enzyme blend, and 4 µL of 1st PCR product were mixed to perform 1st PCR. PCR was performed at 94 °C for 2 minutes, repeated for 40 cycles of 94 °C for 30 seconds, 53°C for 1 minute and 72 °C for 1 minute, and then at 72 °C for 7 minutes. After PCR was completed, PCR cleanup was performed. 100 µL of NTI buffer (MN, 740609.10) was added, and the resulting sample was loaded on a PCR clean-up column (MN, 740609.10) to centrifuge. After removing the flow-through, a washing process of adding 700 µL of Buffer NT3 (MN, 740609.10) and performing re-centrifugation was repeated twice, and centrifugation was then performed 1 minute to dry the membrane. Subsequently, the column was transferred to a new tube, 20 µL of Buffer NE (MN, 740609.10) was added to the column, and the column was incubated for 1 minute at room temperature and then centrifuged, thereby obtaining cDNA.

VH/Vκ/V_{λ} PCR and scFv overlapping PCR were performed using Roche PLUS #03300226001. Primers used for PCR were prepared by mixing the sequences in Table 9 in the same ratio.

**[Table 9]**

| Name | Sequences (5'-3') | SEQ ID NO: | Sequences (5'-3') | SEQ ID NO: |
|---|---|---|---|---|
| VH For | ccgtggcccaggcggccCAGGTGCAGCTGG TGCAGTCT | 395 | ccgtggcccaggcggccCAGGTGCAGCTGG TGGAGTCT | 396 |
| | ccgtggcccaggcggccCAGGTYCAGCTKG TGCAGTCT | 397 | ccgtggcccaggcggccGAGGTACAGCTCG TGGAGTCC | 398 |
| | ccgtggcccaggcggccCAGGTTCAGCTGG TGCAGTCT | 399 | ccgtggcccaggcggccAGGTGGAGCTGAT AGAGTCC | 400 |
| | ccgtggcccaggcggccCAGGTCCAGCTGG TACAGTCT | 401 | ccgtggcccaggcggccCAGGTRCAGCTGG TGGAGTCT | 402 |
| | ccgtggcccaggcggccCAGGTCCAGCTGG TGCAGTCT | 403 | ccgtggcccaggcggccGAGGATCAGCTGG TGGAGTCT | 404 |
| | ccgtggcccaggcggccCAGATGCAGCTGG TGCAGTCT | 405 | ccgtggcccaggcggccGAGGTGCAGCTGG TGGAGWCT | 406 |
| | ccgtggcccaggcggccCAAATGCAGCTGG TGCAGTCT | 407 | ccgtggcccaggcggccSAGGTGCAGCTGG TGGAGTCT | 408 |
| | ccgtggcccaggcggccGAGGTCCAGCTGG TACAGTCT | 409 | ccgtggcccaggcggccGAGGTGCAGCTGG TGGAGTCY | 410 |
| | ccgtggcccaggcggccCAGRTCACCTTGA AGGAGTCT | 411 | ccgtggcccaggcggccCAGGTGCAGCTGC AGGAGTYG | 412 |
| | ccgtggcccaggcggccCAGGTCACCTTGA AGGAGTCT | 413 | ccgtggcccaggcggccCAGGTGCAGCTGC AGGAGTCG | 414 |
| | ccgtggcccaggcggccCAGGTCACCTTGA RGGAGTCT | 415 | ccgtggcccaggcggccCAGCTGCAGCTGC AGGAGTCC | 416 |
| | ccgtggcccaggcggccGAGGTGCAGCTGG TGGAGTCT | 417 | ccgtggcccaggcggccCAGGTGCAGCTGC AGGASTCG | 418 |
| | ccgtggcccaggcggccGAAGTGCAGCTGG TGGAGTCT | 419 | ccgtggcccaggcggccCAGGTGCRGCTGC AGGAGTCG | 420 |
| | ccgtggcccaggcggccCAGGTGCAGCTGK TGGAGTCT | 421 | ccgtggcccaggcggccCAGGTGCAGCTRC ARSAGTSG | 422 |
| | ccgtggcccaggcggccGAGGTGCAKCTGG TGGAGTCT | 423 | ccgtggcccaggcggccCAGSTGCAGCTGC AGGAGTCG | 424 |
| | ccgtggcccaggcggccGAGGTRCARCTGG TGGAGTCT | 425 | ccgtggcccaggcggccGAAGTGCAGCTGG TGCAGTCY | 426 |
| | ccgtggcccaggcggccACAGTGCAGCTGG TGGAGTCT | 427 | ccgtggcccaggcggccGAGGTGCAGCTGG TGCAGTCT | 428 |
| | ccgtggcccaggcggccGAGGTGCARCTGG TGGAGTCT | 429 | ccgtggcccaggcggccCAGGTACAGCTGC AGCAGTCA | 430 |
| | ccgtggcccaggcggccGAGGTGCAGCTGK TGGAGTCT | 431 | ccgtggcccaggcggccCAGGTGCAGCTGG TGCAATCT | 432 |
| | ccgtggcccaggcggccGAGAYGCAGCTGG TGGAGTCT | 433 | ccgtggcccaggcggccCTGCAGCTGGTGC AGTCTGGG | 434 |
| | ccgtggcccaggcggccGAGGTGGAGCTGA TAGAGCCC | 435 | ccgtggcccaggcggccCAGGTGCAGCTGG TGCAGTCT | 436 |
| JH Rev | gcctgaaccacctccgccagatccgccacctccTGAG GAGAC GGTGACCAGGGTG | 437 | gcctgaaccacctccgccagatccgccacctccTGAG GAGAC GGTGACCAGGGT | 438 |
| | gcctgaaccacctccgccagatccgccacctccTGAG GAGAC AGTGACCAGGGTGC | 439 | gcctgaaccacctccgccagatccgccacctccTGAG GAGACGGTGACCGTGGTC | 440 |
| | gcctgaaccacctccgccagatccgccacctccTGAA GAGAC GGTGACCATTGTCCCTT | 441 | | |
| Vκ For | TCTGGCGGAGGTGGTTCAGGCGGT GGAGGCTCGGACATCCAGATGACC | 442 | TCTGGCGGAGGTGGTTCAGGCGGT GGAGGCTCGGATGTTGTGATGACTC | 443 |
| | CAGTCTCCT | | AGTCTCCA | |
| | TCTGGCGGAGGTGGTTCAGGCGGT GGAGGCTCGGCCATCCAGATGACC CAGTCTCCA | 444 | TCTGGCGGAGGTGGTTCAGGCGGT GGAGGCTCGGAGATTGTGATGACC CAGACTCCA | 445 |
| | TCTGGCGGAGGTGGTTCAGGCGGT GGAGGCTCGGYCATCYGGATGACC CAGTCTCCA | 446 | TCTGGCGGAGGTGGTTCAGGCGGT GGAGGCTCGGAAATTGTAATGACA CAGTCTCAA | 447 |
| | TCTGGCGGAGGTGGTTCAGGCGGT GGAGGCTCGGACATCCAGTTGACC CAGTCTCCA | 448 | TCTGGCGGAGGTGGTTCAGGCGGT GGAGGCTCGGAAATTGTGTTGACA CAGTCTCCA | 449 |
| | TCTGGCGGAGGTGGTTCAGGCGGT GGAGGCTCGGACATCCAGATGACC CAGTCTCCA | 450 | TCTGGCGGAGGTGGTTCAGGCGGT GGAGGCTCGGAAATAGTGATGASG CAGTCTCCA | 451 |
| | TCTGGCGGAGGTGGTTCAGGCGGT GGAGGCTCGGCCATCCAGTTGACC CAGTCTCCA | 452 | TCTGGCGGAGGTGGTTCAGGCGGT GGAGGCTCGGAAATTGTGTTGACR CAGTCTCCA | 453 |
| | TCTGGCGGAGGTGGTTCAGGCGGT GGAGGCTCGRACATCCAGATGACC CAGTCTCCA | 454 | TCTGGCGGAGGTGGTTCAGGCGGT GGAGGCTCGGACATCGTGATGACC CAGTCTCCA | 455 |
| | TCTGGCGGAGGTGGTTCAGGCGGT GGAGGCTCGGACATCCAGATGAYC CAGTCTCCA | 456 | TCTGGCGGAGGTGGTTCAGGCGGT GGAGGCTCGGAAACGACACTCACG CAGTCTCCA | 457 |
| | TCTGGCGGAGGTGGTTCAGGCGGT GGAGGCTCGGACATCCAGATGAYC CAGTCTCCA | 458 | TCTGGCGGAGGTGGTTCAGGCGGT GGAGGCTCGGAAATTGTGCTGACT CAGTCTCCA | 459 |
| | TCTGGCGGAGGTGGTTCAGGCGGT GGAGGCTCGGATATTGTGATGACC CAGCATCTG | 460 | TCTGGCGGAGGTGGTTCAGGCGGT GGAGGCTCGGATGTTGTGATGACA CAGTCTCCA | 461 |
| | TCTGGCGGAGGTGGTTCAGGCGGT GGAGGCTCGGATATTGTGATGACC CAGACTCCA | 462 | TCTGGCGGAGGTGGTTCAGGCGGT GGAGGCTCGGACATTGTGCTGACC CAGTCTCCA | 463 |
| | TCTGGCGGAGGTGGTTCAGGCGGT GGAGGCTCGGATATTGTGATGACTC AGTCTCCA | 464 | | |
| Jκ Rev | TGCTGGCCGGCCTGGCCTCGTTTGA TCTCCAGCTTGGTCCCCTG | 465 | TGCTGGCCGGCCTGGCCTCGTTTGA TCTCCACCTTGGTCCCTCC | 466 |
| | TGCTGGCCGGCCTGGCCTCGTTTGA TATCCACTTTGGTCCCAGGGCC | 467 | TGCTGGCCGGCCTGGCCTCGTTTAA TCTCCAGTCGTGTCCCTTGGCC | 468 |
| V_{λ} For | TCTGGCGGAGGTGGTTCAGGCGGT GGAGGCTCGCAGTCTGTGCTGACTC AGCCACC | 469 | TCTGGCGGAGGTGGTTCAGGCGGT GGAGGCTCGCAGTCTGCCCTGACTC AGCCTCA | 470 |
| | TCTGGCGGAGGTGGTTCAGGCGGT GGAGGCTCGCAGTCTGTSSTGACGC AGCCG | 471 | TCTGGCGGAGGTGGTTCAGGCGGT GGAGGCTCGTCCTATGAGCTGAYR CAGCCACCC | 472 |
| | TCTGGCGGAGGTGGTTCAGGCGGT GGAGGCTCGCAGTCTGTGTTGACGC AGCCGC | 473 | TCTGGCGGAGGTGGTTCAGGCGGT GGAGGCTCGTCCTCCATGCTGACTC AGGAGCCA | 474 |
| | TCTGGCGGAGGTGGTTCAGGCGGT | 475 | TCTGGCGGAGGTGGTTCAGGCGGT | 476 |
| | GGAGGCTCGCAGYCTGTGCTGACT CAGCCAC | | GGAGGCTCGTCCTATGAGCTGACA CAGCCAYCCTC | |
| | TCTGGCGGAGGTGGTTCAGGCGGT GGAGGCTCGCAGTCTGTGYTGACG CAGCCG | 477 | TCTGGCGGAGGTGGTTCAGGCGGT GGAGGCTCGACATATGAGCTGTCTC AGCCACCC | 478 |
| | TCTGGCGGAGGTGGTTCAGGCGGT GGAGGCTCGCAGCCTGTGCTGACTC AGATGACC | 479 | TCTGGCGGAGGTGGTTCAGGCGGT GGAGGCTCGTCCTCTGAGCTGAGTC AGGAGCCT | 480 |
| | TCTGGCGGAGGTGGTTCAGGCGGT GGAGGCTCGCAGTCTGCCCTGAYTC AGCCTC | 481 | TCTGGCGGAGGTGGTTCAGGCGGT GGAGGCTCGTCCTCTGGGCCAACTC AGGTGC | 482 |
| | TCTGGCGGAGGTGGTTCAGGCGGT GGAGGCTCGCARTCTGCCCTGACTC AGCCTS | 483 | TCTGGCGGAGGTGGTTCAGGCGGT GGAGGCTCGCTGCCTGTGCTGACTC AGCCC | 484 |
| | TCTGGCGGAGGTGGTTCAGGCGGT GGAGGCTCGCAGTCTGTTCTGACTC AGCCTCGC | 485 | TCTGGCGGAGGTGGTTCAGGCGGT GGAGGCTCGCAGCCTGTGCTGACTC AATCATCC | 486 |
| | TCTGGCGGAGGTGGTTCAGGCGGT GGAGGCTCGTCCTATGAGCTGACTC AGCCACCC | 487 | TCTGGCGGAGGTGGTTCAGGCGGT GGAGGCTCGCAGCTTGTGCTGACTC AATCGCCC | 488 |
| | TCTGGCGGAGGTGGTTCAGGCGGT GGAGGCTCGTCCTCTGAGCTGACTC AGCTGCCT | 489 | TCTGGCGGAGGTGGTTCAGGCGGT GGAGGCTCGCAGCCTGTGCTGACTC AGCCR | 490 |
| | TCTGGCGGAGGTGGTTCAGGCGGT GGAGGCTCGTCCTATGAGCTTACAC AGCCACCCTC | 491 | TCTGGCGGAGGTGGTTCAGGCGGT GGAGGCTCGAATTTTATGCTGACTC AGCCCCACTCT | 492 |
| | TCTGGCGGAGGTGGTTCAGGCGGT GGAGGCTCGTCCTCTGAGCGGACTC AGTTGCCT | 493 | TCTGGCGGAGGTGGTTCAGGCGGT GGAGGCTCGCAGRCTGTGGTGACT CAGGAGCC | 494 |
| | TCTGGCGGAGGTGGTTCAGGCGGT GGAGGCTCGTCCTATGAGCTGACTC AGCCACTCTC | 495 | TCTGGCGGAGGTGGTTCAGGCGGT GGAGGCTCGCAGACTGTGGTGACC CAGGAG | 496 |
| | TCTGGCGGAGGTGGTTCAGGCGGT GGAGGCTCGTCCTATGAGCTGACW CAGCCACMC | 497 | TCTGGCGGAGGTGGTTCAGGCGGT GGAGGCTCGCAGCCTGTGCTGACTC AGCCA | 498 |
| | TCTGGCGGAGGTGGTTCAGGCGGT GGAGGCTCGTCTTCTGAGCTGACTC AGGACCCTG | 499 | TCTGGCGGAGGTGGTTCAGGCGGT GGAGGCTCGCAGGCAGGGCTGACT CAGCCA | 500 |
| | TCTGGCGGAGGTGGTTCAGGCGGT GGAGGCTCGTCCTATGTGCTGACW CAGCYACCC | 501 | TCTGGCGGAGGTGGTTCAGGCGGT GGAGGCTCGCGGCCCGTGCTGACT CAG | 502 |
| J_{λ} Rev | TGCTGGCCGGCCTGGCCGCCTAGG ACGGTGACCTTGGTCCCAGT | 503 | TGCTGGCCGGCCTGGCCGCCTAGG ACGGTCAGCTCSGTCCC | 504 |
| | TGCTGGCCGGCCTGGCCGCCTAGG ACGGTCAGCTTGGTCCCTC | 505 | TGCTGGCCGGCCTGGCCGCCGAGG ACGGTCACCTTGGTGCCA | 506 |
| | TGCTGGCCGGCCTGGCCGCCTAAA ATGATCAGCTGGGTTCCTCCACCAA ATA | 507 | TGCTGGCCGGCCTGGCCGCCGAGG YCGGTCAGCTGGGTGC | 508 |

For the amplification of VH fragments, 10 µL of Expand High Fidelity^{PLUS} Reaction Buffer(5X) with 7.5 mM MgCl₂, 1 µL of dNTP (NEB, 10 mM), 2 µL of VH For VH primer (10 µM), 2 µL of JH Rev primer (10 µM), 32.5 µL of water, 0.5 µL of Expand High Fidelity^{PLUS} Enzyme blend, and 2 µL of Heavy chain cDNA were mixed to perform PCR. PCR was performed at 94 °C for 2 minutes, repeated for 30 cycles of 94 °C for 30 seconds, 53°C for 30 seconds and 72 °C for 1 minute, and then at 72 °C for 7 minutes. PCR for a Vκ fragment (primer: Vκ/Jκ, cDNA: light chain) and a V_{λ} fragment (primer: V_{λ}/J_{λ} cDNA: light chain) was also performed in the same manner as for the VH fragment.

After PCR was completed, gel extraction was performed. The resulting sample was loaded on 0.7% agarose gel, and a gel having a size of 350 bp was cut. 200 µL of NTI buffer was added per 100 mg of the cut gel, and the resulting gel was located on a column to centrifuge. After removing the flow-through, a washing process of adding 700 µL of Buffer NT3 and performing re-centrifugation was repeated twice, and centrifugation was then performed 1 minute to dry the membrane. Subsequently, the column was transferred to a new tube, 20 µL of Buffer NE was added to the column, and the column was incubated for 1 minute at room temperature and then centrifuged, thereby obtaining amplified fragment DNA.

For VH-Vκ scFv overlapping PCR, 10 µL of Expand High Fidelity^{PLUS} Reaction Buffer(5X) with 7.5 mM MgCl₂, 1 µL of dNTP (NEB, 10 mM), 2 µL of VH For VH primer (10 µM), 2 µL of Jκ Rev primer (10 µM), 30.5 µL of water, 0.5 µL of Expand High Fidelity^{PLUS} Enzyme blend, 2 µL of VH fragments, and 2 µL of Vκ fragments were mixed to perform PCR. PCR was performed at 94 °C for 2 minutes, repeated for 30 cycles of 94 °C for 30 seconds, 53°C for 1 minute and 72 °C for 1 minute, and then at 72 °C for 7 minutes. VH-V_{λ} scFv overlapping PCR was also performed in the same manner as above. Here, as the fragments, the VH fragment and the V_{λ} fragment were used, and as the primers, VH For and J_{λ} Rev were used.

After PCR was completed, gel extraction was performed. The resulting sample was loaded on 0.7% agarose gel, and a gel having a size of 750-800 bp was cut. 200 µL of NTI buffer was added per 100 mg of the cut gel, and the resulting gel was located on a column to centrifuge. After removing the flow-through, a washing process of adding 700 µL of Buffer NT3 (MN, 740609.10) and performing re-centrifugation was repeated twice, and centrifugation was then performed 1 minute to dry the membrane. Subsequently, the column was transferred to a new tube, 20 µL of Buffer NE was added to the column, and the column was incubated for 1 minute at room temperature and then centrifuged, thereby obtaining amplified scFv insert DNA.

1.4 µg pcomb3-XTT vector linearized by SfiI (Sigma Aldrich, 11288059001) treatment, 700 ng of scFv insert DNA, 20 µL of 5X ligase reaction buffer, and 5 µL of T4 DNA Ligase (Invitrogen, 15224017) were mixed, autoclaved distilled water was added to a total volume of 100 µL, and then the resulting mixture was incubated at room temperature overnight.

### 3. Selection and manufacture of anti-RSV antibody

### 3.1. Panning of phage-displayed scFv library

Library panning was performed by an immunotube method. 5 µg/mL of DS-Cav1 (in-house) was put into an immunotube (e. g. Nunc MaxiSorp 444202), and incubated at 4 °C overnight. The next day, an antigen was removed from a DS-Cav1-coated tube, the immunotube was washed twice with PBS-Tween20 (0.1%), fully filled with 2% skim milk and then blocked at room temperature for 2 hours. After blocking, a blocking solution was removed from the immunotube, and 1 mL of the blocked phage library was added to incubate at least1 hour at 37 °C. The phage library was removed from the immunotube and then the immunotube was washed five times with 1 mL of PBS-Tween20 (0.1%).

To recover the phages binding to the immunotube, 1 mL of 10 mM glycine-HCl pH 1.5 + 1% BSA was added, and the phages were incubated for 10 minutes. The recovered phages were transferred to a 50 ml Falcon tube and immediately neutralized with 150 µL of 1 M Tris-HCl (pH 8.8). XL1-blue was grown to OD-₆₀₀ = 0.5 ~ 1.0 in 10 mL of a SB medium, and 1.15 mL of the neutralized phages were added for standing incubation at 37 °C for 30 minutes. After 30 minutes, incubation was further performed at 37 °C and 120 rpm.

To calculate the phage output, XL1-blue was diluted 1:10, 1:100, or 1: 1000, and 100 µL each of the Xl1-blue was spread on a SB plate with 100 mg/mL carbenicillin to incubate at 37 °C overnight. The aforementioned XL1-blue was grown with 10 µL carbenicillin (100 mg/mL) and 10 µL tetracycline (50 mg/mL) at 37 °C and 250 rpm for 2 hours. After two hours, a helper phage (10¹² pfu) was added and standing-incubated at 37 °C for 30 minutes. After 30 minutes, incubation was further performed at 37 °C and 120 rpm. In addition, cells grown with 100 µL kanamycin (50 mg/mL) and 100 µL carbenicillin (100 mg/mL) in 90 mL of a SB medium were transferred to a 500 ml flask and grown at 37 °C and 250 rpm overnight. The next day, the cultured cells were centrifuged at 3,500 rpm for 15 minutes, and the supernatant was transferred to a new tube. For phage precipitation, 20 mL of 20% PEG + 2.5 M NaCl was added and the cells were incubated on ice for 1 hour. The cells were centrifuged at 8000 rpm for 40 minutes to remove the supernatant. 1 mL of phage PBSB (PBS+1% BSA) on the tube wall was resuspended and transferred to an e-tube and further centrifuged at 13000 rpm for 10 minutes, thereby recovering the supernatant. The above process was repeated by lowering to 3 µg/mL of DS-Cav1 (2nd panning).

### 3.2. Single-colony screening

96 individual clones collected from a colony obtained by library panning were seeded in a 96-well cell culture plate (U-bottom) filled with 500 µL SBC (SB+ carbenicillin 100 µg/mL). Negative clones were seeded, and each plate was covered with a breathable sealing film to grow the cells at 250 rpm and 37 °C to OD₆₀₀ 0.8 ~ 1.0. After one hour, 50 µL SBC + 10 mm IPTG was further added, and incubation was further performed at 250 rpm and 25 °C overnight. The next day, the supernatant was recovered by centrifugation at 3200 ×g for 20 minutes. The recovered supernatant (scFv) was stored at 4 °C.

To select scFv (1st, 2nd panning output) specifically binding to DS-Cav1, screening was performed by ELISA. An ELISA plate was coated with 50 µL of 3 µg/m DS-Cav1 (in-house) at 4 °C overnight. The next day, the DS-Cav1 on the ELISA plate was removed and washed with PBS + Tween 20 0.05% three times. Each antigen-coated well was blocked with 200 µL of PBS + skim milk 5% at 37 °C for 1 hour. The plate was washed four times with PBS + Tween 20 0.05%. 50 µL of scFv was added to each well to allow binding at 37 °C for 1 hour. Again, the plate was washed with PBS + Tween 20 0.05% four times. 50 µL of an HRP-conjugated anti-HA antibody (3000:1) was added to each well and incubated at 37 °C for 1 hour. The plate was washed with PBS + Tween 20 0.05% four times. 50 µL of a TMB solution was added to each well and the cells were incubated for 1 to 15 minutes. 50 µL of a stop solution was added to each well to stop the reaction. After measurement with an ELISA reader, only wells with OD₄₅₀ ≥ 0.5 were selected. The selected clone was inoculated into 3 mL of SB medium and grown at 250 rpm and 37 °C overnight. The next day, DNA sequencing of the cultured cells was requested.

### 3.3. IgG expression vector cloning

### Insert production

Insert production was performed by a method using gBlock and a PCR method. The cases using gBlock include a clone in which an open reading frame of scFv is broken and a clone in which a framework different from a germline is confirmed. The clone having a framework different from a germline was manufactured in two formed of a sequence before editing and a sequence after editing. The sequence before editing was prepared by PCR and gBlock was used as the sequence after editing. Since the clone was modified after editing, it was represented as m after the clone numbering name. When there is a clone in which any one of a heavy chain and a light chain is modified, there is an m-attached clone.

In addition, by the PCR method, an insert was obtained using scFv as a template. PCR was performed after 10 µL of Expand High Fidelity^{PLUS} Reaction Buffer(5x) with 7.5 mM MgCl₂, 1 µL of dNTP (NEB, 10 mm), 2 µL of HC/LC/KC forward primer (Heavy chain/Lambda chain/Kappa chain forward primer, 10 µm), 2 µL of HC/LC/KC reverse primer (HC/LC/KC reverse primer, 10 µm), 30.5 µL of water, and 0.5 µL, scFv, and 2 µL of Expand High Fidelity^{PLUS} Enzyme blend were mixed. PCR was performed at 94 °C for 2 minutes, repeated for 30 cycles under conditions of 94 °C for 30 seconds, 53 °C for 1 minute and 72 °C for 1 minute, and at 72 °C for 7 minutes. HC/LC/KC PCR was performed by changing only a template and a primer under the same conditions. A total of 63 clones were performed in the same manner. Additionally, from the NGS sequencing result for the chromium B cell library, 25 types of IgGIs and 6 types of IgG2s were obtained. Inserts for the obtained 31 types of IgGs were manufactured by gBlock synthesis.

**[Table 10]**

| PCR primer sequences for cloning IgG expression vectors | | | |
|---|---|---|---|
| | Name | Sequences (5'-3') | SE Q ID NO: |
| Heavy chain | HC_F1 | TGCTGTGGGTGAGTGGTACCTGTGGGGAGGTGCAGCTGGTGGAGTCTG | 509 |
| | HC_F2 | TGCTGTGGGTGAGTGGTACCTGTGGGCAGGTCACCTTGAAGGAGTCTGGG | 510 |
| | HC_F3 | TGCTGTGGGTGAGTGGTACCTGTGGGCAGGTGCAGCTGGTGCAGTCTG | 511 |
| | HC_R1 | GGGGGAAGACCGATGGGCCCTTGGTGGAGGCTGAGGAGACGGTGACCAGGG TTC | 512 |
| | HC_R2 | GGGGGAAGACCGATGGGCCCTTGGTGGAGGCTGAGGAGACAGTGACCAGGG TTCC | 513 |
| | HC_R3 | GGGGGAAGACCGATGGGCCCTTGGTGGAGGCTGAGGAGACGGTGACCGTGG TTC | 514 |
| | HC_R4 | GGGGGAAGACCGATGGGCCCTTGGTGGAGGCTGAGGAGACGGTGACCGTGG TC | 515 |
| Lambd a chain | LC_F1 | TGCTGTGGGTGAGTGGTACCTGTGGGCAGTCTGTGCTGACGCAGCCG | 516 |
| | LC_F2 | TGCTGTGGGTGAGTGGTACCTGTGGGTCCTATGAGCTGACTCAGCCAC | 517 |
| | LC_F3 | TGCTGTGGGTGAGTGGTACCTGTGGGCAGCCTGTGCTGACTCAGCCAC | 518 |
| | LC_F4 | TGCTGTGGGTGAGTGGTACCTGTGGGCAGACTGTGGTGACTCAGGAGCC | 519 |
| | LC_F5 | TGCTGTGGGTGAGTGGTACCTGTGGGCAGTCTGCCCTGACTCAGCCTG | 520 |
| | LC_R1 | GTGGGATTGGCCTTAGGTTGGCCTAGGACGGTGACCTTGGTC | 521 |
| | LC_R2 | GTGGGATTGGCCTTAGGTTGGCCTAGGACGGTCAGCTTGGTC | 522 |
| Kappa chain | KC_F1 | TGCTGTGGGTGAGTGGTACCTGTGGGGATGTTGTGATGACTCAGTCTCCA | 523 |
| | KC_F2 | TGCTGTGGGTGAGTGGTACCTGTGGGGATATTGTGATGACCCAGACTCCA | 524 |
| | KC_F3 | TGCTGTGGGTGAGTGGTACCTGTGGGGACATCCAGATGACCCAGTCTCCA | 525 |
| | KC_F4 | TGCTGTGGGTGAGTGGTACCTGTGGGGAGATTGTGCTGACCCAGTCTCCA | 526 |
| | KC_F5 | TGCTGTGGGTGAGTGGTACCTGTGGGGACATCCAGTTGACCCAGTCTCCA | 527 |
| | KC_R1 | ACGCTTGGAGCGGCCACCGTACGTTTGATCTCCAGCTTGGTCCC | 528 |
| | KC_R2 | ACGCTTGGAGCGGCCACCGTACGTTTAATCTCCAGTCGTGTCCCTTG | 529 |
| | KC_R3 | ACGCTTGGAGCGGCCACCGTACGTTTGATCTCCACCTTGGTCCC | 530 |

**[Table 11]**

| PCR sets for cloning IgG expression vectors | | | | | |
|---|---|---|---|---|---|
| # | Clone | Heavy chain F | Heavy chain R | Light chain F | Light chain R |
| 1 | 2G4 | HC_F1 | HC_R1 | LC_F1 | LC_R1 |
| 2 | 2H3 | HC_F1 | HC_R1 | LC_F1 | LC_R1 |
| 3 | 4H1 | HC_F1 | HC_R1 | LC_F1 | LC_R1 |
| 4 | 4E3 | HC_F1 | HC_R1 | LC_F1 | LC_R1 |
| 5 | 4H5 | HC_F1 | HC_R1 | - | - |
| 6 | 2E7 | HC_F1 | HC_R1 | LC_F2 | LC_R1 |
| 7 | 2H2 | HC_F1 | HC_R1 | LC_F1 | LC_R1 |
| 8 | 12A1 | HC_F1 | HC_R1 | LC_F1 | LC_R1 |
| 9 | 2H1 | HC_F2 | HC_R1 | LC_F1 | LC_R1 |
| 10 | 4G4 | HC_F1 | HC_R1 | LC_F1 | LC_R1 |
| 11 | 12E9 | HC_F1 | HC_R1 | LC_F4 | LC_R1 |
| 12 | 4F11 | HC_F1 | HC_R1 | LC_F3 | LC_R1 |
| 13 | 10E7 | HC_F1 | HC_R1 | LC_F1 | LC_R1 |
| 14 | 2E9 | HC_F2 | HC_R1 | LC_F1 | LC_R1 |
| 15 | 12B11 | HC_F1 | HC_R2 | LC_F2 | LC_R1 |
| 16 | 3H8 | HC_F1 | HC_R3 | LC_F3 | LC_R1 |
| 17 | 4E1 | HC_F1 | HC_R1 | LC_F2 | LC_R1 |
| 18 | 2H9 | HC_F2 | HC_R1 | LC_F2 | LC_R1 |
| 19 | 12C11 | HC_F1 | HC_R1 | LC_F1 | LC_R1 |
| 20 | 3E10 | HC_F1 | HC_R1 | LC_F3 | LC_R1 |
| 21 | 2G1 | HC_F2 | HC_R1 | LC_F1 | LC_R1 |
| 22 | 1E6 | HC_F1 | HC_R2 | LC_F1 | LC_R1 |
| 23 | 2H11 | - | - | - | - |
| 24 | 2F1 | HC_F1 | HC_R1 | LC_F1 | LC_R2 |
| 25 | 13C2 | HC_F1 | HC_R1 | LC_F1 | LC_R1 |
| 26 | 2H8 | HC_F1 | HC_R1 | LC_F3 | LC_R1 |
| 27 | 10A6 | HC_F1 | HC_R1 | LC_F2 | LC_R1 |
| 28 | 4G2 | HC_F1 | HC_R1 | LC_F3 | LC_R1 |
| 29 | 2G11 | HC_F1 | HC_R1 | LC_F1 | LC_R1 |
| 30 | 11H9 | - | - | - | - |
| 31 | 12C9 | - | - | - | - |
| 32 | 7A10 | - | - | - | - |
| 33 | 4G1 | HC_F2 | HC_R1 | LC_F1 | LC_R1 |
| 34 | 7C6 | HC_F1 | HC_R1 | - | - |
| 35 | 7A7 | - | - | - | - |
| 36 | 6A9 | - | - | - | - |
| 37 | 12B8 | - | - | - | - |
| 38 | 12F7 | - | - | - | - |
| 39 | 7A11 | - | - | - | - |
| 40 | 4H11 | HC_F3 | HC_R1 | LC_F2 | LC_R2 |
| 41 | 2G3 | HC_F3 | HC_R1 | LC_F2 | LC_R2 |
| 42 | 12C10 | HC_F3 | HC_R1 | LC_F3 | LC_R2 |
| 43 | 3E8 | HC_F3 | HC_R2 | LC_F3 | LC_R2 |
| 44 | 7D1 | HC_F3 | HC_R1 | LC_F1 | LC_R2 |
| 45 | 4G5 | HC_F3 | HC_R1 | LC_F2 | LC_R2 |
| 46 | 2E8 | HC_F3 | HC_R1 | LC_F2 | LC_R2 |
| 47 | 13H9 | - | - | LC_F2 | LC_R2 |
| 48 | 4E9 | HC_F3 | HC_R1 | LC_F1 | LC_R2 |
| 49 | 4H7 | HC_F3 | HC_R1 | LC_F2 | LC_R2 |
| 50 | 2F8 | HC_F3 | HC_R1 | LC_F2 | LC_R2 |
| 51 | 3E7 | HC_F1 | HC_R2 | - | - |
| 52 | 9H4 | HC_F3 | HC_R4 | KC_F4 | KC_R2 |
| 53 | 8B1 | HC_F3 | HC_R4 | KC_F1 | KC_R1 |
| 54 | 7G12 | - | - | - | - |
| 55 | 12F11 | - | - | KC_F1 | KC_R1 |
| 56 | 5B12 | - | - | - | - |
| 57 | 8C3 | - | - | - | - |
| 58 | 2F4 | HC_F3 | HC_R4 | KC_F2 | KC_R3 |
| 59 | 1C10 | HC_F3 | HC_R1 | KC_F2 | KC_R1 |
| 60 | 5E9 | HC_F3 | HC_R3 | KC_F1 | KC_R2 |
| 61 | 5C9 | HC_F3 | HC_R3 | KC_F1 | KC_R1 |
| 62 | 5C7 | - | - | - | - |
| 63 | 8A11 | HC_F3 | HC_R1 | KC_F1 | KC_R1 |

### Vector

As IgG conversion vectors, a pCIW_heavy chain, a pCIW_lambda chain and a pCIW_kappa chain were prepared. The PCIW_heavy chain was digested with Apa l (NEB,R0114L)/Kpn l (NEB, R0142L). The pCIWlambda chain was digested with Bsu36i (NEB, R0524L)/kpn l (NEB, R0142L). The pCIW_kappa chain was digested with Bsiwi (NEB, r0553l)/kpn l (NEB, R0142L). After enzyme digestion, gel extraction was performed. The resulting product was loaded on a 0.7% agarose gel and then a gel with a size of ~3000 bp was cut. After adding a 3-fold larger amount QG buffer per g of the gel, the cut gel was dissolved at 50 °C. After the gel was added to a column and centrifuged, and a washing process of removing the flow-through, adding 750 µL of EB and performing centrifugation again was performed, centrifugation was performed for 1 minute to dry the membrane. Two minutes after the addition of 50 µL of EB to the membrane, the resulting product was recovered by centrifugation.

### Infusion

50 ng each of the linearized pCIW vectors (pCIW_heavy chain, pCIW_lambda, and pCIW_kappa), 25 ng of purified Heavy chain/Lambda chain/Kappa chain insert DNA, and 2 µL of 5X In-Fusion HD Enzyme Premix were mixed, autoclaved distilled water was added to a final volume of 10 µL, and then the resulting product was incubated at 50 °C for 30 minutes.

### Transformation

After addition of 2 µL of a ligation product to 50 µL of XLI-blue and mixing, the resulting mixture was transferred to a cuvette. Electro transformation conditions were set to 2500v, 200ω, and 25 µf, followed by application of an electric shock. After 1 mL of SB was resuspended to recover as many cells in the cuvette as possible, incubation was performed at 37 °C for 1 hour at 200 rpm. After incubation, the cells were allowed to settle, the supernatant was discarded, the pellet was resuspended at 100 µL, and then the resulting suspension was spread on a LB-carb plate.

### 3.4. Animal cell production

### Midi-prep

A cell pellet was resuspended with 4 mL of P1 buffer. After 4 mL of P2 buffer was added to invert 3 to 4 times, 3 minute-incubation was performed. The resulting product was transferred to a cartridge filter and incubated for 10 minutes. A residue was filtered from the sample in the cartridge filter using a piston, and only the supernatant was obtained. 2 mL of a binding buffer was added, the mixture was added to a column, and the column was washed with 700 µL of ETR buffer and 750 µL of PE buffer. Elution was performed using 200 µL of DDW.

### Transfection (based on 30 mL)

The day before expression, Expi293 cells were subcultured to 2 × 10⁶ cells/mL. On the day of expression, it was confirmed whether 3 × 10⁶ cells/mL or more of the cells were grown. One mixture containing 15 µg of a heavy chain and 15 µg of a light chain prepared in 1.5 mL of an Opti-MEM medium and a mixture containing 80 µL of ExpiFectamine in 1.5 mL of a medium were prepared and incubated for 5 minutes at room temperature. The two mixtures were mixed and incubated for 20 to 30 minutes. The resulting mixture was added to the prepared cells (25.5 mL). After 16 to 20 hours of expression, Enhancers 1 and 2 were added at 150 µL and 1.5 mL, respectively, and then expression was performed in a shaking culture for 5 to 7 days at 37 °C.

### IgG (based on 30 mL)

1 mL of 50% protein A resin washed with PBS was added to the expressed supernatant, and inverted at 4 °C for 2 hours and at room temperature for 30 minutes. After transferring to the purification column, the resin was washed with a 20-fold or more amount of an IgG binding buffer. Elution was performed with 500 µL of an elution buffer six times or more. For each vial, a concentration was measured at a wavelength of 280 nm, and then the measurement results were collected.

A desalting column was centrifuged at 1000 g for 2 minutes to filter a preservative. After adding a PBS buffer to an amount suitable for the column, centrifugation was performed at 1000 g for 2 minutes and then the column was washed repeatedly three times. After adding each sample, centrifugation was performed at 1000 g for 2 minutes, a concentration was measured using a wavelength, and then the measurement results were collected.

### SDS-PAGE assay

The sample and a 4X non-reducing sample buffer were mixed in a volume ratio of 3:1, heated at 90 °C for 3 minutes and then cooled. The prepared sample was loaded on a 4 to 12% Bis-Tris gel at 3 µg per well, and electrophoresis was performed. The gel was isolated and stained with a PageBlue Protein Staining Solution for 30 minutes or more, followed by destaining using DW and determining the size and purity of a protein.

### 3.5. Analysis of binding ability by ELISA

For the analysis of binding ability for 63 types of selected antibodies against DS-Cav1, ELISA was performed. 50 µL of 3 µg/m DS-Cav1 (In-house) was coated on an ELISA plate at 4 °C overnight. The next day, DS-Cav1 on the ELISA plate was removed and washed three times with PBS + Tween 20 0.05%. 200 µL of PBS + skim milk 5% was added to each well coated with an antigen for blocking at 37 °C for 1 hour. After blocking, the plate was washed four times with PBS + Tween 20 0.05%. Subsequently, 63 types of the selected antibodies IgGs were diluted 1/4 from 2 µM. The dilution was performed a total of 11 times, making 12 points. 50 µL of the diluted antibodies were put into each well and bound at 37 °C for 1 hour. The plate was washed again with PBS + Tween 20 0.05% four times. 50 µL of an HRP conjugated anti-Fc antibody (3000:1) was added to each well and incubated at 37 °C for 1 hour. The plate was washed with PBS + Tween 20 0.05% four times. 50 µL of a TMB solution was added to each well, and incubation was performed for 1 to 15 minutes. 50 µL of a stop solution was added to each well to stop the reaction. Only clones with OD₄₅₀ ≥ 0.5 were selected after measurement with an ELISA reader.

### 4. Anti-RSV neutralizing antibody screening: Analysis of antibody neutralizing activity using focus reduction neutralization test (FRNT)

The day before the experiment, A549 cells were prepared in a 96 well plate at 4.5 ×10⁴ cells/well. IgG supernatant dilutions were prepared to make 1:10, 1:100, and 1:1000 dilutions using infection media in a 96 deep well plate to a final volume of 300 µL. Titrated virus RSV A2 (ATCC, Cat# VR-1540) P3 stock was prepared by making a 1:125 dilution in infection media (final titers, first: 1:250, second: 1:500).

To a new 96 deep well plate, each of 200 µL of an IgG supernatant dilution and 200 µL of a virus dilution was mixed, and a neutralization reaction was performed at 37 °C in a 5% CO₂ incubator for 1 hour. A mixture obtained by neutralizing the prepared A549 cells was added at 25 µL /well, and incubated at 37 °C in a 5% CO₂ incubator for 2 hours (the plate was shaken every 15 minutes so as not to dry the cells). 200 µL of 0.8% methylcellulose was added to each well and the cells were cultured at 37 °C in a 5% CO₂ incubator for 3 to 4 days. Subsequently, overlay media were removed by suction and then the plate was washed with 300 µL/well of PBS once. 100% cold methanol was added at 100 µL/well, and the plate was covered with a wrap and stored at 4 °C.

After fixation for more than 4 hours, methanol was removed and washed twice with 300 µL of PBS. An RSV fusion protein antibody was diluted 1:1000 in infection media and added at 100 µL/well, followed by incubation for 2 hours at 37 °C. After washing three times with PBS, 100 uL of anti-human Fc IgG or antimouse IgG-HRP was added per well to be diluted 1:2000 in infection media, and incubated for 1 hour at 37 °C. After washing with PBS three times, the washing solution was thoroughly removed, and 100 µL of a TrueBlue substrate (KPL, 5510-0030) was added per well to develop color at room temperature. After color development, the plate was washed and dried, and then Ec50 was calculated by counting spots using an ELISPOT reader.

Sample layouts used in the first and second tests are shown in Table 12 (sample plate layout for first test) and Table 13 (sample plate layout for second test).

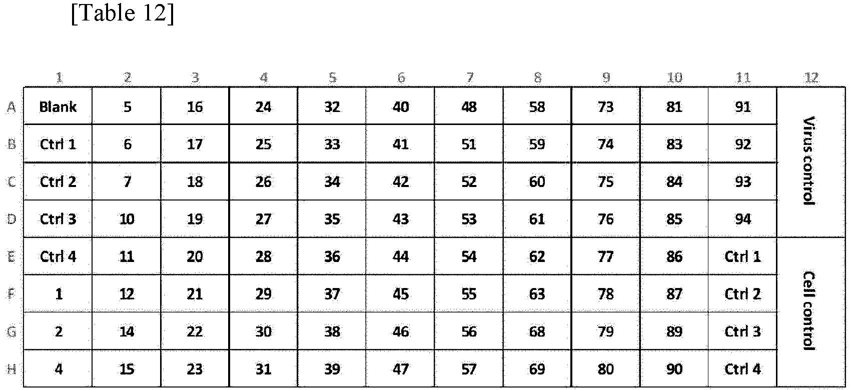

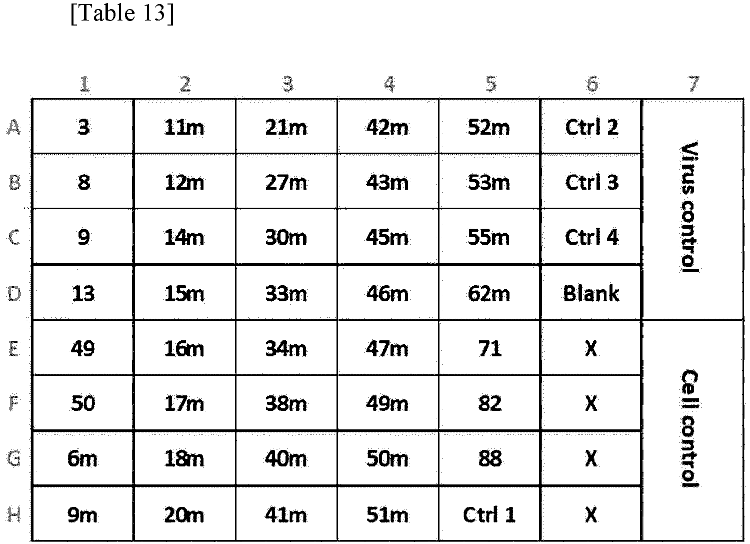

### 5. RSV neutralizing antibody measurement (FRNT) of selected antibody

Among antibody clones selected from a phage display library or NGS analysis, RSV neutralizing antibodies against the antibodies whose RSV neutralizing activity was primarily confirmed were measured through the above-described procedures. Antibody sample data used in this example is shown in the following tables.

**[Table 14]**

| # | Sample list |
|---|---|
| 1^{st} | 4, 5, 6, 7, 10, 11, 12, 14, 15, 16, 17, 18, 19, 20, 21, 22, 23, 26, 33, 38, 40, 42, 43, 45, 46, 47, 48, 51, 52, 53, 61, 62, 63 |
| 2^{nd} | 3, 8, 9, 13, 49 ,6m, 11m, 12m, 14m |
| 3^{rd} | 1, 2, 15m, 16m, 17m, 18m, 20m, 21m, 24, 25, 27, 27m, 28, 29, 33m, 38m, 40m, 41, 41m, 42m, 45m, 46m, 47m, 49m, 50m, 51m, 52m, 53m, 60, 62m, 84, 88 |
| 4^{th} | 30, 30m, 32, 34, 36, 39, 44, 55, 55m, 56, 57, 58, 66, 68, 69, 71, 74, 75, 76, 78, 79, 80, 81, 82, 85, 87, 89, 90, 93, 86 |
| 5^{th} | 9m, 35, 37, 50, 59, 91, 94 |
| 6^{th} (3^{rd} re-test) | 1, 2, 15m, 16m, 17m, 18m, 20m, 21m, 24, 25, 27, 27m, 28, 29, 33m, 38m, 40m, 41, 41m, 42m, 45m, 46m, 47m, 49m, 50m, 51m, 52m, 53m, 60, 62m, 84, 88 |

**[Table 15]**

| # | Clone | Name | Library | Expression (mg/L) | Kd (∼nM) |
|---|---|---|---|---|---|
| 1 | #1 | 2G4 | C-λ | 32 | 1.01 |
| 2 | #2 | 2H3 (2F5) | C-λ | 66 | 0.98 |
| 3 | #3 | 4H1 | C-λ | 75 | 1.73 |
| 4 | #4 | 4E3 | C-λ | 41 | 0.33 |
| 5 | #5 | 4H5 | C-λ | 297 | 0.23 |
| 6 | #6 | 2E7 | C-λ | 350 | 0.40 |
| 7 | #6m | | | 375 | 2.34 |
| 8 | #7 | 2H2 | C-λ | 127 | 0.44 |
| 9 | #8 | 12A1 | C-λ | 245 | 1.66 |
| 10 | #9 | 2H1 | C-λ | 310 | 1.95 |
| 11 | #9m | | | 424 | |
| 12 | #10 | 4G4 | C-λ | 288 | 0.38 |
| 13 | #11 | 12E9 | C-λ | 437 | 0.57 |
| 14 | #11m | | | 240 | 2.01 |
| 15 | #12 | 4F11 | C-λ | 324 | 0.43 |
| 16 | #12m | | | 235 | 1.82 |
| 17 | #13 | 10E7 | M-λ | 220 | 1.73 |
| 18 | #14 | 2E9 | C-λ | 185 | 0.48 |
| 19 | #14m | | | 225 | 1.34 |
| 20 | #15 | 12B11 | C-λ | 376 | 0.72 |
| 21 | #15m | | | 60 | 1.20 |
| 22 | #16 | 3H8 | M-λ | 359 | 0.51 |
| 23 | #16m | | | 118 | 0.97 |
| 24 | #17 | 4E1 | C-λ | 380 | 0.56 |
| 25 | #17m | | | 32 | 1.13 |
| 26 | #18 | 2H9 | C-λ | 55 | 0.38 |
| 27 | #18m | | | 27 | 1.84 |
| 28 | #19 | 12C11 | C-λ | 220 | 0.72 |
| 29 | #20 | 3E10 | M-λ | 306 | 0.52 |
| 30 | #20m | | | 54 | 0.88 |
| 31 | #21 | 2G1 | C-λ | 140 | 0.55 |
| 32 | #21m | | | 22 | 0.94 |
| 33 | #22 | 1E6 | M-λ | 196 | 0.34 |
| 34 | #23 | 2H11 | C-λ | 96 | 0.45 |
| 35 | #24 | 2F1 | C-λ | 16 | 0.66 |
| 36 | #25 | 13C2 | C-λ | 90 | 1.16 |
| 37 | #26 | 2H8 | C-λ | 248 | 0.42 |
| 38 | #27 | 10A6 | M-λ | 96 | 1.56 |
| 39 | #27m | | | 75 | 1.41 |
| 40 | #28 | 4G2 | C-λ | 128 | 1.05 |
| 41 | #29 | 2G11 (2F12) | C-λ | 64 | 0.85 |
| 42 | #30 | 11H9 | M-λ | | |
| 43 | #30m | | | | |
| 44 | #31 | 12C9 | C-λ | x | |
| 45 | #32 | 7A10 | C-λ | | |
| 46 | #33 | 4G1 | C-λ | 95 | 0.27 |
| 47 | #33m | | | 104 | 0.97 |
| 48 | #34 | 7C6 | C-λ | x | |
| 49 | #34m | | | x | |
| 50 | #35 | 7A7 | C-λ | 369 | |
| 51 | #36 | 6A9 | C-λ | | |
| 52 | #37 | 12B8 | C-λ | 13 | |
| 53 | #38 | 12F7 | C-λ | 584 | 0.27 |
| 54 | #38m | | | 436 | 1.24 |
| 55 | #39 | 7A11 | C-λ | | |
| 56 | #40 | 4H11 | C-λ | 559 | 0.39 |
| 57 | #40m | | | 140 | 1.25 |
| 58 | #41 | 2G3 | C-λ | 134 | 2.11 |
| 59 | #41m | | | 314 | 1.23 |
| 60 | #42 | 12C10 | C-λ | 69 | 0.44 |
| 61 | #42m | | | 88 | 1.25 |
| 62 | #43 | 3E8 | C-λ | 549 | 0.45 |
| 63 | #43m | | | x | |
| 64 | #44 | 7D1 | C-λ | | |
| 65 | #45 | 4G5 | C-λ | 385 | 0.67 |
| 66 | #45m | | | 180 | 0.95 |
| 67 | #46 | 2E8 | C-λ | 584 | 0.47 |
| 68 | #46m | | | 438 | 1.26 |
| 69 | #47 | 13H9 | C-λ | 203 | 0.61 |
| 70 | #47m | | | 480 | 0.94 |
| 71 | #48 | 4E9 | C-λ | 452 | 0.70 |
| 72 | #49 | 4H7 | C-λ | 445 | 2.15 |
| 73 | #49m | | | 186 | 1.11 |
| 74 | #50 | 2F8 | M-λ, C-λ | 4 | |
| 75 | #50m | | | 60 | 1.08 |
| 76 | #51 | 3E7 | C-κ | 541 | 0.37 |
| 77 | #51m | | | 6 | 1.26 |
| 78 | #52 | 9H4 | C-κ | 84 | 0.89 |
| 79 | #52m | | | 170 | 0.89 |
| 80 | #53 | 8B1 | C-κ | 314 | 0.82 |
| 81 | #53m | | | 70 | 0.92 |
| 82 | #54 | 7G12 | C-λ | x | |
| 83 | #55 | 12F11 | C-λ | | |
| 84 | #55m | | | | |
| 85 | #56 | 5B12 | C-κ | | |
| 86 | #57 | 8C3 | C-κ | 132 | |
| 87 | #58 | 2F4 | C-λ | 104 | |
| 88 | #59 | 1C10 (1C8) | C-κ | 32 | |
| 89 | #60 | 5E9 | C-κ | 6 | 1.31 |
| 90 | #61 | 5C9 | C-κ | 187 | 0.80 |
| 91 | #62 | 5C7 | C-κ | 61 | 0.84 |
| 92 | #62m | | | 62 | 1.27 |
| 93 | #63 | 8A11 | C-κ | 66 | 0.69 |
| 94 | #64 | H1 | 10X IgG1 | =#50 | |
| 95 | #65 | H2 | | =#2 | |
| 96 | #66 | H3 | | | |
| 97 | #67 | H4 | | =#29 | |
| 98 | #68 | H5 | | x | |
| 99 | #69 | H6 | | | |
| 100 | #70 | H7 | | =#59 | |
| 101 | #71 | H8 | | | |
| 102 | #72 | H9 | | =#13 | |
| 103 | #73 | H10 | | x | |
| 104 | #74 | H11 | | | |
| 105 | #75 | H12 | | | |
| 106 | #76 | H13 | | | |
| 107 | #77 | H14 | | x | |
| 108 | #78 | H15 | | | |
| 109 | #79 | H16 | | | |
| 110 | #80 | H17 | | | |
| 111 | #81 | H18 | | | |
| 112 | #82 | H19 | | | |
| 113 | #83 | H20 | | x | |
| 114 | #84 | H21 | | 8 | 0.95 |
| 115 | #85 | H22 | | | |
| 116 | #86 | H23 | | | |
| 117 | #87 | H24 | | | |
| 118 | #88 | H25 | | 42 | 1.29 |
| 119 | #89 | G2 H1 | 10X IgG2 | | |
| 120 | #90 | G2 H2 | | | |
| 121 | #91 | G2 H3 | | | |
| 122 | #92 | G2 H4 | | x | |
| 123 | #93 | G2 H5 | | | |
| 124 | #94 | G2 H6 | | | |
| 125 | C1 | AM 14 | | 21 | X |
| 126 | C2 | 5C4 | | 142 | 0.57 |
| 127 | C3 | REGN222 | | 59 | 0.19 |
| 128 | C4 | D25 | | 105 | 0.73 |
| 129 | C5 | Synagis | | | |
| Library | | - M-λ: insert prepared manually | | | |
| | | - Γ-λ/C- : chromium library-derived insert | | | |
| | | - 10x: synthesis after chromium library NGS assay | | | |
| Expression | | - CEDEX IgG detection | | | |
| | | - X: no purification | | | |
| Kd | | - Affinity determination by ELISA | | | |

The day before the experiment, A549 cells were prepared at 4.5 × 10⁴ cells/well in a 96 well plate. Each sample antibody was prepared to make 4x dilutions (40 ~ 0.009 nM) with infection media from 40 nM (final 20 nM) in 7 steps in a 96 deep well plate to a final volume of 300 µL.

Titrated virus RSV A2 P3 stock was prepared to make a 1:1000 dilution with infection media. 200 µL of an IgG supernatant dilution and 200 µL of a virus dilution were mixed in a new 96 deep well plate, and a neutralization reaction was performed at 37 °C in a 5% CO₂ incubator for 1 hour. A mixture obtained by neutralizing the prepared A549 cells was added at 25 µL /well, and incubated at 37 °C in a 5% CO₂ incubator for 2 hours (the plate was shaken every 15 minutes so as not to dry the cells). 200 µL of 0.8% methylcellulose was added to each well and the cells were cultured at 37 °C in a 5% CO₂ incubator for 3 to 4 days. Overlay media was removed and the plate was washed with 300 µL /well of PBS once. 100% cold methanol was added at 100 µL/well, and the plate was covered with a wrap and stored at 4 °C.

After fixation for more than 4 hours, methanol was removed and washed twice with 300 µL of PBS. An RSV fusion protein antibody was diluted 1:1000 with infection media and added at 100 µL/well, followed by incubation for 2 hours at 37 °C. After washing three times with PBS, 100 uL of anti-human Fc IgG or antimouse IgG-HRP was added per well to be diluted 1:2000 with infection media, and incubated for 1 hour at 37 °C. After washing with PBS three times, the washing solution was thoroughly removed and 100 µL of a TrueBlue substrate was added per well to develop color at room temperature. After color development, the plate was washed and dried, and then IC₅₀ was calculated by counting spots using an ELISPOT reader.

### 6. Quantitative measurement of binding ability of anti-F antibody to F-protein

The quantitative binding ability (affinity) to recombinant F-protein of antibodies 8, 9, 12m, 13, 15, 16, 33, 46, and 61, which have the most excellent efficacy in the neutralizing antibody analysis, was measured using Biacore T-200 (GE Healthcare). An antibody to be measured was diluted to a concentration of 0.3 µg/mL in an HBS-EP buffer solution (10 mM HEPES (pH7.4), 150 mM NaCl, 3 mM EDTA, 0.005% surfactant P20) on a protein A chip (CAT. No. BR-1005-XX, GE Healthcare) and captured up to 200 Ru while flowing at a flow rate of 30 µL/min for 1 minute. The purified F-protein was sequentially diluted to a concentration range from 0.04 nM to 10 nM in an HBS-EP buffer solution and subjected to association for 12 seconds and dissociation for 1800 seconds while flowing at a flow rate of 30 µL/min. By flowing 10 mM glycine-HCl pH 2.0 for 30 seconds at a flow rate of 30 µL/min, the dissociation of the captured antibody onto the chip was induced. Affinity was obtained from kinetic rate constants (Kₒₙ and K_{off}) and an equilibrium dissociation constant (K_{D}) using Biacore T-200 evaluation software.

### Example 1. Result of production of RSV F-protein

### 1.1. RSV F-protein subtype production and purification

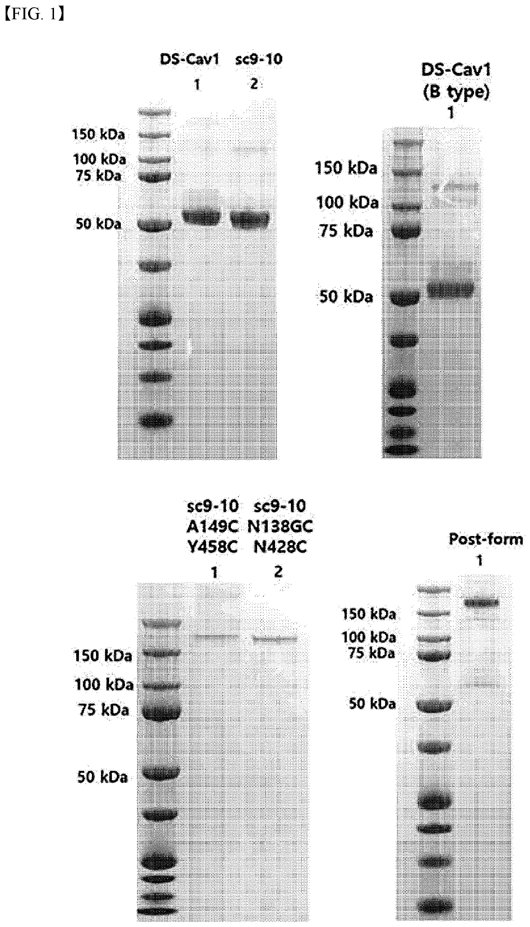

The RSV F-protein is composed of a trimer and its expected size is approximately 180 kDa. The expressed F-protein formed a trimer by a fibritin trimerization domain at the C-terminus. In the cases of the pre-forms of the F protein, DS-Cav1 and DS-Cav1 sc9-10, in SDS-PAGE (a non-covalent bond was broken), it was confirmed that the binding of the fibritin trimerization domain was broken such that most of the F-protein was observed in the form of a monomer. However, in the cases of sc9-10 A149C Y458C and sc9-10 N138GC N428C that have structural stability due to the addition of a covalent bond (disulfide bond), and the post-form F-protein transformed to a more stable structure than a pre-form protein, it was confirmed that even when the binding of the fibritin trimerization domain was broken, the F proteins may be stably present in the form of a trimer (FIG. 1).

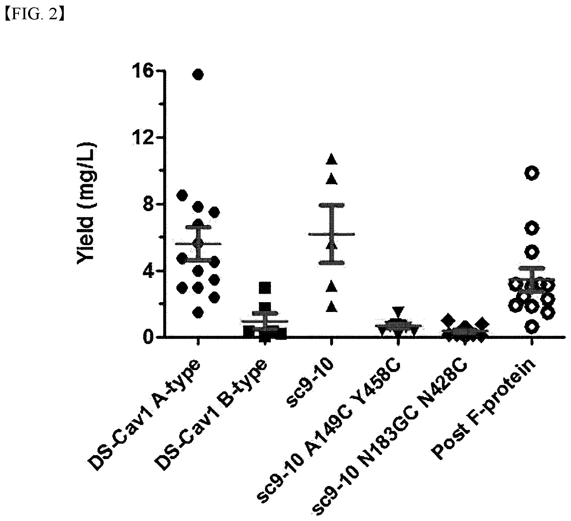

As a result of analyzing the expression rate of the RSV F-protein, it was found that the DS-Cav1 A-type has a better expression rate than the B-type (FIG. 2). In the A-type, it can be seen that the engineered clone sc9-10 has no significant difference in expression level from DS-Cav1, but for a stable trimer form in sc9-10, sc9-10 A149C Y458C and sc9-10 N183GC N428C, which have mutations, exhibit very low expression levels. Overall, depending on the cell passage of Expi293 cells, it was confirmed that there is a difference in expression level. It was confirmed that the longer the cell passage, the lower the expression level, which can eventually affect a yield.

### Example 2. Construction of human B cell-derived library

### 2.1. Human PBMC isolation

All PBMCs isolated from the blood collected from a healthy adult had a viability of 99%, and all PBMCs isolated from the blood collected from RSV-convalescent and healthy infants had a viability of 97 to 99%. Each type of cells was dispensed to have a concentration of 1 × 10⁷ cells per vial and then stored in liquid nitrogen.

### 2.2. Isolation of B cells specific for RSV F-protein (DS-Cav1)

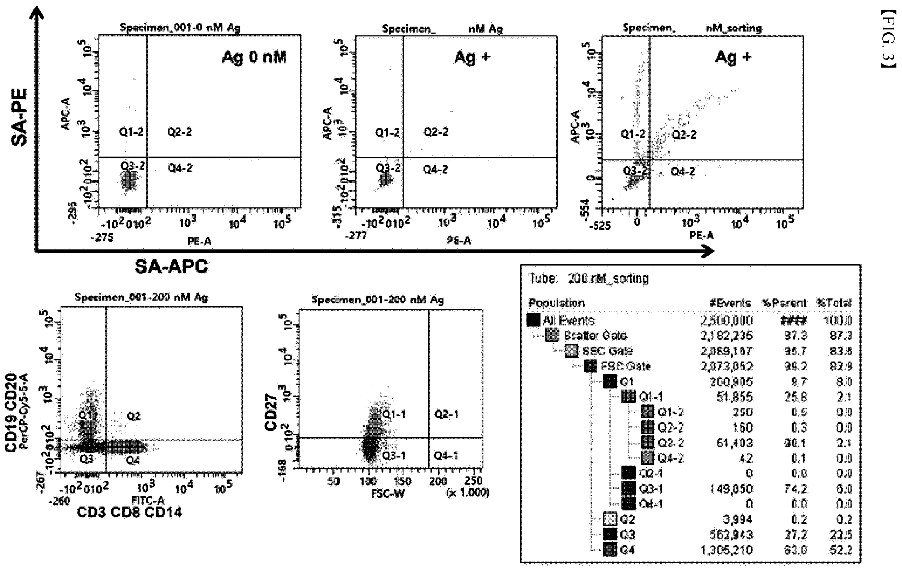

Among lymphocytes of PBMCs, it was confirmed that B cells CD3⁻CD8⁻ CD14⁻CD19⁺CD20⁺ were approximately 8% of the total cells, and memory B cells CD27⁺ were approximately 25% of the B cells. Among memory B cells, cells that are double-positive for APC and PE of the RSV F-protein were approximately 0.3%, and approximately 1000 cells of a total of 3.8 × 10⁷ cells were collected in 0.5 mL of 2% FBS/PBS (FIG. 3).

### 2.3. Construction of B cell-derived V(D)J library

FACS-sorted B cells lysed with Quick extraction buffer were subjected to RT-PCR and cDNA amplification for Ig transcripts using a SMARTer RACE 5' kit and a RT-IgGKLA primer. The cDNA which had experienced RT-PCR and PCR cleanup was immediately used in a volume of approximately 15 µL to construct a phage-displayed scFv library without gel electrophoresis.

### 2.4. Construction of chromium single cell V(D)J library

Chromium single cell V(D)J libraries were constructed for memory B cells (Ag⁺CD27⁺ B cells) double-positive for RSV F, memory B cells (Ag⁻CD27⁺ B cells) double-negative for RSV F used as a control, and unsorted PBMCs used as a control. The concentration after cDNA amplification of Ag⁺CD27⁺ B cells (1.82 ng/µL), Ag⁻ CD27⁺ B cells (0.383 ng/µL), and PBMC (1.57 ng/µL) was 45.5 µL, and the concentration after a target enrichment step of the Ag⁺CD27⁺ B cells (11.2 ng/µL), the Ag⁻CD27⁺ B cells (1.57 ng/µL), and the PBMC (0.542 ng/µL) was 45.5 µL. The concentration of the final sequencing library after enzymatic fragmentation and cleanup of the Ag⁺CD27⁺ B cells (69.4 ng/µL), the Ag⁻CD27⁺ B cells (31.2 ng/µL), and the PBMC (15.4 ng/µL) was 35 µL. All concentrations were measured with Qubit.

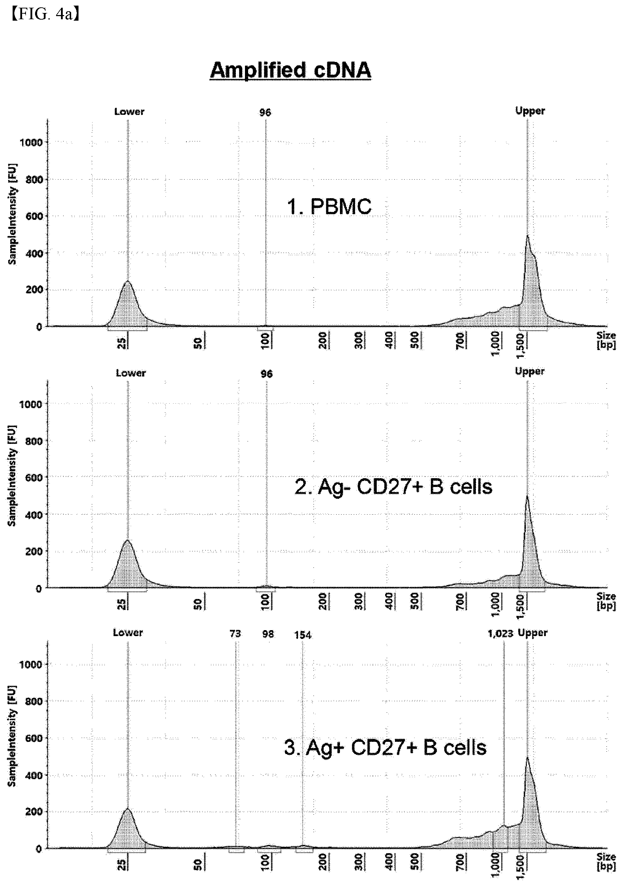

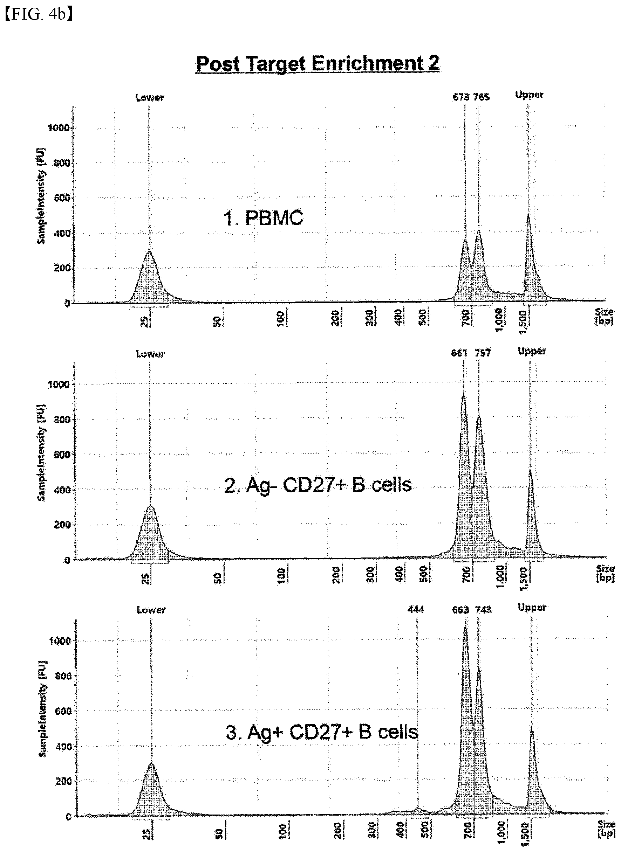

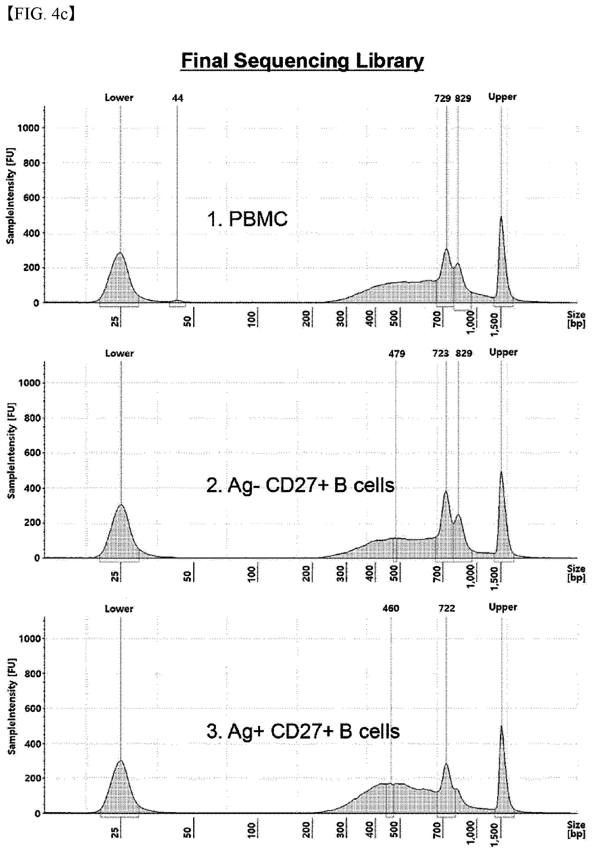

The result of QC performed after each step using Agilent Tapestation is shown in FIG. 4. In post target enrichment, Ig transcripts were observed between 600 to 800 bp, and in the final sequencing library step, traces of enzymatic fragmentation were confirmed.

### 2.5. Construction of phage displayed scFv library

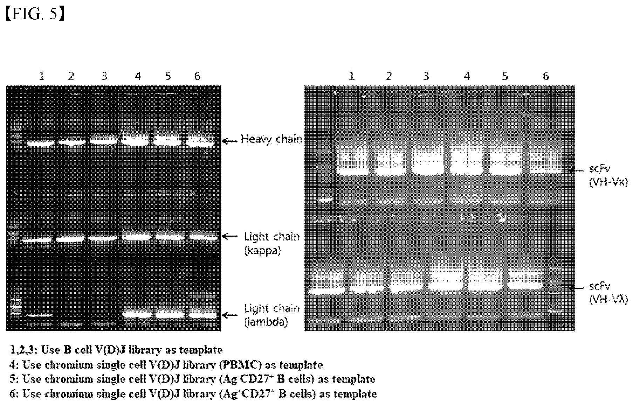

Each phage-displayed scFv library was constructed using the V(D)J library constructed as described above as a template. The results of VH/Vκ/V_{λ} PCR and scFv overlapping PCR are shown in FIG. 5.

### Example 3. Results of selecting and manufacturing anti-RSV antibody

**[Table 16]**

| Result of anti-DS-Cav1 phage display library panning | | | | | |
|---|---|---|---|---|---|
| Round | Antigen (DS-Cav1, In house) | Washing | Insert | Template cDNA | Output titer |
| 1^{st} | 5 µg/mL | PBST X 5 | VH-Vκ | Ag (+) memory B cell pool | 3.3X10⁷ |
| | | | | Chromium B cell + PBMC | 3.4X10⁷ |
| | | | | Chromium Ag (+) memory B cell | 1.4X10⁷ |
| | | | VH-Vλ | Ag (+) memory B cell pool | 1.1X10⁷ |
| | | | | Chromium B cell + PBMC | 1.7X10⁷ |
| | | | | Chromium Ag (+) memory B cell | 2.0X10⁷ |
| 2^{nd} | 3 µg/mL | PBST X 7 | VH-Vκ | Ag (+) memory B cell pool | 2.8X10⁸ |
| | | | | Chromium B cell + PBMC | 1.2X10⁸ |
| | | | | Chromium Ag (+) memory B cell | 7.4X10⁸ |
| | | | VH-Vλ | Ag (+) memory B cell pool | 9.3X10⁸ |
| | | | | Chromium B cell + PBMC | 2.5X10⁷ |
| | | | | Chromium Ag (+) memory B cell | 3.2X10⁹ |

**[Table 17]**

| Result of single colony screening | | | | | |
|---|---|---|---|---|---|
| Insert | Template cDNA | 1^{st} panning output | 2^{nd} panning output | # of hit | Remark |
| VH-Vκ | Ag (+) memory B cell pool | 0/144 | 0/48 | 0/192 | |
| | Chromium B cell + PBMC | 0/48 | 0/48 | 0/96 | Negative control |
| | Chromium Ag (+) memory B cell | 45/336 | 3/48 | 48/384 | |
| VH-Vλ | Ag (+) memory B cell pool | 9/336 | 13/48 | 22/384 | |
| | Chromium B cell + PBMC | 0/48 | 0/48 | 0/96 | Negative control |
| | Chromium Ag (+) memory B cell | 114/336 | 38/48 | 152/384 | |

Single colony screening for a total of 1536 samples was performed using ELISA. Among them, only 222 clones with an ELISA binding signal of 0.5 or more were sequenced. As a result of sequencing, the scFv full sequences of 133 out of 222 clones were confirmed, and except a duplicate sequence, a total of 63 unique clones were selected.

**[Table 18]**

| Nucleotide sequences of heavy chain and light chain variable domains of 63 types of clones | | | | | |
|---|---|---|---|---|---|
| | | Heavy chain | | Light chain | |
| # | Clone name | SEQ (Nucleotides) | Editing with SEQ (Nucleotides) Germline sequence (Modified) | SEQ (Nucleotides) | Editing with SEQ (Nucleotides) Germline sequence (Modified) |
| 1 | 2G4 | GAGGTGCAGCTGGTGGA GTCTGGGGGAGGCCTGG TCAAGCCTGGGGGGTCC CTGAGACTCTCCTGTGCA GCCTCTGGATTCACGTTC AGTAGCTATACCATGCA CTGGGTCCGCCAGGCTC CAGGGAAGGGGCTGGAG TGGGTCTCGTCCATAACT GGTGGCAGTAGTTATGT CGACTACTCAGCCTCAG TGAAGGGCCGATTCACC ATCTCCAGAGACAACGC TCAGAGCTCACTTTATCT GCAAATGAACAGCCTGA GAGCCGAGGACACGGCT GTGTATTACTGTGCGAG AGATGATTATGGTTCGG GGAGTTATTCCAACTGG TTCGACCCCTGGGGCCA GGGAACCCTGGTCACCG TCTCCTCA (SEQ ID NO: 49) | - | CAGTCTGTGCTGACGCAG CCGCCCTCAGTGTCTGGG GCCCCAGGGCAGAGGGTC ACCATCTCCTGCACTGGG AGCAGCTCCAACATCGGG GCAGGTTATGATGTACAC TGGTACCAGCAGGTTCCA GGAACAGCCCCCAAACTC CTCATCTTTGGTAGCACC AATCGGCCCTCAGGGGTC CCTGACCGATTCTCTGGC TCCAAGTCTGGCACCTCA GCCTCCCTGGCCATCACT GGCCTCCAGGCTGACGAT GAGGCTGATTATTACTGC CAGTCCTATGACCGCAGC CTGAGTCATGTCTTCGGA ACTGGGACCAAGGTCACC GTCCTAGGC (SEQ ID NO: 50) | - |
| 2 | 2H3 | GAGGTGCAGCTGGTGGA GTCTGGGGGAGGCGTGG TCCAGTCTGGGAGGTCC CTGAGACTCTCCTGTGCA GGCTCTGGATTCACCTTC AGTAGCTATACCATGCA CTGGGTCCGCCAGGCTC CAGGGAAGGGGCTGGAG TGGGTCTCGTCCATAACT GGTGGCAGTAGTTATGT CGACTACTCAGCCTCAG TGAAGGGCCGATTCACC ATCTCCAGAGACAACGC CCAGAGCTCACTTTATCT GCAAATGAACAGCCTGA GAGCCGAGGACACGGCT GTGTATTACTGTGCGAG AGATGATTATGGTTCGG GGAGTTATTCCAACTGG TTCGACCCCTGGGGCCA GGGAACCCTGGTCACCG TCTCCTCA (SEQ ID NO: 51) | - | CAGTCTGTCCTGACGCAG CCGCCCTCAGTGTCTGGG GCCCCAGGGCAGAGGGTC ACCATCTCCTGCACTGGG AGCAGCTCCAACATCGGG GCAGGTTATGATGTACAC TGGTACCAGCAGGTTCCA GGAACAGCCCCCAAACTC CTCATCTTTGGTAGCACC AATCGGCCCTCAGGGGTC CCTGACCGATTCTCTGGC TCCAAGTCTGGCGCCTCA GCCTCCCTGGCCATCACT GGGCTCCAGACTGAGGAT GAGGCTGATTATTACTGC CAGTCCTATGACCGCAGC CTGAGTCATGTCTTCGGA ACTGGGACCAAGGTCACC GTCCTAGGC (SEQ ID NO: 52) | - |
| 3 | 4H1 | GAGGTGCAGCTGGTGGA GTCTGGGGGAGGCCTGG TCAAGCCTGGGGGGTCC CTGAGACTCTCCTGTGCA GGCTCTGGATTCACCTTC AGTAGCTATACCATGCA CTGGGTCCGCCAGGCTC CAGGGAAGGGGCTGGAG | - | CAGTCTGTGCTGACGCAG CCGCCCTCAGTGTCTGGG GCCCCAGGGCAGAGGGTC ACCATCTCCTGCACTGGG AGCAGCTCCAACATCGGG GCAGGTTACGATGTACAC TGGTACCAGCAGGTTCCA GGAACAGCCCCCAAACTC | - |
| | | TGGGTCTCGTCCATAACT GGTGGCAGTAGTTATGT CGACTACTCAGCCTCAG TGAAGGGCCGATTCACC ATCTCCAGAGACAACGC CCAGAGCTCACTTTATCT GCAAATGAACAGCCTGA GAGCCGAGGACACGGCT GTGTATTACTGTGCGAG AGATGATTATGGTTCGG GGAGTTATTCCAACTGG TTCGACCCCTGGGGCCA GGGAACCCTGGTCACCG TCTCCTCA (SEQ ID NO: 53) | | CTCATCTTTGGTAGCACC AATCGGCCCTCAGGGGTC CCTGACCGATTCTCTGGT TCCAAGTCTGGCGCCTCT GCCTCCCTGGCCATCACT GGCCTCCAGGCTGACGAT GAGGCTGATTATTACTGC CAGCCCTATGACCGCAGC CTGAGTCATGTCTTCGGA ACTGGGACCAAGGTCACC GTCCTACGC (SEQ ID NO: 54) | |
| 4 | 4E3 | GAGGTGCAGCTGGTGGA GTCTGGGGGAGGCCTGG TCAAGCCTGGGGGGTCC CTGAGACTCTCCTGTGCA GGCTCTGGATTCACCTTC AGTAGCTATACCATGCA CTGGGTCCGCCAGGCTC CAGGGAAGGGGCTGGAG TGGGTCTCGTCCATAACT GGTGGCAGTAGTTATGT CGACTACTCAGCCTCAG TGAAGGGCCGATTCACC ATCTCCAGAGACAACGC CCAGAGCTCACTTTATCT GCAAATGAACAGCCTGA GAGCCGAGGACACGGCT GTGTATTACTGTGCGAG AGATGATTATGGTTCGG GGAGTTATTCCAACTGG TTCGACCCCTGGGGCCA GGGAACCCTGGTCACCG TCTCCTCA(SEQ ID NO: 55) | - | CAGTCTGTGCTGACGCAG CCGCCCTCAGTGTCTGGG GCCCCAGGGCAGAGGGTC ACCATCTCCTGCACTGGG AGCAGCTCCAACATCGGG GCAGGTTACGATGTACAC TGGTACCAGCAGGTTCCA GGAACAGCCCCCAAACTC CTCATCTTTGGTAGCACC AATCGGCCCTCAGGGGTC CCTGACCGATTCTCTGGC TCCAAGTCTGGCGCCTCT GCCTCCCTGGCCATCACT GGCCTCCAGGCTGACGAT GAGGCTGATTATTACTGC CAGTCCTATGACCGCAGC CTGAGTCATGTCTTCGGA ACTGGGACCAAGGTCACC GTCCTAGGC (SEQ ID NO: 56) | - |
| 5 | 4H5 | GAGGTGCAGCTGGTGGA GTCTGGGGGAGGCCTGG TCAAGCCTGGGGGGTCC CTGAGACTCTCCTGTGCA GGCTCTGGATTCACCTTC AGTAGCTATACCATGCA CTGGGTCCGCCAGGCTC CAGGGAAGGGGCTGGAG TGGGTCTCGTCCATAACT GGTGGCAGTAGTTATGT CGACTACTCAGCCTCAG TGAAGGGCCGATTCACC ATCTCCAGAGACAACGC CCAGAGCTCACTTTATCT GCAAATGAACAGCCTGA GAGCCGAGGACACGGCT GTGTATTACTGTGCGAG AGATGATTATGGTTCGG GGAGTTATTCCAACTGG TTCGACCCCTGGGGCCA GGGAACCCTGGTCACCG TCTCCTCA(SEQ ID NO: 57) | - | CAGTCTGTGCTGACGCAG CCGCCCTCAGTGTCTGGG GCCCCAGGGCAGAGGGTC ACCATCTCCTGCACTGGG AGCAGCTCCAACATCGGG GCAGGTTATGATGTACAC TGGTATCAGCAGCTTCCG GGAGCAGCCCCCAGACTC CTCATGTTTGGTAACAGC AATCGGCCCTCGGGGGTA CCTGACCGCTTCTCTGGC TCCAAGTCTGGCACCTCC GCCTCCCTGGCCATCACT GGTCTCCAGGCTGAGGAT GAGGCTGATTATTACTGC CAGTCCTATGACCGCAGC CTGAGTCATGTCTTCGGA ACTGGCACCAAGGTGACC GTCCTCGGC (SEQ ID NO: 58) | - |
| 6 | 2E7 | GAGGTGCAGCTGGTGGA GTCTGGGGGAGGCCTGG TCAAGCCTGGGGGGTCC | | TCCTATGAGCTGACTCAG CCACCCTCAGCGTCTGGG ACCCCCGGGCAGAGGGTC | CAGTCTGTGCT GACGCAGCCGC CCTCAGCGTCT |
| | | CTGAGACTCTCCTGTGCA GGCTCTGGATTCACCTTC AGTAGCTATACCATGCA CTGGGTCCGCCAGGCTC CAGGGAAGGGGCTGGAG TGGGTCTCGTCCATAACT GGTGGCAGTAGTTATGT CGACTACTCAGCCTCAG TGAAGGGCCGATTCACC ATCTCCAGAGACAACGC CCAGAGCTCACTTTATCT GCAAATGAACAGCCTGA GAGCCGAGGACACGGCT GTATATTACTGTGCGAG AGATGATTATGGTTCGG GGAGTTATTCCAACTGG TTCGACCCCTGGGGCCA GGGAACCCTGGTCACCG TCTCCTCA(SEQ ID NO: 44) | | ACCATCTCCTGCACTGGG AGCAGCTCCAACATCGGG GCAGGTTATGATGTACAC TGGTATCAGCAGCTTCCG GGAGCAGCCCCCAGACTC CTCATGTTTGGTAACAGC AATCGGCCCTCGGGGGTA CCTGACCGCTTCTCTGGC TCCAAGTCTGGCACCTCC GCCTCCCTGGCCATCACT GGCCTCCAGGCTGACGAT GAGGCTGATTATTACTGC CAGTCCTATGACCGCAGC CTGAGTCATGTCTTCGGA ACTGGGACCAAGGTCACC GTCCTAGGC (SEQ ID NO: 59) | GGGACCCCCGG GCAGAGGGTCA CCATCTCCTGC ACTGGGAGCAG CTCCAACATCG GGGCAGGTTAT GATGTACACTG GTATCAGCAGC TTCCGGGAGCA GCCCCCAGACT CCTCATGTTTG GTAACAGCAAT CGGCCCTCGGG GGTACCTGACC GCTTCTCTGGCT CCAAGTCTGGC ACCTCCGCCTC CCTGGCCATCA CTGGCCTCCAG GCTGACGATGA GGCTGATTATT ACTGCCAGTCC TATGACCGCAG CCTGAGTCATG TCTTCGGAACT GGG ACCAAGGTCAC CGTCCTAGGC (SEQ ID NO: 45) |
| 7 | 2H2 | CAGGTGCAGCTGGTGCA GTCTGGGGGAGGCTTGA TACAGCCTGGGGGGTCC CTGAGACTCTCCTGTGCA GGCTCTGGATTCACCTTC AGTAGCTATACCATGCA CTGGGTCCGCCAGGCTC CAGGGAAGGGGCTGGAG TGGGTCTCGTCCATAACT GGTGGCAGTAGTTATGT CGACTACTCAGCCTCAG TGAAGGGCCGATTCACC ATCTCCAGAGACAACGC CCAGAGCTCACTTTATCT GCAAATGAACAGCCTGA GAGCCGAGGACACGGCC GTGTATTACTGTGCGAG AGATGATTATGGTTCGG GGAGTTATTCCAACTGG TTCGACCCCTGGGGCCA GGGAACCCTGGTCACCG TCTCCTCA(SEQ ID NO: 60) | - | CAGTCTGTGCTGACGCAG CCGCCCTCAGTGTCTGGG GCCCCAGGGCAGAGGGTC ACCATCTCCTGCACTGGG AGCAGCTCCAACATCGGG GCAGGTTATGATGTACAC TGGTACCAGCAACTTCCA GGAACAGCCCCCAAACTC CTCATCTTTGCTAACACC AATCGGCCCTCAGGGGTC CCTGATCGATTCTCTGGC TCCAAGTCTGGCGCCTCT GCCTCCCTGGCCATCACT GGCCTCCAGGCTGACGAT GAGGCTGATTATTACTGC CAGTCCTATGACCGCAGC CTGAGTCATGTCTTCGGA ACTGGGACCAAGGTCACC GTCCTAGGC (SEQ ID NO: 61) | - |
| 8 | 12A1 | GAGGTGCAGCTGGTGGA GTCTGGGGGAGGCCTGA TACAGCCTGGGGGGTCC CTGAGACTCTCCTGTGCA GGCTCTGGATTCACCTTC AGTAGCTATACCATGCA CTGGGTCCGCCAGGCTC CAGGGAAGGGGCTGGAG TGGGTCTCGTCCATAACT GGTGGCAGTAGTTATGT CGACTACTCAGCCTCAG TGAAGGGCCGATTCACC | - | CAGTCTGTCCTGACGCAG CCGCCCTCAGTGTCTGGG GCCCCAGGGCAGAGGGTC ACCATCTCCTGCACTGGG AGCAGCTCCGACATCGGG GCAGGTTATGATGTACAC TGGTACCAGCAACTTCCA GGAACAGCCCCCAAACTC CTCATCTTTGCTAACACC AATCGGCCCTCAGGGGTC CCTGATCGATTCTCTGGC TCCAAGTCTGGCGCCTCT | - |
| | | ATCTCCAGAGACAACGC CCAGAGCTCACTTTATCT GCAAATGAACAGCCTGA GAGCCGAGGACACGGCC GTGTATTACTGTGCGAG AGATGATTATGGTTCGG GGAGTTATTCCAACTGG TTCGACCCCTGGGGCCA GGGAACCCTGGTCACCG TCTCCTCA(SEQ ID NO: 34) | | GCCTCCCTGGCCATCACT GGCCTCCAGGCTGACGAT GAGGCTGATTATTACTGC CAGTCCTATGACCGCAGC CTGAGTCATGTCTTCGGA ACTGGGACCAAGGTCACC GTCCTAGGC (SEQ ID NO: 35) | |
| 9 | 2H1 | CAGGTCACCTTGAAGGA GTCTGGGGGAGGCGTGG TCCAGTCTGGGAGGTCC CTGAGACTCTCCTGTGCA GCCTCTGGATTCACCTTC AGTAGCTATACCATGCA CTGGGTCCGCCAGGCTC CAGGGAAGGGGCTGGAG TGGGTCTCGTCCATAACT GGTGGCAGTAGTTATGT CGACTACTCAGCCTCAG TGAAGGGCCGATTCACC ATCTCCAGAGACAACGC CCAGAGCTCACTTTATCT GCAAATGAACAGCCTGA GAGCCGAGGACACGGCT GTGTATTACTGTGCGAG AGATGATTATGGTTCGG GGAGTTATTCCAACTGG TTCGACCCCTGGGGCCA GGGAACCCTGGTCACCG TCTCCTCA (SEQ ID NO: 36) | TGCTGTGGGTGA GTGGTACCTGTG GGGAGGTGCAG CTGGTGGAGTCT GGGGGAGGCGT GGTCCAGTCTGG GAGGTCCCTGA GACTCTCCTGTG CAGCCTCTGGAT TCACCTTCAGTA GCTATACCATGC ACTGGGTCCGCC AGGCTCCAGGG AAGGGGCTGGA GTGGGTCTCGTC CATAACTGGTG GCAGTAGTTATG TCGACTACTCAG CCTCAGTGAAG GGCCGATTCACC ATCTCCAGAGA CAACGCCCAGA GCTCACTTTATC TGCAAATGAAC AGCCTGAGAGC CGAGGACACGG CTGTGTATTACT GTGCGAGAGAT GATTATGGTTCG GGGAGTTATTCC AACTGGTTCGAC CCCTGGGGCCA GGGAACCCTGG TCACCGTCTCCT CAGCCTCCACCA AGGGCCCATCG GTCTTCCCCC (SEQ ID NO: 62) | CAGTCTGTGCTGACGCAG CCGCCCTCAGTGTCTGGG GCCCCAGGGCAGAGGGTC ACCATCTCCTGCACTGGG AGCAGCTCCAACATCGGG GCAGGTTATGATGTACAC TGGTACCAGCAGCTTCCA GGAAGAGCCCCCAAACTC CTCATCTATGCTAACACC AATCGGCCCTCAGGGGTC GCTGACCGATTCTCTGGC TCCAAGTCTGGCGCCTCT GCCTCCCTGGCCATCACT GGCCTCCAGGCTGACGAT GAGGCTGATTATTACTGC CAGTCCTATGACCGCAGC CTGAGTCATGTCTTCGGA ACTGGGACCAAGGTCACC GTCCTAGGC (SEQ ID NO: 37) | - |
| 10 | 4G4 | GAGGTGCAGCTGGTGGA GTCTGGGGGAGGCCTGG TCAAGCCTGGGGGGTCC CTGAGACTCTCCTGTGCA GGCTCTGGATTCACCTTC AGTAGCTATACCATGCA CTGGGTCCGCCAGGCTC CAGGGAAGGGGCTGGAG TGGGTCTCGTCCATAACT GGTGGCAGTAGTTATGT CGACTACTCAGCCTCAG TGAAGGGCCGATTCACC ATCTCCAGAGACAACGC CCAGAGCTCACTTTATCT GCAAATGAACAGCCTGA | - | CAGTCTGTGCTGACGCAG CCGCCCTCAGTGTCTGGG GCCCCAGGGCAGAGGGTC ACCATTTCCTGCACTGGG AACAGCTCCAACCTCGGG GCAGGTTATGATGTACAC TGGTACCAGCAACTTCCA GGAACAGCCCCCAAACTC CTCATCTATGCTAACACC AATCGGCCCTCAGGGGTC CCTGACCGATTCTCTGGC TCCAAGTCTGGCGCCTCA GCCTCCCTGGCCATCACT GGGCTCCAGACTGAGGAT GAGGCTGATTATTACTGC | - |
| | | GAGCCGAGGACACGGCT GTGTATTACTGTGCGAG AGATGATTATGGTTCGG GGAGTTATTCTAACTGGT TCGACCCCTGGGGCCAG GGAACCCTGGTCACCGT CTCCTCA (SEQ ID NO: 63) | | CAGTCCTATGACCGCAGC CTGAGTCATGTCTTCGGA ACTGGGACCAAGGTCACC GTCCTAGGC (SEQ ID NO: 64) | |
| 11 | 12E9 | GAGGTGCAGCTGGTGGA GTCTGGGGGAGGCCTGG TCAAGCCTGGGGGGTCC CTGAGACTCTCCTGTGCA GCCTCTGGATTCACCTTC ACTAGTTATAGGATGCA TTGGGTCCGCCAGGCTC CAGGGAAGGGGCTGGAG TGGGTCTCATCAATTACT GGTGGTGGTAATTATAT AGAGTACGCAGACTCAG TGAAGGGCCGATTCACC ATCTCCAGAGACAACGC CCAGAGCTCACTTTATCT GCAAATGAACAGCCTGA GAGCCGAGGACACGGCT GTGTATTACTGTGCGAG AGATGATTATGGTTCGG GGAGTTATTCCAACTGG TTCGACCCCTGGGGCCA GGGAACCCTGGTCACCG TCTCCTCA(SEQ ID NO: 46) | - | CAGACTGTGGTGACTCAG GAGCCCTCAGTGTCTGGG GCCCCAGGGCAGAGGGTC ACCATCTCCTGCACTGGG AGCAGCTCCAACATCGGG GCAGGTTATGATGTACAC TGGTACCAGCAGCTTCCA GGAAGAGCCCCCAAACTC CTCATCTTTGCTAACACC AATCGGCCCTCAGGGGTC CCTGACCGATTCTCTGGC TCCAAGTCTGGCGCCTCT GCCTCCCTGGCCATCACT GGCCTCCAGGCTGACGAT GAGGCTGATTATTACTGC CAGTCCTATGACCGCAGC CTGAGTCATGTCTTCGGA ACTGGGACCAAGGTCACC GTCCTAGGC (SEQ ID NO: 65) | CAGTCTGTGCT GACGCAGCCGC CCTCAGTGTCT GGGGCCCCAGG GCAGAGGGTCA CCATCTCCTGC ACTGGGAGCAG CTCCAACATCG GGGCAGGTTAT GATGTACACTG GTACCAGCAGC TTCCAGGAAGA GCCCCCAAACT CCTCATCTTTGC TAACACCAATC GGCCCTCAGGG GTCCCTGACCG ATTCTCTGGCTC CAAGTCTGGCG CCTCTGCCTCCC TGGCCATCACT GGCCTCCAGGC TGACGATGAGG CTGATTATTACT GCCAGTCCTAT GACCGCAGCCT GAGTCATGTCT TCGGAACTGGG ACCAAGGTCAC CGTCCTAGGC (SEQ ID NO: 39) |
| 12 | 4F11 | GAGGTGCAGCTGGTGGA GTCTGGGGGAGGCCTGG TCAAGCCTGGGGGGTCC CTGAGACTTTCCTGTGCT GGCTCTGGATTCGCCTTC AGTAGTTACACTATGCA CTGGGTGCGCCAGGCTC CAGGGAAGGGGCTGGAG TGGGTCTCATCCATCACT GGCGGCAGTAGTTACCT AGACTACGCACACTCAG TGAAGGGCCGATTCACC ATCTCCAGAGACAATGG CCAGAACTCACTGTTTCT GCAAATGAACAGCCTGA GGACCGAGGACACGGCT GTATATTACTGTGCGAG AGATGACTATGGTTCGG GGAGTTATTCCAACTACT TCGACCCCTGGGGCCAG GGAACCCTGGTCACCGT CTCCTCA (SEQ ID NO: 47) | - | CAGCCTGTGCTGACTCAG CCACCCTCAGTGTCTGGG GCCCCAGGGCAGAGGGTC ACCATCTCCTGCACTGGG AGCAGCTCCAACATCGGG GCAGGTTATGATGTACAC TGGTACCAGCAGGTTCCA GGAACGGCCCCCAAACTC CTCATCTTTGGTAGCACC AATCGGCCCTCAGGGGTC CCTGACCGATTCTCTGGC TCCAAGTCTGGCGCCTCA GCCTCCCTGGCCATCACT GGGCTCCAGACTGAGGAT GAGGCTGATTATTACTGC CAGTCCTATGACCGCAGC CTGAGTCATGTCTTCGGA ACTGGGACCAAGGTCACC GTCCTAGGC (SEQ ID NO: 66) | CAGTCTGTGCT GACGCAGCCGC CCTCAGTGTCT GGGGCCCCAGG GCAGAGGGTCA CCATCTCCTGC ACTGGGAGCAG CTCCAACATCG GGGCAGGTTAT GATGTACACTG GTACCAGCAGG TTCCAGGAACG GCCCCCAAACT CCTCATCTTTGG TAGCACCAATC GGCCCTCAGGG GTCCCTGACCG ATTCTCTGGCTC CAAGTCTGGCG CCTCAGCCTCC CTGGCCATCAC TGGGCTCCAGA CTGAGGATGAG GCTGATTATTA CTGCCAGTCCT |
| | | | | | ATGACCGCAGC CTGAGTCATGT CTTCGGAACTG GGACCAAGGTC ACCGTCGCTAG GC (SEQ ID NO: 48) |
| 13 | 10E7 | GAGGTGCAGCTGGTGGA GTCTGGGGGAGGCCTGG TCAAGCCTGGGGGGTCC CTGAGACTCTCCTGTGCA GCCTCTGGATTCACCTTC ACTAGTTATAGGATGCA TTGGGTCCGCCAGGCTC CAGGGAAGGGGCTGGAG TGGGTCTCATCAATTACT GGTGGTGGTAATTATAT AGAGTACGCAGACTCAG TGAAGGGCCGGTTCACC ATCTCCAGAGACAACGC CAAGAACTCACTGGATC TGCAAATGAACAGCCTG AGAGCCGAGGACACGGC TATTTATTACTGTGCGAG AGATATGTATGGTTTGG GGAGTTATTATTCGCCTA ACTACTTCGACTCCTGGG GCCAGGGAACCCTGGTC ACCGTCTCCTCA (SEQ ID NO: 38) | - | CAGTCTGTGCTGACGCAG CCGCCCTCAGTGTCTGGG GCCCCAGGGCAGAGGGTC ACCATCTCCTGCACTGGG AGCAGCTCCAACATCGGG GCAGGTTATGATGTACAC TGGTACCAGCAGCTTCCA GGAAGAGCCCCCAAACTC CTCATCTTTGCTAACACC AATCGGCCCTCAGGGGTC CCTGACCGATTCTCTGGC TCCAAGTCTGGCGCCTCT GCCTCCCTGGCCATCACT GGCCTCCAGGCTGACGAT GAGGCTGATTATTACTGC CAGTCCTATGACCGCAGC CTGAGTCATGTCTTCGGA ACTGGGACCAAGGTCACC GTCCTAGGC (SEQ ID NO: 39) | - |
| 14 | 2E9 | CAGGTCACCTTGAAGGA GTCTGGGGGAGGCCTGG TCAAGCCTGGGGGGTCC CTGAGACTCTCCTGTGCA GGCTCTGGATTCACCTTC AGTAGCTATACCATGCA CTGGGTCCGCCAGGCTC CAGGGAAGGGGCTGGAG TGGGTCTCGTCCATAACT GGTGGCAGTAGTTATGT CGACTACTCAGCCTCAG TGAAGGGCCGATTCACC ATCTCCAGAGACAACGC CAAGAACTCACTGTTTCT GCAAATGAACAGCCTGA GAGCCGAGGACACGGCT GTGTATTACTGTGCGAG AGATGATTATGGTTCGG GGAGTTATTCCAACTGG TTCGACCCCTGGGGCCA GGGAACCCTGGTCACCG TCTCCTCA(SEQ ID NO: 67) | AGGTGCAGCTG GTGGAGTCTGG GGGAGGCCTGG TCAAGCCTGGG GGGTCCCTGAG ACTCTCCTGTGC AGGCTCTGGATT CACCTTCAGTAG CTATACCATGCA CTGGGTCCGCCA GGCTCCAGGGA AGGGGCTGGAG TGGGTCTCGTCC ATAACTGGTGG CAGTAGTTATGT CGACTACTCAGC CTCAGTGAAGG GCCGATTCACCA TCTCCAGAGAC AACGCCAAGAA CTCACTGTTTCT GCAAATGAACA GCCTGAGAGCC GAGGACACGGC TGTGTATTACTG TGCGAGAGATG ATTATGGTTCGG GGAGTTATTCCA ACTGGTTCGACC CCTGGGGCCAG GGAACCCTGGT CACCGTCTCCTC A (SEQ ID NO: 68) | CAGTCTGTGCTGACGCAG CCGCCCTCAGTTTCTGGG GCCCCAGGGCAGAGGGTC ACCATCTCCTGCACTGGG AGCAGCTCCAACATCGGG GCAGGTTATGATGTACAC TGGTACCAGCAGCTTCCA GGAAGAGCCCCCAAACTC CTCATCTTTGCTAACACC AATCGGCCCTCAGGGGTC CCTGACCGATTCTCTGGC TCCAAGTCTGGCGCCTCT GCCTCCCTGGCCATCACT GGCCTCCAGGCTGACGAT GAGGCTGATTATTACTGC CAGTCCTATGACCGCAGC CTGAGTCATGTCTTCGGA ACTGGGACCAAGGTCACC GTCCTAGGC (SEQ ID NO: 69) | - |
| 15 | 12B11 | GAGGTGCAGCTGGTGGA GTCTGGGGGAGGCGTGG TCCAGCCTGGGAGGTCC CTGAGACTCGCCTGTGC AGCCTCTGGATTCACCTT CAATAGCTATGCTATGC ACTGGGTCCGCCAGGCT CCAGGGAAGGGGCTGGA GTGGGTCTCGTCCATAA CTGGTGGCAGTAGTTAT GTCGACTACTCAGCCTC AGTGAAGGGCCGATTCA CCATCTCCAGAGACAAC GCCCAGAGCTCACTTTAT CTGCAAATGAACAGCCT GAGAGCCGAGGACACGG CTGTGTATTACTGTGCGA GAGATATGTATGGTTTG GGGAGTTATTATTCGCCT AACTACTTCGACCCCTG GGGCCAGGGAACCCTGG TCACTGTCTCCTCA(SEQ ID NO: 70) | - | TCCTATGAGCTGACTCAG CCACCCTCAGTGTCTGGG GCCCCAGGGCAGAGGGTC ACCATCTCCTGCACTGGG AGCAGCTCCAACATCGGG GCAGGTTATGATGTACAC TGGTACCAGCAGCTTCCA GGAAGAGCCCCCAAACTC CTCATCTTTGCTAACACC AATCGGCCCTCAGGGGTC CCTGACCGATTCTCTGGC TCCAAGTCTGGCACCTCC GCCTCCCTGGCCATCACT GGTCTCCAGGCTGAGGAT GAGGCTGATTATTACTGC CAGTCCTATGACAGTAGC CTGAGTTATGTCTTCGGA ACTGGGACCAAGGTCACC GTCCTAGGC (SEQ ID NO: 71) | CAGTCTGTGCT GACGCAGCCGC CCTCAGTGTCT GGGGCCCCAGG GCAGAGGGTCA CCATCTCCTGC ACTGGGAGCAG CTCCAACATCG GGGCAGGTTAT GATGTACACTG GTACCAGCAGC TTCCAGGAAGA GCCCCCAAACT CCTCATCTTTGC TAACACCAATC GGCCCTCAGGG GTCCCTGACCG ATTCTCTGGCTC CAAGTCTGGCA CCTCCGCCTCC CTGGCCATCAC TGGTCTCCAGG CTGAGGATGAG GCTGATTATTA CTGCCAGTCCT ATGACAGTAGC CTGAGTTATGT CTTCGGAACTG GG ACCAAGGTCAC CGTCCTAGGC (SEQ ID NO: 72) |
| 16 | 3H8 | GAGGTGCAGCTGGTGGA GTCTGGGGGAGGCCTGG TCAAGCCTGGGGGGTCC CTGAGACTCTCCTGTGCA GGCTCTGGATTCACCTTC AGTAGCTATACCATGCA CTGGGTCCGCCAGGCTC CAGGGAAGGGGCTGGAG TGGGTCTCGTCCATAACT GGTGGCAGTAGTTATGT CGACTACTCAGCCTCAG TGAAGGGCCGATTCACC ATCTCCAGAGATAACGC CCAGAGCTCACTTTATCT GCAAATGAACAGCCTGA GAGCCGAGGACACGGCT GTGTATTACTGTGCGAG AGATGATTATGGTTCGG GGAGTTATTCCAACTGG TTCGACCCCTGGGGCCG GGGAACCACGGTCACCG TCTCCTCA(SEQ ID NO: 40) | - | CAGCCTGTGCTGACTCAG CCA C TCTCAGCGTCTGGGACCC CCGGGCAGAGGGTCACCA TCTCCTGCACTGGGAGCA GCTCCAACATCGGGGCAG GTTATGATGTACACTGGT ACCAGCAGCTTCCAGGAA GAGCCCCCAAACTCCTCA TCTTTGCTAACACCAATC GGCCCTCAGGGGTCCCTG ACCGATTCTCTGGCTCCA AGTCGGGCACCTCAGCCT CCCTGGCCATCACTGGGC TCCAGGCTGAGGATGAGG CTGATTATTACTGCCAGT CCTATGACCGCAGCCTGA GTCATGTCTTCGGAACTG G GACCAAGGTCACCGTCCT AGGC (SEQ ID NO: 41) | CAGTCTGTGCT GACGCAGCCG CTC TCA GCGTCTGGGAC CCCCGGGCAGA GGGTCACCATC TCCTGCACTGG GAGCAGCTCCA ACATCGGGGCA GGTTATGATGT ACACTGGTACC AGCAGCTTCCA GGAAGAGCCCC CAAACTCCTCA TCTTTGCTAAC ACCAATCGGCC CTCAGGGGTCC CTGACCGATTC TCTGGCTCCAA GTCGGGCACCT CAGCCTCCCTG GCCATCACTGG GCTCCAGGCTG AGGATGAGGCT GATTATTACTG CCAGTCCTATG ACCGCAGCCTG AGTCATGTCTT CGGAACTGGG ACCAAGGTCAC CGTCCTAGGC (SEQ ID NO: 73) |
| 17 | 4E1 | GAGGTGCAGCTGGTGGA GTCTGGGGGAGGCTTGG TACAGCCTGGAGGGTCC CTGAGACTCGCCTGTGC AGCCTCTGGATTCACCTT CAGTAGCTATACCATGC ACTGGGTCCGCCAGGCT CCAGGGAAGGGGCTGGA GTGGGTCTCGTCCATAA CTGGTGGCAGTAGTTAT GTCGACTACTCAGCCTC AGTGAAGGGCCGATTCA CCATCTCCAGAGACAAC GCCCAGAGCTCACTTTAT CTGCAAATGAACAGCCT AAGAGCCGAGGACACGG CTGTGTATTACTGTGCGA GAGATGATTATGGTTCG GGGAGTTATTCCAACTG GTTCGACCCCTGGGGCC AGGGAACCCTGGTCACC GTCTCCTCA(SEQ ID NO: 74) | - | TCCTATGAGCTGACTCAG CCAC CCTCAGTGTCTGGGGCCC CAGGGCAGAGGGTCACC ATCTCCTGCACTGGGAGC AGCTCCAACATCGGGGCA GGTTATGATGTACACTGG TACCAGCAGCTTCCAGGA AGAGCCCCCAAACTCCTC ATCTTTGCTAACACCAAT CGGCCCTCAGGGGTCCCT GACCGATTCTCTGGCTCC AAGTCTGGCGCCTCTGCC TCCCTGGCCATCACTGGC CTCCAGGCTGAGGATGAG GCTGATTATTACTGCCAG TCCTATGACCGCAGCCTG AGTCATGTCTTCGGAACT GGGACCAAGGTCACCGTC CTAGGC (SEQ ID NO: 75) | CAG    TCT GTG CTG ACG CAG CCG CCC TCAGTGTCTGG GGCCCCAGGGC AGAGGGTCACC ATCTCCTGCAC TGGGAGCAGCT CCAACATCGGG GCAGGTTATGA TGTACACTGGT ACCAGCAGCTT CCAGGAAGAGC CCCCAAACTCC TCATCTTTGCTA ACACCAATCGG CCCTCAGGGGT CCCTGACCGAT TCTCTGGCTCC AAGTCTGGCGC CTCTGCCTCCCT GGCCATCACTG GCCTCCAGGCT GAGGATGAGGC TGATTATTACT GCCAGTCCTAT GACCGCAGCCT GAGTCATGTCT TCGGAACTGGG ACCAAGGTCAC CGTCCTAGGC (SEQ ID NO: 76) |
| 18 | 2H9 | CAGGTCACCTTGAAGGA GTCTGGGGGAGGCCTGG TCAAGCCTGGGGGGTCC CTGAGACTCTCCTGTGCA GCCTCTGGATTCACCTTC ACTAGTTATAGGATGCA TTGGGTCCGCCAGGCTC CAGGGAAGGGGCTGGAG TGGGTCTCATCAATTACT GGTGGTGGTAATTATAT AGAGTACGCAGACTCAG TGAAGGGCCGGTTCACC ATCTCCAGAGACAACGC CAAGAACTCACTGGATC TGCAAATGAACAGCCTG AGAGCCGAGGACACGGC TATTTATTACTGTGCGAG AGATATGTATGGTTTGG GGAGTTATTATTCGCCTA ACTACTTCGACCCCTGG GGCCAGGGAACCCTGGT CACCGTCTCCTCA (SEQ ID NO: 77) | GAGGTGCAGCT GGTGGAGTCTG GGGGAGGCCTG GTCAAGCCTGG GGGGTCCCTGA GACTCTCCTGTG CAGCCTCTGGAT TCACCTTCACTA GTTATAGGATGC ATTGGGTCCGCC AGGCTCCAGGG AAGGGGCTGGA GTGGGTCTCATC AATTACTGGTGG TGGTAATTATAT AGAGTACGCAG ACTCAGTGAAG GGCCGGTTCACC ATCTCCAGAGA CAACGCCAAGA ACTCACTGGATC TGCAAATGAAC AGCCTGAGAGC CGAGGACACGG CTATTTATTACT GTGCGAGAGAT ATGTATGGTTTG GGGAGTTATTAT TCGCCTAACTAC TTCGACCCCTGG GGCCAGGGAAC CCTGGTCACCGT | TCCTATGAGCTGACTCAG CCACTCTCAGTGTCTGGG GCCCCAGGGCAGAGGGTC ACCATTTCCTGCACTGGG AGCAGCTCCAACATCGGG GCAGGTTATGATGTACAC TGGTACCAGCAGGTTCCA GGAACAGCCCCCAAACTC CTCATCTTTGGTAGCACC AATCGGCCCTCAGGGGTC CCTGACCGATTCTCTGGC TCCAAGTCTGGCGCCTCT GCCTCCCTGGCCATCACT GGCCTCCAGGCTGACGAT GAGGCTGATTATTACTGC CAGTCCTATGACCGCAGC CTGAGTCATGTCTTCGGA ACTGGGACCAAGGTCACC GTCCTAGGC (SEQ ID NO: 79) | CAGTCTGTGCT GACGCAGCCG CTCTCAGTGTCT GGGGCCCCAGG GCAGAGGGTCA CCATTTCCTGC ACTGGGAGCAG CTCCAACATCG GGGCAGGTTAT GATGTACACTG GTACCAGCAGG TTCCAGGAACA GCCCCCAAACT CCTCATCTTTGG TAGCACCAATC GGCCCTCAGGG GTCCCTGACCG ATTCTCTGGCTC CAAGTCTGGCG CCTCTGCCTCCC TGGCCATCACT GGCCTCCAGGC TGACGATGAGG CTGATTATTACT GCCAGTCCTAT GACCGCAGCCT GAGTCATGTCT TCGGAACTGGG ACCAAGGTCAC CGTCCTAGGC (SEQ ID NO: 80) |
| | | | CTCCTCA (SEQ ID NO: 78) | | |
| 19 | 12C11 | GAGGTGCAGCTGGTGGA GTCTGGGGGAGGCCTGG TCAAGCCTGGGGGGTCC CTGAGACTCTCCTGTGCA GCCTCTGGATTCACCTTC ACTAGTTATAGGATGCA TTGGGTCCGCCAGGCTC CAGGGAAGAGGCTGGAG TGGGTCTCATCAATTACT GGTGGTGGTAATTATAT AGAGTACGCAGACTCAG TGAAGGGCCGGTTCACC ATCTCCAGAGACAACGC CAAGAACTCACTGGATC TGCAAATGAACAGCCTG AGAGCCGAGGACACGGC TATTTATTACTGTGCGAG AGATATGTATGGTTTGG GGAGTTATTATTCGCCTA ACTACTTCGACCCCTGG GGCCAGGGAACCCTGGT CACCGTCTCCTCA(SEQ ID NO: 81) | - | CAGTCTGTGCTGACGCAG CCGCCCTCAGTGTCTGGG GCTCCAGGGCAGAGGGTC ACCATCTCCTGCACTGGG AGCAGCTCCAACATCGGG GCAGGTTATGATGTACAC TGGTACCAGCAGGTTCCA GGAACAGCCCCCAAACTC CTCATCTTTGGTAGCACC AATCGGCCCTCAGGGGTC CCTGACCGATTCTCTGGC TCCAAGTCTGGCGCCTCA GCCTCCCTGGCCATCACT GGGCTCCAGACTGAGGAT GAGGCTGATTATTACTGC CAGTCCTATGACCGCAGC CTGAGTCATGTCTTCGGA ACTGGG ACCAAGGTCACCGTCCTA GGC (SEQ ID NO: 82) | - |
| 20 | 3E10 | ID NO: 81) GAGGTGCAGCTGGTGGA GTCTGGGGGAGGCCTGG TCAAGCCTGGGGGGTCC CTGAGACTCTCCTGTGCA GCCTCTGGATTCACCTTC ACTAGTTATAGGATGCA TTGGGTCCGCCAGGCTC CAGGGAAGGGGCTGGAG TGGGTCTCATCAATTACT GGTGGTGGTAATTATAT AGAGTACGCAGACTCAG TGAAGGGCCGGTTCACC ATCTCCAGAGACAACGC CAAGAACTCACTGGATC TGCAAATGAACAGCCTG AGAGCCGAGGACACGGC TATTTATTACTGTGCGAG AGATATGTATGGTTTGG GGAGTTATTATTCGCCTA ACTACTTCGACCCCTGG GGCCAGGGAACCCTGGT CACCGTCTCCTCA (SEQ ID NO: 83) | - | CAGCCTGTGCTGACTCAG CCACCCTCAGTGTCTGGG GCCCCAGGGCAGAGGGTC ACCATCTCCTGCACTGGG AGCAGCTCCAACATCGGG GCAGGTTATGATGTACAC TGGTACCAGCAGGTTCCA GGAACAGCCCCCAAACTC CTCATCTTTGGTAGCACC AATCGGCCCTCAGGGGTC CCTGACCGATTCTCTGGC TCCAAGTCTGGCGCCTCA GCCTCCCTGGCCATCACT GGGCTCCAGACTGAGGAT GAGGCTGATTATTACTGC CAGTCCTATGACCGCAGC CTGAGTCATGTCTTCGGA ACTGGGACCAAGGTCACC GTCCTAGGC (SEQ ID NO: 84) | CAGTCTGTGCT GACGCAGCCGC CCTCAGTGTCT GGGGCCCCAGG GCAGAGGGTCA CCATCTCCTGC ACTGGGAGCAG CTCCAACATCG GGGCAGGTTAT GATGTACACTG GTACCAGCAGG TTCCAGGAACA GCCCCCAAACT CCTCATCTTTGG TAGCACCAATC GGCCCTCAGGG GTCCCTGACCG ATTCTCTGGCTC CAAGTCTGGCG CCTCAGCCTCC CTGGCCATCAC TGGGCTCCAGA CTGAGGATGAG GCTGATTATTA CTGCCAGTCCT ATGACCGCAGC CTGAGTCATGT CTTCGGAACTG GG ACCAAGGTCAC CGTCCTAGGC (SEQ ID NO: 85) |
| 21 | 2G1 | CAGGTCACCTTGAAGGA GTCTGGGGGAGGCCTGG TCAAGCCTGGGGGGTCC CTGAGACTCTCCTGTGCA GCCTCTGGATTCACCTTC ACTAGTTATAGGATGCA TTGGGTCCGCCAGGCTC | GAGGTGCAGCT GGTGGAGTCTG GGGGAGGCCTG GTCAAGCCTGG GGGGTCCCTGA GACTCTCCTGTG CAGCCTCTGGAT | CAGTCTGTGCTGACGCAG CCGCCCTCAGTGTCTGGG GCCCCAGGGCAGAGGGTC ACCATCTCCTGCACTGGG AGCAGCTCCAACATCGGG GCAGGTTATGATGTACAC TGGTATCAGCAGCTTCCA | - |
| | | CAGGGAAGGGGCTGGAG TGGGTCTCATCAATTACT GGTGGTGGTAATTATAT AGAGTACGCAGACTCAG TGAAGGGCCGGTTCACC ATCTCCAGAGACAACGC CAAGAACTCACTGGATC TGCAAATGAACAGCCTG AGAGCCGAGGACACGGC TATTTATTACTGTGCGAG AGATATGTATGGTTTGG GGAGTTATTATTCGCCTA ACTACTTCGACCCCTGG GGCCAGGGAACCCTGGT CACCGTCTCCTCA (SEQ ID NO: 86) | TCACCTTCACTA GTTATAGGATGC ATTGGGTCCGCC AGGCTCCAGGG AAGGGGCTGGA GTGGGTCTCATC AATTACTGGTGG TGGTAATTATAT AGAGTACGCAG ACTCAGTGAAG GGCCGGTTCACC ATCTCCAGAGA CAACGCCAAGA ACTCACTGGATC TGCAAATGAAC AGCCTGAGAGC CGAGGACACGG CTATTTATTACT GTGCGAGAGAT ATGTATGGTTTG GGGAGTTATTAT TCGCCTAACTAC TTCGACCCCTGG GGCCAGGGAAC CCTGGTCACCGT CTCCTCA (SEQ ID NO: 87) | GGAACAGCCCCCAAACTC CTCATCTTTGGTAGCACC AATCGGCCCTCAGGGGTC CCTGACCGATTCTCTGGC TCCAAGTCTGGCGCCTCA GCCTCCCTGGCCATCACT GGGCTCCAGACTGAGGAT GAGGCTGATTATTACTGC CAGTCCTATGACCGCAGC CTGAGTCATGTCTTCGGA ACTGGG ACCAAGGTCACCGTCCTA GGC (SEQ ID NO: 88) | |
| 22 | 1E6 | GAGGTGCAGCTGGTGGA GTCTGGGGGAGGCCTGG TCAAGCCTGGGGGGTCC CTGAGACTCTCCTGTGCA GCCTCTGGATTCACCTTC ACTAGTTATAGGATGCA TTGGGTCCGCCAGGCTC CAGGGAAGGGGCTGGAG TGGGTCTCATCAATTACT GGTGGTGGTAATTATAT AGAGTACGCAGACTCAG TGAAGGGCCGGTTCACC ATCTCCAGAGACAACGC CAAGAACTCACTGGACC TGCAAATGAACAGCCTG AGAGCCGAGGACACGGC TATTTATTACTGTGCGAG AGATATGTATGGTTTGG GGAGTTATTATTCGCCTA ACTACTTCGACCCCTGG GGCCAGGGAACCCTGGT CACTGTCTCCTCA (SEQ ID NO: 89) | - | CAGTCTGTGCTGACGCAG CCGCCC TCA GTTTCTGGGGCCCCAGGG CAGAGGGTCACCATCTCC TGCACTGGGAGCAGCTCC AACATCGGGGCAGGTTAT GATGTACACTGGTACCAG CAGCTTCCAGGAAGAGCC CCCAAACTCCTCATCTTT GCTAACACCAATCGGCCC TCAGGGGTCCCTGACCGA TTCTCTGGCTCCAAGTCT GGCGCCTCTGCCTCCCTG GCCATCACTGGCCTCCAG GCTGACGATGAGGCTGAT TATTACTGCCAGTCCTAT GACCGCAGCCTGAGTCAT GTCTTCGGAACTGGG ACCAAGGTCACCGTCCTA GGC (SEQ ID NO: 90) | - |
| 23 | 2H11 | CAGGTCCAGCTGGTACA GTCTGGGGGAGGCCTGG TCAAGCCTGGGGGGTCC CTGAGACTCTCCTGTGCA GCCTCTGGATTCACCTTC ACTAGTTATAGGATGCA TTGGGTCCGCCAGGCTC CAGGGAAGGGGCTGGAG TGGGTCTCATCAATTACT GGTGGTGGTAATTATAT AGAGTACGCAGACTCAG TGAAGGGCCGGTTCACC ATCTCCAGAGACAACGC CAAGAACTCACTGGATC | - | CAGTCTGTGTTGACGCAG CCGCCCTCAGTGTCTGGG GCCCCAGGGCAGAGGGTC ACCATCTCCTGCACTGGG AGCAGCTCCAACATCGGG GCAGGTTATGATGTACAC TGGTACCAGCAGCTTCCA GGAAGAGCCCCCAAACTC CTCATCTTTGCTAACACC AATCGGCCCTCAGGGGTC CCTGACCGATTCTCTGGC TCCAAGTCTGGCGCCTCT GCCTCCCTGGCCATCTCT GGGCTCCAGGCTGAGGAT | - |
| | | TGCAAATGAACAGCCTG AGAGCCGAGGACACGGC TATTTATTACTGTGCGAG AGATATGTATGGTTTGG GGAGTTATTATTCGCCTA ACTACTTCGACCCCTGG GGCCAGGGAACCACGGT CACCGTCTCCTCA(SEQ ID NO: 91) | | GAGGCTCATTATTACTGC CAGTCCTATGACAGGAGC CTGAATGTGGTTTTCGGC GGAGGGACCGAGCTGAC CGTCCTAGGC (SEQ ID NO: 92) | |
| 24 | 2F1 | GAGGTGCAGCTGGTGGA GTCTGGGGGAGGCCTGG TCAAGCCTGGGGGGTCC CTGAGACTCTCCTGTGCA GCCTCTGGATTCACCTTC ACTAGTTATAGGATGCA TTGGGTCCGCCAGGCTC CAGGGAAGGGGCTGGAG TGGGTCTCATCAATTACT GGTGGTGGTAATTATAT AGAGTACGCAGACTCAG TGAAGGGCCGGTTCACC ATCTCCAGAGACAACGC CAAGAACTCACTGGATC TGCAAATGAACAGCCTG AGAGCCGAGGACACGGC TATTTATTACTGTGCGAG AGATATGTATGGTTTGG GGAGTTATTATTCGCCTA ACTACTTCGACCCCTGG GGCCAGGGAACCCTGGT CACCGTCTCCTCA (SEQ ID NO: 93) | - | CAGTCTGTGCTGACGCAG CCGCCCTCAGTGTCTGGG GCCCCAGGGCAGAGGGTC ACCATCTCCTGCACTGGG AGCAGCTCCAACATCGGG GCAGGTTATGATGTACAC TGGTACCAGCAGGTTCCA GGAACAGCCCCCAAACTC CTCATCTTTGGTAGCACC AATCGGCCCTCAGGGGTC CCTGACCGATTCTCTGGC TCCAAGTCTGGCGCCTCA GCCTCCCTGGCCATCACT GGGCTCCAGACTGAGGAT GAGGCTGATTATTATTGC CAGTCCTATGACAGAAGC CTGAGTGCTTGGGTGTTC GGCGGAGGG ACCAAGCTGACCGTCCTA GGC (SEQ ID NO: 94) | - |
| 25 | 13C2 | GAGGTGCAGCTGGTGGA GTCTGGGGGAGGCCTGG TCAAGCCTGGGGGGTCC CTGAGACTCTCCTGTGCA GCCTCTGGATTCACCTTC ACTAGTTATAGGATGCA TTGGGTCCGCCAGGCTC CAGGGAAGGGGCTGGAG TGGGTCTCATCAATTACT GGTGGTGGTAATTATAT AGAGTACGCAGACTCAG TGAAGGGCCGGTTCACC ATCTCCAGAGACAACGC CAAGAACTCACTGGATC TGCAAATGAACAGCCTG AGAGCCGAGGACACGGC TATTTATTACTGTGCGAG AGATATGTATGGTTTGG GGAGTTATTATTCGCCTA ACTACTTCGACCCCTGG GGCCAGGGAACCCTGGT CACCGTCTCCTCA (SEQ ID NO: 95) | - | CAGTCTGTCCTGACGCAG CCGCCCTCAGTGTCTGGG GCCCCAGGGCAGAGGGTC ACCATCTCCTGCACTGGG AGCAGCTCCAACATCGGG GCAGGTTATGATGTACAC TGGTACCAGCAGGTTCCA GGAACAGCCCCCAAACTC CTCATCTTTGGTAGCACC GATCGGCCCTCAGGGGTC CCTGATCGCTTCTCTGGCT CCAAGTCTGGCAACACGG CCTCCCTGACCATCTCTG GGCTCCAGGCTGAGGATG AGGCTGATTATTATTGCC AGTCCTATGACCGCAGCC TGAGTCATGTCTTCGGAA CTGGG ACCAAGGTCACCGTCCTA GGC (SEQ ID NO: 96) | - |
| 26 | 2H8 | GAGGTGCAGCTGGTGGA GTCTGGGGGAGGCCTGG CCAAGCCTGGGGGGTCC CTGAGACTCTCCTGTGCA GCCTCTGGATTCACCTTC ACTAGTTATAGGATGCA TTGGGTCCGCCAGGCTC CAGGGAAGGGGCTGGAG TGGGTCTCATCAATTACT | - | CAGTCTGTGCTGACGCAG CCGCCCTCAGTGTCTGGG GCCCCAGGGCAGAGGGTC ACCATCTCCTGCACTGGG AGCAGCTCCAACATCGGG GCAGGTTATGATGTACAC TGGTATCAGCAGCTTCCG GGAGCAGCCCCCAGACTC CTCATGTTTGGTAACAGC | - |
| | | GGTGGTGGTAATTATAT AGAGTACGCAGACTCAG TGAAGGGCCGGTTCACC ATCTCCAGAGACAACGC CAAGAACTCACTGGATC TGCAAATGAACAGCCTG AGAGCCGAGGACACGGC TATTTATTACTGTGCGAG AGATATGTATGGTTTGG GGAGTTATTATTCGCCTA ACTACTTCGACCCCTGG GGCCAGGGAACCCTGGT CACCGTCTCCTCA(SEQ ID NO: 97) | | AATCGGCCCTCGGGGGTA CCTGACCGCTTCTCTGGC TCCAAGTCTGGCACCTCC GCCTCCCTGGCCATCACT GGTCTCCAGGCTGAGGAT GAGGCTGATTATTACTGC CAGTCCTATGACCGCAGC CTGAGTCATGTCTTCGGA ACTGGGACCAAGGTCACC GTCCTAGGC (SEQ ID NO: 98) | |
| 27 | 10A6 | GAGGTGCAGCTGGTGGA GTCTGGGGGAGGCCTGG TCAAGCCTGGGGGGTCC CTGAGACTCTCCTGTGCA GCCTCTGGATTCACCTTC ACTAGTTATAGGATGCA TTGGGTCCGCCAGGCTC CAGGGAAGGGGCTGGAG TGGGTCTCATCAATTACT GGTGGTGGTAATTATAT AGAGTACGCAGACTCAG TGAAGGGCCGGTTCACC ATCTCCAGAGACAACGC CAAGAACTCACTGGATC TGCAAATGAACAGCCTG AGAGCCGAGGACACGGC TATTTATTACTGTGCGAG AGATATGTATGGTTTGG GGAGTTATTATTCGCCTA ACTACTTCGACTCCTGGG GCCAGGGAACCCTGGTC ACCGTCTCCTCA(SEQ ID NO: 99) | - | TCCTATGAGCTGACTCAG CCACCCTCAGTGTCTGGG GCCCCAGGGCAGAGGGTC ACCATCTCCTGCACTGGG AGCAGCTCCAACATCGGG GCAGGTTATGATGTACAC TGGTATCAGCAGCTTCCG GGAGCAGCCCCCAGACTC CTCATGTTTGGTAACAGC AATCGGCCCTCGGGGGTA CCTGACCGCTTCTCTGGC TCCAAGTCTGGCACCTCC GCCTCCCTGGCCATCACT GGTCTCCAGGCTGAGGAT GAGGCTGATTATTACTGC CAGTCCTATGACAGTAGC CTGAGTTATGTCTTCGGA ACTGGGACCAAGGTCACC GTCCTAGGC (SEQ ID NO: 100) | CAGTCTGTGCT GACGCAGCCGC CC TCA GTGTCTGGGGC CCCAGGGCAGA GGGTCACCATC TCCTGCACTGG GAGCAGCTCCA ACATCGGGGCA GGTTATGATGT ACACTGGTATC AGCAGCTTCCG GGAGCAGCCCC CAGACTCCTCA TGTTTGGTAAC AGCAATCGGCC CTCGGGGGTAC CTGACCGCTTC TCTGGCTCCAA GTCTGGCACCT CCGCCTCCCTG GCCATCACTGG TCTCCAGGCTG AGGATGAGGCT GATTATTACTG CCAGTCCTATG ACAGTAGCCTG AGTTATGTCTTC GGAACTGGGAC CAAGGTCACCG TCCTAGGC (SEQ ID NO: 101) |
| 28 | 4G2 | GAGGTGCAGCTGGTGGA GTCTGGGGGAGGCCTGG TCAAGCCTGGGGGGTCC CTGAGACTCTCCTGTGCA GCCTCTGGATTCACCTTC ACTAGTTATAGGATGCA TTGGGTCCGCCAGGCTC CAGGGAAGGGGCTGGAG TGGGTCTCATCAATTACT GGTGGTGGTAATTATAT AGAGTACGCAGACTCAG TGAAGGGCCGGTTCACC ATCTCCAGAGACAACGC CAAGAACTCACTGGATC TGCAAATGAACAGCCTG AGAGCCGAGGACACGGC TATTTATTACTGTGCGAG AGATATGTATGGTTTGG | - | CAGTCTGTGCTGACGCAG CCGCCC TCA GTGTCTGGGGCCCCAGGG CAGAGGGTCACCATCTCC TGCACTGGGAGCAGCTCC AACATCGGGGCAGGTTAT GATGTACACTGGTATCAG CAGCTTCCGGGAGCAGCC CCCAGACTCCTCATGTTT GGTAACAGCAATCGGCCC TCGGGGGTACCTGACCGC TTCTCTGGCTCCAAGTCT GGCACCTCCGCCTCCCTG GCCATCACTGGTCTCCAG GCTGAGGATGAGGCTGAT TATTACTGCCAGTCCTAT GACAGTAGCCTGAGTCAT GTCTTCGGAACTGGGACC | - |
| | | GGAGTTATTATTCGCCTA ACTACTTCGACCCCTGG GGCCAGGGAACCCTGGT CACCGTCTCCTCA(SEQ ID NO: 102) | | AAGGTCACCGTCCTAGGC (SEQ ID NO: 103) | |
| 29 | 2G11 | GAGGTGCAGCTGGTGGA GTCTGGGGGAGGCTTGG TCCAGCCTGGGGGGTCC CTGAGACTCTCCTGTGCA GCCTCTGGATTCACCTTC ACTAGTTATAGGATGCA TTGGGTCCGCCAGGCTC CAGGGAAGGGGCTGGAG TGGGTCTCATCAATTACT GGTGGTGGTAATTATAT AGAGTACGCAGACTCAG TGAAGGGCCGGTTCACC ATCTCCAGAGACAACGC CAAGAACTCACTGGATC TGCAAATGAACAGCCTG AGAGCCGAGGACACGGC TATTTATTACTGTGCGAG AGATATGTATGGTTTGG GGAGTTATTATTCGCCTA ACTACTTCGACCCCTGG GGCCAGGGAACCCTGGT CACCGTCTCCTCA(SEQ ID NO: 104) | - | CAGTCTGTGCTGACGCAG CCGCCCTCAGTGTCTGGG GCCCCAGGGCAGAGGGTC ACCATCTCCTGCACTGGG AGCAGCTCCAACATCGGG GCAGGTTATGATGTACAC TGGTATCAGCAGCTTCCG GGAGCAGCCCCCAGACTC CTCATGTTTGGTAACAGC AATCGGCCCTCGGGGGTA CCTGACCGCTTCTCTGGC TCCAAGTCTGGCACCTCC GCCTCCCTGGCCATCACT GGTCTCCAGGCTGAGGAT GAGGCTGATTATTACTGC CAGTCCTATGACAGTAGC CTGAGTTATGTCTTCGGA ACTGGGACCAAGGTCACC GTCCTAGGC (SEQ ID NO: 105) | - |
| 30 | 11H9 | GAGGTGCAGCTGGTGGA GTCTGGGGGAGGCTTGG TACAGCCTGGGGGGTCC CTGAGACTCTCCTGTGCA GCATCTGAATTCACCTTT AGCATGAACTGGGTCCG ACAGGCTCCAGGGAAGG GGCTGGAGTGGGTCTCA TCAGTTCGAGGTGGCGG TACTGAAACATACTATG CAGACTCCGTGAAGGGC CGGTTCACCGTCTCCAG AGACAATTCCAAGAACA CCCTGCATCTGCAAATG AACAGCCTGAGAGCCGA GGACACGGCCCTTTATT ACTGTGCGGGCGGCCCT ATAGTGGAACCCAACAT TGACTACTTTAACTCCTG GGGCCAGGGAACCCTGG TCACCGTCTCCTCA (SEQ ID NO: 106) | - | TGCTGTGGGTGAGTGGTA CCTGTGGGCAGTCTGTGC TGACGCAGCCGCCCTCAG TGTCTGGGGCCCCAGGGC AGAGGGTCACCATCTCCT GCACTGGGAGCAGCTCCA ACATCGGGGCAGGTTATG ATGTACACTGGTACCAGC AGCTTCCAGGAAGAGCCC CCAAACTCCTCATCTTTG CTAACACCAATCGGCCCT CAGGGGTCCCTGACCGAT TCTCTGGCTCCAAGTCTG GCGCCTCTGCCTCCCTGG CCATCACTGGCCTCCAGG CTGACGATGAGGCTGATT ATTACTGCCAGTCTTATG ACAGCAAACATCATGTGG TCTTTGGCACAGGGACCA AGCTGACCGTCCTAGGCC AACCTAAGGCCAATCCCA C (SEQ ID NO: 107) | CAGTCTGTGCT GACGCAGCCGC CC TCA GTGTCTGGGGC CCCAGGGCAGA GGGTCACCATC TCCTGCACTGG GAGCAGCTCCA ACATCGGGGCA GGTTATGATGT ACACTGGTACC AGCAGCTTCCA GGAAGAGCCCC CAAACTCCTCA TCTTTGCTAAC ACCAATCGGCC CTCAGGGGTCC CTGACCGATTC TCTGGCTCCAA GTCTGGCGCCT CTGCCTCCCTG GCCATCACTGG CCTCCAGGCTG ACGATGAGGCT GATTATTACTG CCAGTCTTATG ACAGCAAACAT CATGTGGTCTTT GGCACAGGG ACCAAGCTGAC CGTCCTAGGC (SEQ ID NO: 108) |
| 31 | 12C9 | "GAGGTGCAGCTGGTGG AGTCTGGGGGAGGCTTG GTCCAGCCTGGGGGGTC CCTGAGACTCTCCTGTGC | - | CAGTCTGTGTTGACGCAG CCGCCCTCAGTGTCTGGG GCCCCAGGGCAGAGGGTC ACCATCTCCTGCACTGGG | - |
| | | AGCCTCTGGGTTCTCCTT TAGGAGTTATTGGATGA GCTGGCTCCGCCAGGCC CCAGGGAAGGGGCTGCA GTGGGTGGCCAACATAA AGCCAGACGGAAGTGTG GAAAGTTATGTGGACTC CGTGGAGGGCCGATTCA CCATCTCCAGAGACAAC GCCAAGAATTCACTGTTT CTGCAAATGAACAGCCT GTCAGCCGAGGACATGG CTGTTTATTACTGTGCGA GGACGGACGACGGCAGC AGCTGGTTCGTGTCCACC AGTAGTTTCTACGGTATG GACGTCTGGGGCCAAGG GACCACGGTCACCGTCT CCTCA " (SEQ ID NO: 109) | | AGCAGCTCCAACATCGGG GCAGGTTATGATGTACAC TGGTACCAGCAGGTTCCA GGAACAGCCCCCAAACTC CTCATCTTTGGTAGCACC AATCGGCCCTCAGGGGTC CCTGACCGATTCTCTGGC TCCAAGTCTGGCGCCTCA GCCTCCCTGGCCATCATT GGGCTCCAGACTGAGGAT GAGGCTGATTATTACTGC CAGTCCTATGACCGCAGC CTGAGTCATGTCTTCGGA ACTGGCACCCAGCTGACC GACCTCGGC (SEQ ID NO: 110) | |
| 32 | 7A10 | GAGGTGCAGCTGGTGGA GTCTGGGGGAGGCCTGG TCAAGCCTGGGGGGTCC CTGAGACTCTCCTGTGCA GCCTCTGGATTCACCTTC AGTAGCTATACCATGCA CTGGGTCCGCCAGGCTC CAGGGAAGGGGCTGGAG TGGGTCTCGTCCATAACT GGTGGCAGTAGTTATGT CGACTACTCAGCCTCAG TGAAGGGCCGATTCACC ATCTCCCGAGACAATTC CAAGAACACGCTGTATC TGCAAATGAGCAGCCTG AGAACTGACGACACGGC CGTATATTTCTGTGCGAA AGACCAACGTGGAGACA GCTATGACTCGGTTATG AACTGGTACTTCGATCTC TGGGGCCGTGGCACCAC GGTCACCGTCTCCTCA(S EQ ID NO: 111) | - | CAGTCTGTGTTGACGCAG CCGCCCTCAGTGTCTGGG GCCCCAGGGCAGAGGGTC ACCATTTCCTGCACTGGG AACAGCTCCAACCTCGGG GCAGGTTATGATGTACAC TGGTACCAGCAGGTTCCA GGAACAGCCCCCAAACTC CTCATCTTTGGTAGCACC AATCGGCCCTCAGGGGTC CCTGACCGATTCTCTGGC TCCAAGTCTGGCGCCTCA GCCTCCCTGACAATCTCT GGGCTCCAGGCTGAGGAC GAGGCTGATTATTACTGC TGTTCGTATGCAGCTAGT AGCTCTAGGGTCTTCGGA ACTGGCACCAAGGTGACC GTCCTCGGC (SEQ ID NO: 112) | - |
| 33 | 4G1 | CAGGTCACCTTGAAGGA GTCTGGGGGCGGCCTGG TTAAGCCTGGGGGGTCC CTGAGACTCTCGTGTGC AGCCTCTGGGTTCAGGC TCAGTAGCTATGGCATG AACTGGGTCCGCCAGGC TCCAGGGAAGGGGCTGG AGTGGGTCTCATCCATTT CTGCTAGTAGTAGTTTTA TAAACTATGCAGACTCA GTGAGGGACCGATTCAC CATCTCCAGAGACAACG CCAAGAACTCACTGTAT CTGCAAATGAACAGCCT GAGAGCCGAGGACACGG CTGTGTATTACTGTGCGA GAGATGATTATGGTTCG GGGAGTTATTCCAACTG GTTCGACCCCTGGGGCC | TGCTGTGGGTGA GTGGTACCTGTG GGGAGGTGCAG CTGGTGGAGTCT GGGGGCGGCCT GGTTAAGCCTG GGGGGTCCCTG AGACTCTCGTGT GCAGCCTCTGG GTTCAGGCTCAG TAGCTATGGCAT GAACTGGGTCC GCCAGGCTCCA GGGAAGGGGCT GGAGTGGGTCT CATCCATTTCTG CTAGTAGTAGTT TTATAAACTATG CAGACTCAGTG AGGGACCGATT | CAGTCTGTGCTGACGCAG CCGCCCTCAGTGTCTGGG GCCCCAGGGCAGAGGGTC ACCATTTCCTGCACTGGG AACAGCTCCAACCTCGGG GCAGGTTATGATGTACAC TGGTACCAGCAGCTTCCA GGAAGAGCCCCCAAACTC CTCATCTTTGCTAACACC AATCGGCCCTCAGGGGTC CCTGACCGATTCTCTGGC TCCAAGTCTGGCGCCTCT GCCTCCCTGGCCATCACT AGCCTCCAGGCTGACGAT GAGGCTGATTATTACTGC CAGTCCTATGACCGCAGC CTGAGTCATGTCTTCGGA ACTGGGACCAAGGTCACC GTCCTAGGC (SEQ ID NO: 43) | - |
| | | AGGGAACCCTGGTCACC GTCTCCTCA (SEQ ID NO: 42) | CACCATCTCCAG AGACAACGCCA AGAACTCACTGT ATCTGCAAATG AACAGCCTGAG AGCCGAGGACA CGGCTGTGTATT ACTGTGCGAGA GATGATTATGGT TCGGGGAGTTAT TCCAACTGGTTC GACCCCTGGGG CCAGGGAACCC TGGTCACCGTCT CCTCAGCCTCCA CCAAGGGCCCA TCGGTCTTCCCC C (SEQ ID NO: 113) | | |
| 34 | 7C6 | GAGGTGCAGCTGGTGGA GTCTGGGGGAGGCCTGG TCAAGCCTGGGGGGTCC CTGAGACTCTCCTGTGCA GCCTCTGGATTCACCTTC AGTCATTATGGTATTAGT TGGGTGCGCCAGGCCCC TGGACAAGGCCTTGAGT GGATGGCCTGGATCAGC GCTTACAATGGTAACAC AGACTCCATACAGAAGG TCCAGGGCAGAGTCACC ATGACCACAGACACATC CACGAACACAGCCTACT TGGAATTGAGGAGCCTG AGATCTGACGACACGGC CGTGTATTACTGTGTAAG AGATGTCCCGGCCACAG GAGGAGCTGCCACGGCT GACTACTGGGGCCAAGG AACCCTGGTCACCGTCTC CTCA (SEQ ID NO: 114) | TGCTGTGGGTGA GTGGTACCTGTG GGCAGGTTCAG CTGGTGCAGTCT GGAGCTGAGGT GAAGAAGCCTG GGGCCTCAGTG AAGGTC TCCTGCAAGGCT TCTGGATTCACC TTCAGTCATTAT GGTATTAGTTGG GTGCGCCAGGC CCCTGGACAAG GCCTTGAGTGG ATGGCCTGGATC AGCGCTTACAAT GGTAACACAGA CTCCATACAGA AGGTCCAGGGC AGAGTCACCAT GACCACAGACA CATCCACGAAC ACAGCCTACTTG GAATTGAGGAG CCTGAGATCTGA CGACACGGCCG TGTATTACTGTG TAAGAGATGTC CCGGCCACAGG AGGAGCTGCCA CGGCTGACTACT GGGGCCAAGGA ACCCTGGTCACC GTCTCCTCAGCC TCCACCAAGGG CCCATCGGTCTT CCCCC (SEQ ID NO: 115) | CAGTCTGTGTTGACGCAG CCGCCCTCAGTGTCTGGG GCCCCAGGGCAGAGGGTC ACCATCTCCTGCACTGGG AGCAGCTCCAACATCGGG GCAGGTTATGATGTACAC TGGTACCAGCAGGTTCCA GGAACAGCCCCCAAACTC CTCATCTTTGGTAGCACC AATCGGCCCTCAGGGGTC CCTGACCGATTCTCTGGC TCCAAGTCTGGCGCCTCA GCCTCCCTGGCCATCACT GGGCTCCAGGCTGAGGAC GAGGCTGATTATTACTGC AGCTCATATACAAGCAGC AGCACTTTCGTGGTATTT GGTGGAGGAACCCAGCTG ATCATTTTAGGC (SEQ ID NO: 116) | - |
| 35 | 7A7 | CAGGTGCAGCTGGTGGA GTCTGGGGGAGGCGTGG TCCAGCCTGGGAGGTCC CTGAGACTCTCCTGTGCA GCCTCT GGATTCACCTTCAATAG | - | CAGTCTGCCCTGACTCAG CCTGCCTCCGTGTCTGGG TCTCCTGGACAGTCGATC ACCATCTCCTGCACTGGA ACCAGCAGTGACGTTGGT GGTTATAACTATGTCTCC | - |
| | | CTATGCTATGCACTGGGT CCGCCAGGCTCCAGGCA ACGGGCTGGACTGGGTG GCAGGTATATCATATGA TGGAGGCAATAAATATT ACGCAGACTCCGTGAAG GACCGATTCACCATCTCC CGAGACAATTCCAAGAA CACGCTGTATCTGCAAA TGAGCAGCCTGAGAACT GACGACACGGCTGTATA TTACTGTGCGAAAGATG CCGGATGGGAGGCTTGG TGGTACTTCGACCTCTGG GGCCGTGGCACCCTGGT CACTGTCTCCTCA (SEQ ID NO: 117) | | TGGTTCCAACAGTACCCA GGCAAAGCCCCCAAACTC ATGATTTATGATGTCAGT AAGCGGCCCTCAGGGGTT TCTAATCGCTTCTCTGGCT CCAAGTCTGGCAACACGG CCTCCCTGACCATCTCTG AGCTCCAGGCTGAGGATG AGGCTGATTATTACTGCA GCTCATATACAAGCAGCA GCACTGGCGTATTCGGCG GAGGGACCAAGCTGACC GTCCTAGGC (SEQ ID NO: 118) | |
| 36 | 6A9 | GAGGTGCAGCTGGTGGA GTCTGGGGGAGGCTTGG TACAGCCTGGGGGGTCC CTGAGACTCTCCTGTGCA GCCTCTGGAACCTTTACC AAATATGCCATGACCTG GGTCCGCCAGGCTCTAG GGAAGGGGCTGGAGTGG GTCTCAACTATTGGCAGT GGTAGTGACACTCACTA CGCAGACTCCGTGAAGG GCCGCTTCACCATCTCCA GAGACAATTCCAGGAAC ACTCTGTCTCTACAAATG AACAGCCTGAGAGCCGA GGACACGGCCGTATATT ACTGTGCCAAATATCTG GGAATAACAGTGGGTGA TACGGGTTTTCGGACCTT TGACTACTGGGGCCAGG GAACCACGGTCACCGTC TCTTCA (SEQ ID NO: 119) | - | TGCTGTGGGTGAGTGGTA CCTGTGGGCAGTCTGCCC TGACTCAGCCTCGCTCAG TGTCCGGGTCTCCTGGAC AGTCAGTCACCATCTCCT GCACTGGAACCAGCAGTG ATGTTGGGAGTTATAACC TTGTCTCCTGGTACCAAC AGCACCCAGGCAAAGCCC CCAAACTCATGATTTATG ATGTCAGTAAGCGGCCCT CAGGGGTTTCTAATCGCT TCTCTGGCTCCAAGTCTG GCAACACGGCCTCCCTGA CCATCTCCGGGCTCCAGG CTGAGGATGAGGCTGATT ATTACTGCAGCTCATATG CAGGCAGCGGGAATGTG GTATTTGGTGGAGGAACC CAGCTGATCATTTTAGGC CAACCTAAGGCCAATCCC AC (SEQ ID NO: 120) | - |
| 37 | 12B8 | CAGGTGCAGCTGGTGGA GTCTGGGGGAGGCGTGG TCCAGCCTGGGAGGTCC CTGAGACTCTCCTGTGCA GCCTCTGGATTCACCTTC AGTATCTATGCTATGCAC TGGGTCCGCCAGGCTCC AGGCAAGGGGCTAGAGT GGGTGGCAGTTGTTTCAT ATGATGGAAGCGAGAAA TACTACGCAGACTCCGT GCAGGGCCGATTCACCA TCTCCAGAGACAAGTCC AAGAACACCCTGTATCT GCAAATGAACAGCCTGA CAGCTGAGGACACGGCT GTCTATTACTGTGCGAG AGAGCCCTGGGTGGGGA CAATTGGCTACTGGGGC CAGGGAACCCTGGTCAC CGTCTCCTCA (SEQ ID NO: 121) | - | CAGTCTGCCCTGACTCAG CCTCCCTCCGCGTCCGGG TCTCCTGGACAGTCAGTC ACCATCTCCTGCACTGGA ACCAGCAGTGACGTTGGT GGTTATAACTATGTCTCC TGGTACCGACAGCACCCA GGCAAAGCCCCCAAACTC CTGATTTATGAGGTCAAT AACCGGCCCTCAGGGGTC CCTAGTCGCTTCTCTGGCT CCAAGTCTGGCAACACGG CCTCCCTGACCATCTCTG GGCTCCAGGCTGAGGATG AGGCTGATTATTACTGCA GCTCATATACAAGCAGCA GCACTTTCGTGGTACTTG GTGGAGGAACCCAGCTGA TCATTTTAGGC (SEQ ID NO: 122) | - |
| 38 | 12F7 | CAAATGCAGCTGGTGCA GTCTGGGCCTGAGGTGA | TGCTGTGGGTGA GTGGTACCTGTG | TGCTGTGGGTGAGTGGTA CCTGTGGGCAGTCTGTGC | CAGTCTGTGCT GACTCAGCCAC |
| | | AGAAGCCTGGGTCCTCG GTGAAGGTCTCCTGCAA GGCTTCGGGTTACACCTT TACCGACTACGGTCTCA GCTGGGTCCGGCAGGCC CCCGGCCACGGCCTTGA GTGGATGGGATGGATCA CCGCTTACAATGGCGAC ACAAACTATGCACAGAA GTTCCAGGACAGACTGT CCGTGACCACTGACATA TCCACGAGCACAGCCTA CATGGAATTACGGAGCC TGAAATCTGACGACACG GCCGTTTATTATTGTGCG AGACACGTCTTTTGCAGT GGTGATGGGTGTTACTC CGGCCTTGGGTCCTGGG GCCAGGGAACCCTGGTC ACCGTCTCCTCA (SEQ ID NO: 123) | GGCAGGTTCAG CTGGTGCAGTCT GGAGCTGAGGT GAAGAAGCCTG GGGCCTCAGTG AAGGTCTCCTGC AAGGCTTCGGG TTACACCTTTAC CGACTACGGTCT CAGCTGGGTCC GGCAGGCCCCC GGCCACGGCCTT GAGTGGATGGG ATGGATCACCG CTTACAATGGCG ACACAAACTAT GCACAGAAGTT CCAGGACAGAC TGTCCGTGACCA CTGACATATCCA CGAGCACAGCC TACATGGAATTA CGGAGCCTGAA ATCTGACGACA CGGCCGTTTATT ATTGTGCGAGA CACGTCTTTTGC AGTGGTGATGG GTGTTACTCCGG CCTTGGGTCCTG GGGCCAGGGAA CCCTGGTCACCG TCTCCTCAGCCT CCACCAAGGGC CCATCGGTCTTC CCCC (SEQ ID NO: 124) | TGACTCAGCCACCCTCAG CGTCTGGGACCCCCGGGC AGAGGGTCACCATCTCTT GTTCTGGAAGCAGCTCCA ACGTCGGAAGTAATACTG TCAACTGGTACCAGCAGC TCCCAGGAACGGCCCCCA AACTCCTCATCTACGATA AGAATGAGCGGCCCTCAG GGGTCCCGGACCGATTCT CTGCCTCCAAGTCTGGCA CCTCAGCCTCCCTGGCCA TCAGTGGCCTCCAGTCTG AAGATGAGGCTGATTATT ACTGTGCAGCATGGGATG ACAGCCTGAATGGTTGGG TGTTCGGTGGAGGGACCG AGCTGACCGTCCTAGGCC AACCTAAGGCCAATCCCA C (SEQ ID NO: 125) | CCTCAGCGTCT GGGGCCCCCGG GCAGCGGGTCA TCATCTCTTGTT CTGGAAGCAGC TCCAACGTCGG AAGTAATACTG TCAACTGGTAC CAGCAGCTCCC AGGAACGGCCC CCAAACTCCTC ATCTACGATAA GAATGAGCGGC CCTCAGGGGTC CCGGACCGATT CTCTGCCTCCA AGTCTGGCACC TCAGCCTCCCT GGCCATCAGTG GCCTCCAGTCT GAAGATGAGGC TGATTATTACT GTGCAGCATGG GATGACAGCCT GAATGGTTGGG TGTTCGGTGGA GGGACCAAGCT GACCGTCCTAG GC (SEQ ID NO: 126) |
| 39 | 7A11 | GAGGTGCAGCTGGTGGA GTCTGGGGGAGGCCTGG TCAAGCCTGGGGGGTCC CTGAGACTCTCCTGTGCA GCCTCTGGGTTCAGGCTC AGTAGCTATGGCATGAA CTGGGTCCGCCAGGCTC CAGGGAAGGGGCTGGAG TGGGTCTCATCCATTTCT GCTAGTAGTAGTTTTATA AACTATGCAGACTCAGT GAGGGGCCGATTCACCA TCTCCAGAGACAAGTCC AAGAACACCCTGTATCT GCAAATGAACAGCCTGA CAGCTGAGGACACGGCT GTCTATTACTGTGCGAG AGAGCCCTGGGTGGGGA CAATTGACTACTGGGGC CAGGGAACCCTGGTCAC CGTCTCCTCA (SEQ ID NO: 127) | - | CAGTCTGCCCTGACTCAG CCTGCCTCCGTGTCTGGG TCTCCTGGACAGTCGATC ACCATCTCCTGCACTGGA ACCAGCAGTGATGTTGGG AGTTATAACCTTGTCTCCT GGTACCAACAGCACCCAG GCAAAGCCCCCAAACTCC TGATTTATGAGGACACTA AGCGGCCCTCAGGGATCC CAGACCGATTCTCTGGCT CCAGCTCAGGAAACACAG CTTCCTTGACCATCACTG GGGCTCAGGCGGAAGAT GAGGCTGAGTATTACTGT AGTTCCCGGGACAGCAGT GGTAACCATCTGGTGTTC GGCGGAGGGACCAAGCT GACCGTCCTAGGC (SEQ ID NO: 128) | - |
| 40 | 4H11 | CAGGTGCAGCTGGTGCA GTCTGGAGCTGAGGTGA AGAAGCCTGGGGCCTCA GTGAAGGTCTCCTGCAA GGCTTCGGGTTACACCTT | - | TCCTATGAGCTGACTCAG CCACCCTCAGCGTCTGGG GCCCCCGGGCAGCGGGTC ATCATCTCTTGTTCTGGA AGCAGCTCCAACGTCGGA | CAGTCTGTGCT GACTCAGCCAC CCTCAGCGTCT GGGGCCCCCGG GCAGCGGGTCA |
| | | TACCGACTACGGTCTCA GCTGGGTCCGGCAGGCC CCCGGCCACGGCCTTGA GTGGATGGGATGGATCA CCGCTTACAATGGCGAC ACAAACTATGCACAGAA GTTCCAGGACAGACTGT CCGTGACCACTGACATA TCCACGAGCACAGCCTA CATGGAATTACGGAGCC TGAAATCTGACGACACG GCCGTTTATTATTGTGCG AGACACGTCTTTTGCAGT GGTGATGGGTGTTACTC CGGCCTTGGGTCCTGGG GCCAGGGAACCCTGGTC ACCGTCTCCTCA (SEQ ID NO: 129) | | AGTAATACTGTCAACTGG TACCAGCAGCTTCCAGGA AGAGCCCCCAAACTCCTC ATCTTTGCTAACACCAAT CGGCCCTCAGGGGTCCCT GACCGATTCTCTGGCTCC AAGTCTGGCACCTCAGCC TCCCTGGCCATCAGTGGC CTCCAGTCTGAAGATGAG GCTGATTATTACTGTGCA GCATGGGATGACAGCCTG AATGGTTGGGTGTTCGGT GGAGGGACCAAGCTGAC CGTCCTAGGC (SEQ ID NO: 130) | TCATCTCTTGTT CTGGAAGCAGC TCCAACGTCGG AAGTAATACTG TCAACTGGTAC CAGCAGCTTCC AGGAAGAGCCC CCAAACTCCTC ATCTTTGCTAA CACCAATCGGC CCTCAGGGGTC CCTGACCGATT CTCTGGCTCCA AGTCTGGCACC TCAGCCTCCCT GGCCATCAGTG GCCTCCAGTCT GAAGATGAGGC TGATTATTACT GTGCAGCATGG GATGACAGCCT GAATGGTTGGG TGTTCGGTGGA GGGACCAAGCT GACCGTCCTAG GC (SEQ ID NO: 131) |
| 41 | 2G3 | CAGGTGCAGCTGGTGCA GTCTGGGGCTGAGGTGA AGAAGCCGGGGGCCTCA GTGAAGGTCTCCTGCAA GGCTTCGGGTTACACCTT TACCGACTACGGTCTCA GCTGGGTCCGGCAGGCC CCCGGCCACGGCCTTGA GTGGATGGGATGGATCA CCGCTTACAATGGCGAC ACAAACTATGCACAGAA GTTCCAGGACAGACTGT CCGTGACCACTGACATA TCCACGAGCACAGCCTA CATGGAATTACGGAGCC TGAAATCTGACGACACG GCCGTTTATTATTGTGCG AGACACGTCTTTTGCAGT GGTGATGGGTGTTACTC CGGCCTTGGGTCCTGGG GCCAGGGAACCCTGGTC ACCGTCTCCTCA (SEQ ID NO: 132) | - | TCCTATGAGCTGACTCAG CCACTCTCAGCGTCTGGG ACCCCCGGGCAGAGGGTC ACCATCTCTTGTTCTGGA AGCAGCTCCAACATCGGA AGTGGTACTGTAAACTGG TACCAGCAGCTCTCAGGA ACGGCCCCCAAACTCCTC ATGCATAGTGATAATCAG CGCCCCTCAGGGGTCCCT GACCGATTCTCTGGCTCC AAGTCTGGCACCTCAGCC TCCCTGGCCATCAGTGGC CTCCAGTCTGAAGATGAG GCTGATTATTACTGTGCA GCATGGGATGACAGCCTG AATGGTTGGGTGTTCGGT GGAGGGACCAAGCTGAC CGTCCTAGGC (SEQ ID NO: 133) | CAGTCTGTGCT GACTCAGCCAC TCTCAGCGTCT GGGACCCCCGG GCAGAGGGTCA CCATCTCTTGTT CTGGAAGCAGC TCCAACATCGG AAGTGGTACTG TAAACTGGTAC CAGCAGCTCTC AGGAACGGCCC CCAAACTCCTC ATGCATAGTGA TAATCAGCGCC CCTCAGGGGTC CCTGACCGATT CTCTGGCTCCA AGTCTGGCACC TCAGCCTCCCT GGCCATCAGTG GCCTCCAGTCT GAAGATGAGGC TGATTATTACT GTGCAGCATGG GATGACAGCCT GAATGGTTGGG TGTTCGGTGGA GGGACCAAGCT GACCGTCCTAG GC (SEQ ID NO: 134) |
| 42 | 12C10 | CAGGTGCAGCTGGTGCA GTCTGGAAGTGAGGTGA AGAAGCCTGGGGCCTCA GTGAAGGTCTCCTGCAA GGCTTCGGGTTACACCTT | | CAGCCTGTGCTGACTCAG CCACCCTCAGTGTCTGGG ACCCCCGGGCAGAGGGTC ACCATCTCTTGTTCTGGA AGCAGCTCCAACATCGGA | CAGTCTGTGCT GACTCAGCCAC CCTCAGTGTCT GGGACCCCCGG GCAGAGGGTCA |
| | | TACCGACTACGGTCTCA GCTGGGTCCGGCAGGCC CCCGGCCACGGCCTTGA GTGGATGGGATGGATCA CCGCTTACAATGGTGAC ACAAACTATGCACAGAA GTTCCAGGACAGACTGT CCGTGACCACTGACATA TCCACGAGCACAGCCTA CATGGAATTACGGAGCC TGAAATCTGACGACACG GCCGTTTATTATTGTGCG AGACACGTCTTTTGCAGT GGTGATGGGTGTTACTC CGGCCTTGGGTCCTGGG GCCAGGGAACCCTGGTC ACCGTCTCCTCA (SEQ ID NO: 135) | | AGTGATACTGTAAACTGG TACCAGCAGCTCTCAGGA ACGGCCCCCAAACTCCTC ATGCATAGTGATAATCAG CGCCCCTCAGGGGTCCCT GACCGATTCTCTGGCTCC AAGTCTGGCACCTCAGCC TCCCTGGCCATCAGTGGC CTCCAGTCTGAAGATGAG GCTGATTATTACTGTGCA GCATGGGATGACAGCCTG AATGGTTGGGTGTTCGGT GGAGG GACCAAGCTGACCGTCCT AGGC (SEQ ID NO: 136) | CCATCTCTTGTT CTGGAAGCAGC TCCAACATCGG AAGTGATACTG TAAACTGGTAC CAGCAGCTCTC AGGAACGGCCC CCAAACTCCTC ATGCATAGTGA TAATCAGCGCC CCTCAGGGGTC CCTGACCGATT CTCTGGCTCCA AGTCTGGCACC TCAGCCTCCCT GGCCATCAGTG GCCTCCAGTCT GAAGATGAGGC TGATTATTACT GTGCAGCATGG GATGACAGCCT GAATGGTTGGG TGTTCGGTGGA GGG ACCAAGCTGAC CGTCCTAGGC (SEQ ID NO: 137) |
| 43 | 3E8 | CAGGTTCAGCTGGTGCA GTCTGGAAGTGAGGTGA AGAAGCCGGGGGCCTCA GTGAAGGTCTCCTGCAA GGCTTCGGGTTACACCTT TACCGACTACGGTCTCA GCTGGGTCCGGCAGGCC CCCGGCCACGGCCTTGA GTGGATGGGATGGATCA CCGCTTACAATGGCGAC ACAAACTATGCACAGAA GTTCCAGGACAGACTGT CCGTGACCACTGACATA TCCACGAGCACAGCCTA CATGGAATTACGGAGCC TGAAATCTGACGACACG GCCGTTTATTATTGTGCG AGACACGTCTTTTGCAGT GGTGATGGGTGTTACTC CGGCCTTGGGTCCTGGG GCCAGGGAACCCTGGTC ACCGTCTCCTCA (SEQ ID NO: 138) | CAGGTTCAGCTG GTGCAGTCTGG AAGTGAGGTGA AGAAGCCGGGG GCCTCAGTGAA GGTCTCCTGCAA GGCTTCGGGTTA CACCTTTACCGA CTACGGTCTCAG CTGGGTCCGGC AGGCCCCCGGC CACGGCCTTGA GTGGATGGGAT GGATCACCGCTT ACAATGGCGAC ACAAACTATGC ACAGAAGTTCC AGGACAGACTG TCCGTGACCACT GACATATCCAC GAGCACAGCCT ACATGGAATTA CGGAGCCTGAA ATCTGACGACA CGGCCGTTTATT ATTGTGCGAGA CACGTCTTTTGC AGTGGTGATGG GTGTTACTCCGG CCTTGGGTCCTG GGGCCAGGGAA CCCTGGTCACCG TCTCCTCA (SEQ ID NO: 139) | CAGCCTGTGCTGACTCAG CCACCCTCAGCGTCTGGG ACCCCCGGGCAGAGGGTC ACCATCTCTTGTTCTGGA AGCAGCTCCAACATCGGA AGTGATACTGTAAACTGG TACCAGCAGCTCTCAGGA ACGGCCCCCAAACTCCTC ATGCATAGTGATAATCAG CGCCCCTCAGGGGTCCCT GACCGATTCTCTGGCTCC AAGTCTGGCACCTCAGCC TCCCTGGCCATCAGTGGC CTCCAGTCTGAAGATGAG GCTGATTATTACTGTGCA GCATGGGATGACAGCCTG AATGGTTGGGTGTTCGGT GGAGGGACCAAGCTGAC CGTCCTAGGC (SEQ ID NO: 140) | CAGTCTGTGCT GACTCAGCCAC CCTCAGCGTCT GGGACCCCCGG GCAGAGGGTCA CCATCTCTTGTT CTGGAAGCAGC TCCAACATCGG AAGTGATACTG TAAACTGGTAC CAGCAGCTCTC AGGAACGGCCC CCAAACTCCTC ATGCATAGTGA TAATCAGCGCC CCTCAGGGGTC CCTGACCGATT CTCTGGCTCCA AGTCTGGCACC TCAGCCTCCCT GGCCATCAGTG GCCTCCAGTCT GAAGATGAGGC TGATTATTACT GTGCAGCATGG GATGACAGCCT GAATGGTTGGG TGTTCGGTGGA GGGACCAAGCT GACCGTCCTAG GC (SEQ ID NO: 141) |
| 44 | 7D1 | CAGGTGCAGCTGGTGCA GTCTGGAGCTGAGGTGA AGAAGCCGGGGGCCTCA | - | CAGTCTGTGCTGACTCAG CCACCCTCAGCGTCTGGG ACCCCCGGGCAGAGGGTC | - |
| | | GTGAAAGTCTCCTGCAA GGCTTCGGGTTACACCTT TACCGACTACGGTCTCA GCTGGGTCCGGCAGGCC CCCGGCCACGGCCTTGA GTGGATGGGATGGATCA CCGCTTACAATGGCGAC ACAAACTATGCACAGAA GTTCCAGGACAGACTGT CCGTGACCACTGACATA TCCACGAGCACAGCCTA CATGGAATTACGGAGCC TGAAATCTGACGACACG GCCGTTTATTATTGTGCG AGACACGTCTTTTGCAGT GGTGATGGGTGTTACTC CGGCCTTGGGTCCTGGG GCCAGGGAACCCTGGTC ACCGTCTCCTCA (SEQ ID NO: 142) | | ACCATCTCTTGTTCTGGA AGCAGCTCCAACATCGGA AGTAATACTGTAAACTGG TACCAGCAGCTCCCAGGA ACGGCCCCCAAACTCCTC ATCTATAGTAATAATCAG CGGCCCTCAGGGGTCCCT GACCGATTCTCTGGCTCC ACGTCTGGCACCTCAGCC TCCCTGGCCATCAGTGGA CTCCAGTCTGAAGATGAG GCTGATTATTACTGTGCA GCATGGGATGACAGCCTG AATGGTTGGGTGTTCGGT GGAGGGACCAAGCTGAC CGTCCTAGGC (SEQ ID NO: 143) | |
| 45 | 4G5 | CAGGTGCAGCTGGTGCA GTCTGGAAGTGAGGTGA AGAAGCCTGGGGCCTCA GTGAAGGTCTCCTGCAA GGCTTCGGGTTACACCTT TACCGACTACGGTCTCA GCTGGGTCCGGCAGGCC CCCGGCCACGGCCTTGA GTGGATGGGATGGATCA CCGCTTACAATGGCGAC ACAAACTATGCACAGAA GTTCCAGGACAGACTGT CCGTGACCACTGACATA TCCACGAGCACAGCCTA CATGGAATTACGGAGCC TGAAATCTGACGACACG GCCGTTTATTATTGTGCG AGACACGTCTTTTGCAGT GGTGATGGGTGTTACTC CGGCCTTGGGTCCTGGG GCCAGGGAACCCTGGTC ACCGTCTCCTCA(SEQ ID NO: 144) | - | TCCTATGAGCTGACTCAG CCACTCTCAGCGTCTGGG ACCCCCGGGCAGAGGGTC ACCATCTCTTGTTCTGGA AGCAGCTCCAACATCGGA AGTAATACTGTAAACTGG TACCAGCAGCTCCCAGGA ACGGCCCCCAAACTCCTC ATCTATAGTAATAATCAG CGGCCCTCAGGGGTCCCT GACCGATTCTCTGGCTCC AAGTCTGGCACCTCAGCC TCCCTGGCCATCAGTGGG CTCCAGTCTGAGGATGAG GCTGATTATTACTGTGCA GCATGGGATGACAGCCTG AATGGTTGGGTGTTCGGT GGAGGGACCAAGCTGAC CGTCCTAGGC (SEQ ID NO: 145) | CAGTCTGTGCT GACTCAGCCAC TCTCAGCGTCT GGGACCCCCGG GCAGAGGGTCA CCATCTCTTGTT CTGGAAGCAGC TCCAACATCGG AAGTAATACTG TAAACTGGTAC CAGCAGCTCCC AGGAACGGCCC CCAAACTCCTC ATCTATAGTAA TAATCAGCGGC CCTCAGGGGTC CCTGACCGATT CTCTGGCTCCA AGTCTGGCACC TCAGCCTCCCT GGCCATCAGTG GGCTCCAGTCT GAGGATGAGGC TGATTATTACT GTGCAGCATGG GATGACAGCCT GAATGGTTGGG TGTTCGGTGGA GGGACCAAGCT GACCGTCCTAG GC (SEQ ID NO: 146) |
| 46 | 2E8 | CAGGTGCAGCTGGTGCA GTCTGGGCCTGAGGTGA AGAAGCCTGGGTCCTCG GTGAAGGTCTCCTGCAA GGCTTCGGGTTACACCTT TACCGACTACGGTCTCA GCTGGGTCCGGCAGGCC CCCGGCCACGGCCTTGA GTGGATGGGATGGATCA CCGCTTACAATGGCGAC ACAAACTATGCACAGAA GTTCCAGGACAGACTGT | - | TCCTATGAGCTGACTCAG CCACCCTCAGCGTCTGGG GCCCCCGGGCAGCGGGTC ATCATCTCTTGTTCTGGA AGCAGCTCCAACGTCGGA AGTAATACTGTCAACTGG TACCAGCAGCTCCCAGGA ACGGCCCCCAAACTCCTC ATCTACGATAAGAATGAG CGGCCCTCAGGGGTCCCG GACCGATTCTCTGCCTCC AAGTCTGGCACCTCAGCC | CAGTCTGTGCT GACTCAGCCAC CCTCAGCGTCT GGGGCCCCCGG GCAGCGGGTCA TCATCTCTTGTT CTGGAAGCAGC TCCAACGTCGG AAGTAATACTG TCAACTGGTAC CAGCAGCTCCC AGGAACGGCCC |
| | | CCGTGACCACTGACATA TCCACGAGCACAGCCTA CATGGAATTACGGAGCC TGAAATCTGACGACACG GCCGTTTATTATTGTGCG AGACACGTCTTTTGCAGT GGTGATGGGTGTTACTC CGGCCTTGGGTCCTGGG GCCAGGGAACCCTGGTC ACCGTCTCCTCA(SEQ ID NO: 147) | | TCCCTGGCCATCAGTGGC CTCCAGTCTGAAGATGAG GCTGATTATTACTGTGCA GCATGGGATGACAGCCTG AATGGTTGGGTGTTCGGT GGAGGGACCAAGCTGAC CGTCCTAGGC (SEQ ID NO: 148) | CCAAACTCCTC ATCTACGATAA GAATGAGCGGC CCTCAGGGGTC CCGGACCGATT CTCTGCCTCCA AGTCTGGCACC TCAGCCTCCCT GGCCATCAGTG GCCTCCAGTCT GAAGATGAGGC TGATTATTACT GTGCAGCATGG GATGACAGCCT GAATGGTTGGG TGTTCGGTGGA GGG ACCAAGCTGAC CGTCCTAGGC (SEQ ID NO: 149) |
| 47 | 13H9 | CAGGTTCAGCTGGTGCA GTCTGGAGCTGAGGTGA AGAAGCCTGGGGCCTCA GTGAAGGTCTCCTGCAA GGCTTCTGGTTACACCG GTTACACCTTTACCGACT ACGGTCTCAGCTGGGTC CGGCAGGCCCCCGGCCA CGGCCTTGAGTGGATGG GATGGATCACCGCTTAC AATGGCGACACAAACTA TGCACAGAAGTTCCAGG ACAGACTGTCCGTGACC ACTGACATATCCACGAG CACAGCCTACATGGAAT TACGGAGCCTGAAATCT GACGACACGGCCGTTTA TTATTGTGCGAGACACG TCTTTTGCAGTGGTGATG GGTGTTACTCCGGCCTTG GGTCCTGGGGCCAGGGA ACCCTGGTCACCGTCTCC TCA(SEQ ID NO: 150) | - | TCCTATGAGCTGATACAG CCACCCTCAGCGTCTGGG GCCCCCGGGCAGCGGGTC ATCATCTCTTGTTCTGGA AGCAGCTCCAACGTCGGA AGTAATACTGTCAACTGG TACCAGCAGCTCCCAGGA ACGGCCCCCAAACTCCTC ATCTACGATAAGAATGAG CGGCCCTCAGGGGTCCCG GACCGATTCTCTGCCTCC AAGTCTGGCACCTCAGCC TCCCTGGCCATCAGTGGC CTCCAGTCTGAAGATGAG GCTGATTATTACTGTGCA GCATGGGATGACAGCCTG AATGGTTGGGTGTTCGGT GGAGGGACCGAGCTGAC CGTCCTAGGC (SEQ ID NO: 151) | CAGTCTGTGCT GACTCAGCCAC CCTCAGCGTCT GGGGCCCCCGG GCAGCGGGTCA TCATCTCTTGTT CTGGAAGCAGC TCCAACGTCGG AAGTAATACTG TCAACTGGTAC CAGCAGCTCCC AGGAACGGCCC CCAAACTCCTC ATCTACGATAA GAATGAGCGGC CCTCAGGGGTC CCGGACCGATT CTCTGCCTCCA AGTCTGGCACC TCAGCCTCCCT GGCCATCAGTG GCCTCCAGTCT GAAGATGAGGC TGATTATTACT GTGCAGCATGG GATGACAGCCT GAATGGTTGGG TGTTCGGTGGA GGGACCAAGCT GACCGTCCTAG GC (SEQ ID NO: 152) |
| 48 | 4E9 | CAGGTGCAGCTGGTGCA GTCTGGAGCTGAGGTGA AGAAGCCTGGGTCCTCG GTGAAGGTCTCCTGCAA GGCTTCGGGTTACACCTT TACCGACTACGGTCTCA GCTGGGTCCGGCAGGCC CCCGGCCACGGCCTTGA GTGGATGGGATGGATCA CCGCTTACAATGGCGAC ACAAACTATGCACAGAA GTTCCAGGACAGACTGT | - | CAGTCTGTGCTGACGCAG CCGCCCTCAGTGTCTGGG GCCCCAGGGCAGAGGGTC ACCATCTCTTGTTCTGGA AGCAGCTCCAACGTCGGA AGTAATACTGTCAACTGG TACCAGCAGCTCCCAGGA ACGGCCCCCAAACTCCTC ATCTACGATAAGAATGAG CGGCCCTCAGGGGTCCCG GACCGATTCTCTGCCTCC AAGTCTGGCACCTCATCC | - |
| | | CCGTGACCACTGACATA TCCACGAGCACAGCCTA CATGGAATTACGGAGCC TGAAATCTGACGACACG GCCGTTTATTATTGTGCG AGACACGTCTTTTGCAGT GGTGATGGGTGTTACTC CGGCCTTGGGTCCTGGG GCCAGGGAACCCTGGTC ACCGTCTCCTCA(SEQ ID NO: 153) | | TCCCTGGCCATCGGTGGG CTCCGGTCTGAAGATGAG GCTGATTATTACTGTGGG ACATGGGATGACAACCTG AATGGTTGGGTGTTCGGC GGAGGGACCAAGCTGAC CGTCCTAGGC (SEQ ID NO: 154) | |
| 49 | 4H7 | CAGGTGCAGCTGGTGCA GTCTGGAGCTGAGGTGA AGAAGCCTGGGGCCTCA GTGAAGGTCTCCTGCAA GGCTTCGGGTTACACCTT TACCGACTACGGTCTCA GCTGGGTCCGGCAGGCC CCCGGCCACGGCCTTGA GTGGATGGGATGGATCA CCGCTTACAATGGCGAC ACAAACTATGCACAGAA GTTCCAGGACAGACTGT CCGTGACCACTGACATA TCCACGAGCACAGCCTA CATGGAATTACGGAGCC TGAAATCTGACGACACG GCCGTTTATTATTGTGCG AGACACGTCTTTTGCAGT GGTGATGGGTGTTACTC CGGCCTTGGGTCCTGGG GCCAGGGAACCCTGGTC ACCGTCTCCTCA(SEQ ID NO: 155) | - | TCCTATGAGCTGACTCAG CCACCCTCAGCGTCTGGG GCCCCCGGGCAGCGGGTC ATCATCTCTTGTTCTGGA AGCAGCTCCAACGTCGGA AGTAATACTGTCAACTGG TACCAGCAGCTCCCAGGA ACGGCCCCCAAACTCCTC ATCTACGATAAGAATGAG CGGCCCTCAGGGGTCCCG GACCGATTCTCTGCCTCC AAGTCTGGCACCTCATCC TCCCTGGCCATCGGTGGG CTCCGGTCTGAAGATGAG GCTGATTATTACTGTGCG ACATGGGATGACAACCTG AATGGTTGGGTGTTCGGC GGAGGGACCAAGCTGAC CGTCCTAGGC (SEQ ID NO: 156) | CAGTCTGTGCT GACTCAGCCAC CCTCAGCGTCT GGGGCCCCCGG GCAGCGGGTCA TCATCTCTTGTT CTGGAAGCAGC TCCAACGTCGG AAGTAATACTG TCAACTGGTAC CAGCAGCTCCC AGGAACGGCCC CCAAACTCCTC ATCTACGATAA GAATGAGCGGC CCTCAGGGGTC CCGGACCGATT CTCTGCCTCCA AGTCTGGCACC TCATCCTCCCTG GCCATCGGTGG GCTCCGGTCTG AAGATGAGGCT GATTATTACTG TGCGACATGGG ATGACAACCTG AATGGTTGGGT GTTCGGCGGAG GGACCAAGCTG ACCGTCCTAGG C (SEQ ID NO: 157) |
| 50 | 2F8 | CAGGTGCAGCTGGTGCA GTCTGGAGCTGAGGTGA AGAAGCCTGGGGCCTCA GTGAAGGTCTCCTGCAA GGCTTCGGGTTACACCTT TACCGACTACGGTCTCA GCTGGGTCCGGCAGGCC CCCGGCCACGGCCTTGA GTGGATGGGATGGATCA CCGCTTACAATGGCGAC ACAAACTATGCACAGAA GTTCCAGGACAGACTGT CCGTGACCACTGACATA TCCACGAGCACAGCCTA CATGGAATTACGGAGCC TGAAATCTGACGACACG GCCGTTTATTATTGTGCG AGACACGTCTTTTGCAGT GGTGATGGGTGTTACTC CGGCCTTGGGTCCTGGG GCCAGGGAACCCTGGTC | - | TCCTATGAGCTGACTCAG CCACCCTCAGCGTCTGGG GCCCCCGGGCAGCGGGTC ATCATCTCTTGTTCTGGA AGCAGCTCCAACGTCGGA AGTAATACTGTCAACTGG TACCAGCAGCTCCCAGGA ACGGCCCCCAAACTCCTC ATCTACGATAAGAATGAG CGGCCCTCAGGGGTCCCG GACCGATTCTCTGCCTCC AAGTCTGGCACCTCATCC TCCCTGGCCATCGGTGGG CTCCGGTCTGAAGATGAG GCTGATTATTACTGTGGG ACATGGGATGACAACCTG AATGGTTGGGTGTTCGGC GGAGGGACCAAGCTGAC CGTCCTAGGC (SEQ ID NO: 159) | CAGTCTGTGCT GACTCAGCCAC CCTCAGCGTCT GGGGCCCCCGG GCAGCGGGTCA TCATCTCTTGTT CTGGAAGCAGC TCCAACGTCGG AAGTAATACTG TCAACTGGTAC CAGCAGCTCCC AGGAACGGCCC CCAAACTCCTC ATCTACGATAA GAATGAGCGGC CCTCAGGGGTC CCGGACCGATT CTCTGCCTCCA AGTCTGGCACC TCATCCTCCCTG GCCATCGGTGG |
| | | ACCGTCTCCTCA(SEQ ID NO: 158) | | | GCTCCGGTCTG AAGATGAGGCT GATTATTACTG TGGGACATGGG ATGACAACCTG AATGGTTGGGT GTTCGGCGGAG GG ACCAAGCTGAC CGTCCTAGGC (SEO ID NO: 160) |
| 51 | 3E7 | GAGGTGCAGCTGGTGGA GTCTGGGACTGAGGTGA AGAAGCCTGGGGCCTCA GTGAAGGTCTCCTGCAA GGCTTCGGGTTACACCTT TACCGACTACGGTCTCA GCTGGGTCCGGCAGGCC CCCGGCCACGGCCTTGA GTGGATGGGATGGATCA CCGCTTACAATGGCGAC ACAAACTATGCACAGAA GTTCCAGGACAGACTGT CCGTGACCACTGACATA TCCACGAGCACAGCCTA CATGGAATTACGGAGCC TGAAATCTGACGACACG GCCGTTTATTATTGTGCG AGACACGTCTTTTGCAGT GGTGATGGGTGTTACTC CGGCCTTGGGTCCTGGG GCCAGGGAACCCTGGTC ACTGTCTCCTCA (SEQ ID NO: 161) | TGCTGTGGGTGA GTGGTACCTGTG GGGAGGTGCAG CTGGTGCAGTCT GGGGCTGAGGT GAAGAAGCCTG GGGCCTCAGTG AAGGTCTCCTGC AAGGCTTCGGG TTACACCTTTAC CGACTACGGTCT CAGCTGGGTCC GGCAGGCCCCC GGCCACGGCCTT GAGTGGATGGG ATGGATCACCG CTTACAATGGCG ACACAAACTAT GCACAGAAGTT CCAGGACAGAC TGTCCGTGACCA CTGACATATCCA CGAGCACAGCC TACATGGAATTA CGGAGCCTGAA ATCTGACGACA CGGCCGTTTATT ATTGTGCGAGA CACGTCTTTTGC AGTGGTGATGG GTGTTACTCCGG CCTTGGGTCCTG GGGCCAGGGAA CCCTGGTCACTG TCTCCTCAGCCT CCACCAAGGGC CCATCGGTCTTC CCCC (SEQ ID NO: 162) | CAGTCTGTGCTGACTCAG CCACCCTCAGCGTCTGGG GCCCCCGGGCAGCGGGTC ATCATCTCTTGTTCTGGA AGCAGCTCCAACGTCGGA AGTAATACTGTCAACTGG TACCAGCAGCTCCCAGGA ACGGCCCCCAAACTCCTC ATCTACGATAAGAATGAG CGGCCCTCAGGGGTCCCG GACCGATTCTCTGCCTCC AAGTCTGGCACCTCATCC TCCCTGGCCATCGGTGGG CTCCGGTCTGAAGATGAG GCTGATTATTACTGTGGG ACATGGGATGACAACCTG AATGGTTGGGTGTTCGGC GGAGGGACCAAGCTGAC CGTCCTAGGC (SEQ ID NO: 163) | - |
| 52 | 9H4 | CAGGTGCAGCTGGTGCA GTCTGGGGGAGGCTTGG TACAGCCTGGAGGGTCC CTGAGAATCTCCTGTAC AGCCTCTGGATTCACCTT CAGGAATTATGAAATGA ATTGGGTCCGCCAGGCT CCAGGGAAGGGGCTGGA GTGGGTTGCATACATTA GTAGTAGTGGTAGTTCC AGATACTACGCAGACTC TGTGAAGGGCCGATTCA CCATCTCCAGAGACAAC GCCAAGAACTCACTGTT | GAGAGGTGCAG CTGGTGGAGTCT GGGGGAGGCTT GGTACAGCCTG GAGGGTCCCTG AGAATCTCCTGT ACAGCCTCTGG ATTCACCTTCAG GAATTATGAAA TGAATTGGGTCC GCCAGGCTCCA GGGAAGGGGCT GGAGTGGGTTG CATACATTAGTA | GAAATTGTGCTGACCCAG TCTCCAGGCATCCAGTCT TTGTCTCCAGGGGAAACA GCCACCCTCTCCTGCAGG GCCAGTGAGAGTATTAGC AGCAGTTACTTCGCCTGG TACCAGCAGAAACCTGGC CAGGCTCCCAGGCTCCTC ATCTATGGTGCATCCAGC AGGGCCTCTGGCATCCCA GACAGGTTCAGTGGCAGT GGGTCTGGGACAGACTTC ACTCTCACCATCAGCAGA CTGGAGCCTGAAGATTTT | - |
| | | TCTGCAAATGAACAGCC TGAGAGCCGAGGACATG GCTGTTTATTACTGTGCG AGGACGGACGACGGCAG CAGCTGGTTCGTGTCCAC CAGTAGTTTCTACGGTAT GGACGTCTGGGGCCAAG GGACCACGGTCACCGTC TCCTCA (SEQ ID NO: 164) | GTAGTGGTAGTT CCAGATACTAC GCAGACTCTGTG AAGGGCCGATT CACCATCTCCAG AGACAACGCCA AGAACTCACTGT TTCTGCAAATGA ACAGCCTGAGA GCCGAGGACAT GGCTGTTTATTA CTGTGCGAGGA CGGACGACGGC AGCAGCTGGTTC GTGTCCACCAGT AGTTTCTACGGT ATGGACGTCTG GGGCCAAGGGA CCACGGTCACC GTCTCCTCA (SEQ ID NO: 165) | GCAGTGTATTACTGTCAG CAGTATGGTAGCTCACCT CCCATCACCTTCGGC CAAGGGACACGACTGGA GATTAAACGA (SEQ ID NO: 166) | |
| 53 | 8B1 | CAGGTGCAGCTGGTGCA GTCT GGGGGAGGCTTGGTACA GCCTGGAGGGTCCCTGA GAATCTCCTGTACAGCCT CTGGATTCACCTTCAGG AATTATGAAATGAATTG GGTCCGCCAGGCTCCAG GGAAGGGGCTGGAGTGG GTTGCATACATTAGTAGT AGTGGTAGTTCCAGATA CTACGCAGACTCTGTGA AGGGCCGATTCACCATC TCCAGAGACAACGCCAA GAACACGCTGTATCTGC AAATGAGCAGCCTGAGA TCTGACGACACGGCCGT GTATTACTGTGCGAGGA CGGACGACGGCAGCAGC TGGTTCGTGTCCACCAGT AGTTTCTACGGTATGGA CGTCTGGGGCCAAGGGA CCACGGTCACCGTCTCCT CA(SEQ ID NO: 167) | GAGAGGTGCAG CTGGTGGAGTCT GGGGGAGGCTT GGTACAGCCTG GAGGGTCCCTG AGAATCTCCTGT ACAGCCTCTGG ATTCACCTTCAG GAATTATGAAA TGAATTGGGTCC GCCAGGCTCCA GGGAAGGGGCT GGAGTGGGTTG CATACATTAGTA GTAGTGGTAGTT CCAGATACTAC GCAGACTCTGTG AAGGGCCGATT CACCATCTCCAG AGACAACGCCA AGAACACGCTG TATCTGCAAATG AGCAGCCTGAG ATCTGACGACA CGGCCGTGTATT ACTGTGCGAGG ACGGACGACGG CAGCAGCTGGTT CGTGTCCACCAG TAGTTTCTACGG TATGGACGTCTG GGGCCAAGGGA CCACGGTCACC GTCTCCTCA (SEQ ID NO: 168) - | GATGTTGTGATGACTCAG TCTCCA GGCACCCTGTCTTTGTCTC CAGGGGAAAGAGCCACC CTCTCCTGCAGGGCCAGT CAGAGTGTTAGTAGCAGC TACTTAGCCTGGTACCAG CAGAAACCTGGCCAGGCT CCCAGGCTCCTCATCTAC GGTGTGTCCAGCAGGGCC ACTGGCATCCCAGACAGG TTCAGTGGCAGTGGGTCT GGGACAGACTTCACTCTC ACCATCAGCAGACTGGAG CCTGAAGATTTTGCAGTG TATTACTGTCAGCAGTAT GGTAGCTCACCTCCCATC ACCTTCGGCCAGGGGACC AAGCTGGAGATCAAACG A (SEQ ID NO: 169) | GAGATTGTGCT GACCCAGTCTC CAGGCACCCTG TCTTTGTCTCCA GGGGAAAGAGC CACCCTCTCCT GCAGGGCCAGT CAGAGTGTTAG TAGCAGCTACT TAGCCTGGTAC CAGCAGAAACC TGGCCAGGCTC CCAGGCTCCTC ATCTACGGTGT GTCCAGCAGGG CCACTGGCATC CCAGACAGGTT CAGTGGCAGTG GGTCTGGGACA GACTTCACTCT CACCATCAGCA GACTGGAGCCT GAAGATTTTGC AGTGTATTACT GTCAGCAGTAT GGTAGCTCACC TCCCATCACCTT CGGCCAG GGG ACC AAG CTG GAG ATC AAA CGT (SEQ ID NO: 170) |
| 54 | 7G12 | GAGGTGCAGCTGGTGGA GTCTGGGGGAGGCTTGG TACAGCCTGGGGGGTCC CTGAGACTCTCCTGTGCA GCC TCTGAATTCACCTTTAGC ATGAACTGGGTCCGACA GGCTCCAGGGAAGGGGC | - | GACATCCAGTTGACCCAG TCTCCATCTTCCGTGTCTG CATCTGTAGGAGAGAGAG TCACCATCACTCGTCGGG CGAGTCAGAATATTGACA GGTGGTTAGCTTGGTATC AGCAGAAACCAGGGAAA GCCCCTGACCTCCTGATC | - |
| | | TGGAGTGGGTCTCATCA GTTCGAGGTGGCGGTAC TGAAACATACTATGCAG ACTCCGTGAAGGGCCGG TTCACCGTCTCCAGAGA CAATTCCAAGAACACAC TGTATCTGCAGATGAAC AGCCTGAGAGTCGAGGA CACGGCCGTGTATTACT GTACGGTGTGTGTTGTTA TGTTTCGGGGATGGTGC AACTGGTTCGACCCCTG GGGCCAGGGAACCCTGG TCACCGTCTCCTCA (SEQ ID NO: 171) | | TTTGCTGCATCCAGTTTGC AGAGTGGGGTCCCATCAA GGTTCAGCGGCAGTGGAT CTGGGACAGATTTCACTC TCACCATCAGCAGCCTGC AGCCTGAAGATTTTGCAA CTTACTATTGTCAACAGG GTAAAACTTTCCCTCCCA CGTTCGGCCAGGGGACCA AGCTGGAGATCAAACGA (SEQ ID NO: 172) | |
| 55 | 12F11 | GAGGTGCAGCTGTTGGA GTCTGGGGGAGGCTTGG TACAGCCTGGGGGGTCC CTGAGACTCTCCTGTGCA GCCTCTGAATTCACCTTT AGCATGAACTGGGTCCG ACAGGCTCCAGGGAAGG GGCTGGAGTGGGTCTCA TCAGTTCGAGGTGGCGG TACTGAAACATACTATG CAGACTCCGTGAAGGGC CGGTTCACCGTCTCCAG AGACAATTCCAAGAACA CACTGTATCTGCAGATG AACAGCCTGAGAGTCGA GGACACGGCCGTGTATT ACTGTACGGTGTGTGTTG TTATGTTTCGGGGATGGT GCAACTGGTTCGACCCC TGGGGCCAGGGAACCCT GGTCACCGTCTCCTCA(S EQ ID NO: 173) | - | GATGTTGTGATGACTCAG TCTCCATCGTCCCTGTCTG CATCCGTTGGGGACAGAG TCACCATCACTTGCCGGG CAAGTCAGAGCCTCAGTA GTTATTTAAATTGGTATC AGCAGAAACCAGGGAAA GCCCCTAAACTGCTCATC TATGCTACAACCAACTTG CAAAGTGGGGTCCCTTCA AGGTTCAGTGGCAGTGGA TCTGGGACACATTTCACT CTCACCATCGGGAGTCTG CAACCTGAAGATTTTGCA ACTTATTACTGTCAACAG AGTTTCCAGACCCCGCTC ACTTTCGGCGGAGGGACC AAGGTGGAGATCAAACG A (SEQ ID NO: 174) | GACATCCAGAT GACCCAG TCTCCATCGTCC CTGTCTGCATC CGTTGGGGACA GAGTCACCATC ACTTGCCGGGC AAGTCAGAGCC TCAGTAGTTAT TTAAATTGGTA TCAGCAGAAAC CAGGGAAAGCC CCTAAACTGCT CATCTATGCTA CAACCAACTTG CAAAGTGGGGT CCCTTCAAGGT TCAGTGGCAGT GGATCTGGGAC ACATTTCACTCT CACCATCGGGA GTCTGCAACCT GAAGATTTTGC AACTTATTACT GTCAACAGAGT TTCCAGACCCC GCTCACTTTCG GCGGAGGG ACC AAG CTG GAG ATC AAA CGT (SEQ ID NO: 175) |
| 56 | 5B12 | CAGGTGCAGCTGGTGGA GTCTGGGGGAGGCGTGG TCCAGCCTGGGAGGTCC CTGAGACTCTCCTGTGCA GCCTCTGGATTCACCTTC ATTAGCTATGTCATGCAC TGGGTCCGCCAGGCTCC AGGCAAGGGGCTGGAGT GGGTGGCAGTTGTTTCAT ATGATGGAAGCGAGAAA TACTACGCAGACTCCGT GAAGGGCCGATTCACCA TCTCCAGAGACAATTCC AAGAACACGCTGTATCT GCAAATGAACAGCCTGA CAGCTGAGGACACGGCT GTCTATTACTGTGCGAG | - | GACATCCAGATGACCCAG TCTCCAGCCACCCTGTCT GCATCTGTAGGAGACAGA GTCACCATCACTTGCCGG GCAAGTCAGAGCATTAAC AACAATTTAAATTGGTAT CAACAGAAACCAGGGCA AGCCCCTAAGCTCCTGAT CTATGCTGCATCCACTTT ACAAGGTGGGGTCCCATC AAGGTTCAGCGGCAGTGG ATCTGGGACAGAATTCAC TCTCACAATCAGCAGCCT GCAGCCTGAAGATTTTGC AACTTATTACTGTCAACA GCTTAATGGTTACCCTCT CACTTTCGGCGGAGGGAC | - |
| | | AGAGCCCTGGGTGGGGA CAATTGACTACTGGGGC CAGGGAACCCTGGTCAC CGTCTCCTCA(SEQ ID NO: 176) | | CAAGGTGGAGATCAAAC GA (SEQ ID NO: 177) | |
| 57 | 8C3 | GAGGTGCAGCTGTTGGA GTCTGGGGGAGGCTTGG TACAGCCTGGGGGGTCC CTGAGACTCTCCTGTGCA GCCTCTGGATTCACCTTT AGCGACTATGCCATGAG TTGGGTCCGCCAGGCTC CAGGGAAGGGGCTGGAG TGGGTCTCAAGTATTACT GCTAATGGACTTATGAC ATACTACACAGACTCCG TGAAGGGCCGATTCACC ATCTCCCGAGACAATTC CAAGAACACGCTGTATC TGCAAATGAGCAGCCTG AGAACTGACGACACGGC TGTATATTACTGTGCGAA AGATGCCGGATGGGAGG CTTGGTGGTACTTCGACC TCTGGGGCCGTGGCACC CTGGTCACCGTCTCCTCA (SEQ ID NO: 178) | - | GACATCCAGATGACCCAG TCTCCAGACTCCCTGGCT GTGTCTCTGGGCGAGAGG GCCACCATCAACTGCAAG TCCAGCCAGAGTGTTTTA TACAGCTCCAACAATAAG AACTACTTAGCTTGGTAC CAGCAGAAACCAGGACA GCCTCCTAAGATGCTCAT TTACTGGGCATCTATCCG GGAAACCGGGGTCCCTGA CCGATTCAGTGGCAGCGG GTCTGAGACAGATTTCAC TCTCACCATCAGCAGCCT GCAGGCTGAAGATGTGGC AGTTTATTACTGTCAGCA ATATTATAGTACTCTCAC TTTCGGCGGAGGGACCAA GGTGGAGATCAAACGA (SEQ ID NO: 179) | - |
| 58 | 2F4 | CAGGTGCAGCTGGTGCA GTCTGGGGCTGAGGTGA AGAAGCCTGGGGCCTCA GTGAAGGTTTCCTGCAA GGCATCTGGATACACCT TCACCAGCTACTATATGC GCTGGCTGCGACAGGCC CCTGGACAAGGGCTTGA GTGGATGGGAATAATCG ACCCTAGTGGTGGTAGC ACAACCTATGCACAGAA GTTCCAGGGCAGAGTCA GCATAACCAGGGACACG TCCACGAGCACAGTCTA CATGGAGCTGAGCAGCC TGAGATCTGAGGACACG GCCGTGTATTACTGTGCG AGAGGGAGGGGCGTAGT ACCAGCTGGCAACCCCT CAACGGGGGGGGTCGGC ATGGACGTCTGGGGCCA AGGGACCACGGTCACCG TCTCCTCA(SEQ ID NO: 180) | - | GAGATTGTGCTGACCCAG TCTCCAGCCACCGTGTCT CTGTCTCCCGGGGAAAGA ACCATGTTGTCTTGCAGG GCCAGTCAGAATGTTAGT AACTACTTAGGGTGGTAT CAGCAGAGACGTGGCCA GCCTCCCAGACTCCTCAT TTCCGATGCGTCCAACAG GGCCTCTGGCGTCCCAGC CAGGTTCAGTGGAAGTGG GTCTGGGACAGACTTCAC TCTTACTATCACCAGTCTT CAGCCTGAAGATTTTGCA GTTTATTTCTGTCAGCACC GGAGCAGCTGGCCCGTCA CTTTCGGCGGAGGG ACCAAGGTGGAGATCAA ACGA (SEQ ID NO: 181) | - |
| 59 | 1C10 | CAGGTGCAGCTGGTGCA GTCTGGAAGTGAGGTGA AGAAGCCTGGGGCCTCA GTGAAGGTCTCCTGCAA GGCCTCTGGATACACCTT TGGTCATTATGGTATTAG TTGGGTGCGCCAGGCCC CTGGACAAGGCCTTGAG TGGATGGCCTGGATCAG CGCTTACAATGGTAACA CAGACTCCATACAGAAG GTCCAGGGCAGAGTCAC CATGACCACAGACACAT | - | GATGTTGTGATGACTCAG TCTCCACTCTCCCTGCCCG TCACCCTTGGCCAGCCGG CCTCCATCTCCTGCAGGT CTAGTCAAAGCCTCGTCT TCAGTGATGGAAACACCT ACTTGAGTTGGTTTCAAC AGAGGCCAGGCCAATCTC CAAGGCGCCTAATTTATA AGGTTTCTAACCGGGACT CTGGGGTCCCAGACAGAT TCAGCGCCAGTGGGTCAG GCACTGATTTCACACTGA | - |
| | | CCACGAACACAGCCTAC TTGGAATTGAGGAGCCT GAGATCTGACGACACGG CCGTGTATTACTGTGTAA GAGATGTCCCGGCCACA GGAGGAGCTGCCACGGC TGACTACTGGGGCCAGG GAACCCTGGTCACCGTC TCCTCA (SEQ ID NO: 182) | | AAATCAGCAGGGTGGAG GCTGACGATGTTGGGGTT TATTACTGCATGCAAGTT ACACACTGGCCTCGGACT TTTGGCCAG GGGACCAAGCTGGAGATC AAACGA (SEQ ID NO: 183) | |
| 60 | 5E9 | CAGGTGCAGCTGGTGCA GTCTGGGCCTGAGGTGA AGAAGCCTGGGTCCTCA GTGAAGGTCTCCTGCAA GGCCTCTGGATACACCTT TGGTCATTATGGTATTAG TTGGGTGCGCCAGGCCC CTGGACAAGGCCTTGAG TGGATGGCCTGGATCAG CGCTTACAATGGTAACA CAGACTCCATACAGAAG GTCCAGGGCAGAGTCAC CATGACCACAGACACAT CCACGAACACAGCCTAC TTGGAATTGAGGAGCCT GAGATCTGACGACACGG CCGTGTATTACTGTGTAA GAGATGTCCCGGCCACA GGAGGAGCTGCCACGGC TGACTACTGGGGCCAGG GAACCACGGTCACCGTC TCCTCA(SEQ ID NO: 184) | - | GATGTTGTGATGACTCAG TCTCCACTCTCCCTGCCCG TCACCCTTGGCCAGCCGG CCTCCATCTCCTGCAGGT CTAGTCAAAGCCTCGTCT TCAGTGATGGAAACACCT ACTTGAGTTGGTTTCAAC AGAGGCCAGGCCAATCTC CAAGGCGCCTAATTTATA AGGTTTCTAACCGGGACT CTGGGGTCCCAGACAGAT TCAGCGCCAGTGGGTCAG GCACTGATTTCACACTGA AAATCAGCAGGGTGGAG GCTGACGATGTTGGGGTT TATTACTGCATGCAAGTT ACACACTGGCCTCGGACT TTTGGCCAAGGGACACGA CTGGAGATTAAACGA (SEQ ID NO: 185) | - |
| 61 | 5C9 | CAGGTGCAGCTGGTGCA GTCTGGAAGTGAGGTGA AGAAGCCTGGGGCCTCA GTGAAGGTCTCCTGCAA GGCCTCTGGATACACCTT TGGTCATTATGGTATTAG TTGGGTGCGCCAGGCCC CTGGACAAGGCCTTGAG TGGATGGCCTGGATCAG CGCTTACAATGGTAACA CAGACTCCATACAGAAG GTCCAGGGCAGAGTCAC CATGACCACAGACACAT CCACGAACACAGCCTAC TTGGAATTGAGGAGCCT GAGATCTGACGACACGG CCGTGTATTACTGTGTAA GAGATGTCCCGGCCACA GGAGGAGCTGCCACGGC TGACTACTGGGGCCAGG GAACCACGGTCACCGTC TCCTCA(SEQ ID NO: 186) | - | GATGTTGTGATGACTCAG TCTCCACTCTCCCTGCCCG TCACCCTTGGCCAGCCGG CCTCCATCTCCTGCAGGT CTAGTCAAAGCCTCGTCT TCAGTGATGGAAACACCT ACTTGAGTTGGTTTCAAC AGAGGCCAGGCCAATCTC CAAGGCGCCTAATTTATA AGGTTTCTAACCGGGACT CTGGGGTCCCAGACAGAT TCAGCGCCAGTGGGTCAG GCACTGATTTCACACTGA AAATCAGCAGGGTGGAG GCTGACGATGTTGGGGTT TATTACTGCATGCAAGTT ACACACTGGCCTCGGACT TTTGGCCAG GGGACCAAGCTGGAGATC AAACGA (SEQ ID NO: 187) | - |
| 62 | 5C7 | CAGGTACAGCTGCAGCA GTCTGGAAGTGAGGTGA AGAAGCCTGGGGCCTCA GTGAAGGTCTCCTGCAA GGCCTCTGGATACACCTT TGGTCATTATGGTATTAG TTGGGTGCGCCAGGCCC CTGGACAAGGCCTTGAG TGGATGGCCTGGATCAG CGCTTACAATGGTAACA | TGCTGTGGGTGA GTGGTACCTGTG GGCAGGTACAG CTGGTGCAGTCT GGAGCTGAGGT GAAGAAGCCTG GGGCCTCAGTG AAGGTCTCCTGC AAGGCCTCTGG ATACACCTTTGG | GATGTTGTGATGACACAG TCTCCACTCTCCTTACCTG TCCCCCTTGGACAGCCGG CCTCCATCTCCTGCAGGT CTAGTCAAAGCCTCGTAC ACAGTGATGGAAACACCT ACTTGAGTTGGTTTCAGC AGAGGCCAGGCCAATCTC CAAGGCGCCTAATTTATA AGGTTTCTGACCGGGACT | - |
| | | CAGACTCCATACAGAAG GTCCAGGGCAGAGTCAC CATGACCACAGACACAT CCACGAACACAGCCTAC TTGGAATTGAGGAGCCT GAGATCTGACGACACGG CCGTGTATTACTGTGTAA GAGATGTCCCGGCCACA GGAGGAGCTGCCACGGC TGACTACTGGGGCCAGG GAACCCTGGTCACCGTC TCCTCA(SEQ ID NO: 188) | TCATTATGGTAT TAGTTGGGTGCG CCAGGCCCCTG GACAAGGCCTT GAGTGGATGGC CTGGATCAGCG CTTACAATGGTA ACACAGACTCC ATACAGAAGGT CCAGGGCAGAG TCACCATGACCA CAGACACATCC ACGAACACAGC CTACTTGGAATT GAGGAGCCTGA GATCTGACGAC ACGGCCGTGTAT TACTGTGTAAGA GATGTCCCGGCC ACAGGAGGAGC TGCCACGGCTG ACTACTGGGGC CAGGGAACCCT GGTCACCGTCTC CTCAGCCTCCAC CAAGGGCCCAT CGGTCTTCCCCC (SEQ ID NO: 189) | CTGGGGTCCCAGACAGAT TCAGCGGCAGTGGGTCAG GCACTGATTTCACACTGA AAATCAGCAGGGTGGAG GCTGAGGATGTTGGGGTT TATTACTGCATGCAAGTT ACACACTGGCCTCGGACT TTTGGCCAAGGGACACGA CTGGAGATTAAACGA (SEQ ID NO: 190) | |
| 63 | 8A11 | CAGGTGCAGCTGGTGCA GTCTGGAAGTGAGGTGA AGAAGCCTGGGGCCTCA GTGAAGGTCTCCTGCAA GGCCTCTGGATACACCTT TGGTCATTATGGTATTAG TTGGGTGCGCCAGGCCC CTGGACAAGGCCTTGAG TGGATGGCCTGGATCAG CGCTTACAATGGTAACA CAGACTCCATACAGAAG GTCCAGGGCAGAGTCAC CATGACCACAGACACAT CCACGAACACAGCCTAC TTGGAATTGAGGAGCCT GAGATCTGACGACACGG CCGTGTATTACTGTGTAA GAGATGTCCCGGCCACA GGAGGAGCTGCCACGGC TGACTACTGGGGCCAGG GAACCCTGGTCACCGTC TCCTCA(SEQ ID NO: 191) | - | GATGTTGTGATGACTCAG TCTCCACTCTCCCTGCCCG TCACCCTTGGACAACCGG CCTCCATCTCCTGCAAGT CTAGTCGAAGCCTCGTAC ACAGTGATGGAAACACCT ACTTGAGTTGGTTTCAAC AGAGGCCAGGCCAATCTC CAAGGCGCCTAATTTATA AGGTTTCTAACCGGGACT CTGGGGTCCCAGACAGAT TCAGCGCCAGTGGGTCAG GCACTGATTTCACACTGA AAATCAGCAGGGTGGAG GCTGACGATGTTGGGGTT TATTACTGCATGCAAGTT ACACACTGGCCTCGGACT TTTGGCCAG GGGACCAAGCTGGAGATC AAACGA (SEQ ID NO: 192) | - |

**[Table 19] Amino acid sequences of heavy chain and light chain variable domains of 63 types of clones**

| | | Heavy chain | | Light chain | |
|---|---|---|---|---|---|
| # | Clone name | SEQ (amino acid) | Editing with SEQ (amino acid) | SEQ (amino acid) | Editing with SEQ (amino acid) |
| | | | Germline sequence (Modified) | | Germline sequence (Modified) |
| 1 | 2G4 | EVQLVESGGGLVK PGGSLRLSCAASGF TFSSYTMHWVRQA PGKGLEWVSSITG GSSYVDYSASVKG RFTISRDNAQSSLY LQMNSLRAEDTAV YYCARDDYGSGSY SNWFDPWGQGTL VTVSS (SEQ ID NO: 193) | - | QSVLTQPPSVSGAPG QRVTISCTGSSSNIGA GYDVHWYQQVPGT APKLLIFGSTNRPSG VPDRFSGSKSGTSAS LAITGLQADDEADY YCQSYDRSLSHVFG TGTKVTVLG (SEQ ID NO: 194) | - |
| 2 | 2H3 | EVQLVESGGGVVQ SGRSLRLSCAGSGF TFSSYTMHWVRQA PGKGLEWVSSITG GSSYVDYSASVKG RFTISRDNAQSSLY LQMNSLRAEDTAV YYCARDDYGSGSY SNWFDPWGQGTL VTVSS (SEQ ID NO: 195) | - | QSVLTQPPSVSGAPG QRVTISCTGSSSNIGA GYDVHWYQQVPGT APKLLIFGSTNRPSG VPDRFSGSKSGASAS LAITGLQTEDEADYY CQSYDRSLSHVFGT GTKVTVLG (SEQ ID NO: 196) | - |
| 3 | 4H1 | EVQLVESGGGLVK PGGSLRLSCAGSGF TFSSYTMHWVRQA PGKGLEWVSSITG GSSYVDYSASVKG RFTISRDNAQSSLY LQMNSLRAEDTAV YYCARDDYGSGSY SNWFDPWGQGTL VTVSS (SEQ ID NO: 197) | - | QSVLTQPPSVSGAPG QRVTISCTGSSSNIGA GYDVHWYQQVPGT APKLLIFGSTNRPSG VPDRFSGSKSGASAS LAITGLQADDEADY YCQPYDRSLSHVFG TGTKVTVLR (SEQ ID NO: 198) | - |
| 4 | 4E3 | EVQLVESGGGLVK PGGSLRLSCAGSGF TFSSYTMHWVRQA PGKGLEWVSSITG GSSYVDYSASVKG RFTISRDNAQSSLY LQMNSLRAEDTAV YYCARDDYGSGSY SNWFDPWGQGTL VTVSS(SEQ ID NO: 199) | - | QSVLTQPPSVSGAPG QRVTISCTGSSSNIGA GYDVHWYQQVPGT APKLLIFGSTNRPSG VPDRFSGSKSGASAS LAITGLQADDEADY YCQSYDRSLSHVFG TGTKVTVLG (SEQ ID NO: 200) | - |
| 5 | 4H5 | EVQLVESGGGLVK PGGSLRLSCAGSGF TFSSYTMHWVRQA PGKGLEWVSSITG GSSYVDYSASVKG RFTISRDNAQSSLY LQMNSLRAEDTAV YYCARDDYGSGSY SNWFDPWGQGTL VTVSS(SEQ ID NO: 201) | - | QSVLTQPPSVSGAPG QRVTISCTGSSSNIGA GYDVHWYQQLPGA APRLLMFGNSNRPS GVPDRFSGSKSGTSA SLAITGLQAEDEADY YCQSYDRSLSHVFG TGTKVTVLG (SEQ ID NO: 202) | - |
| 6 | 2E7 | EVQLVESGGGLVK PGGSLRLSCAGSGF TFSSYTMHWVRQA PGKGLEWVSSITG GSSYVDYSASVKG RFTISRDNAQSSLY LQMNSLRAEDTAV | - | SYELTQPPSASGTPG QRVTISCTGSSSNIGA GYDVHWYQQLPGA APRLLMFGNSNRPS GVPDRFSGSKSGTSA SLAITGLQADDEAD YYCQSYDRSLSHVF | QSVLTQPPSASG TPGQRVTISCTGS SSNIGAGYDVHW YQQLPGAAPRLL MFGNSNRPSGVP DRFSGSKSGTSA SLAITGLQADDE |
| | | YYCARDDYGSGSY SNWFDPWGQGTL VTVSS(SEQ ID NO: 29) | | GTGTKVTVLG (SEQ ID NO: 203) | ADYYCQSYDRSL SHVFGTGTKVTV LG (SEQ ID NO: 30) |
| 7 | 2H2 | QVQLVQSGGGLIQ PGGSLRLSCAGSGF TFSSYTMHWVRQA PGKGLEWVSSITG GSSYVDYSASVKG RFTISRDNAQSSLY LQMNSLRAEDTAV YYCARDDYGSGSY SNWFDPWGQGTL VTVSS(SEQ ID NO: 204) | - | QSVLTQPPSVSGAPG QRVTISCTGSSSNIGA GYDVHWYQQLPGT APKLLIFANTNRPSG VPDRFSGSKSGASAS LAITGLQADDEADY YCQSYDRSLSHVFG TGTKVTVLG (SEQ ID NO: 205) | - |
| 8 | 12A1 | EVQLVESGGGLIQP GGSLRLSCAGSGFT FSSYTMHWVRQAP GKGLEWVSSITGG SSYVDYSASVKGR FTISRDNAQSSLYL QMNSLRAEDTAVY YCARDDYGSGSYS NWFDPWGQGTLV T VSS(SEQ ID NO: 19) | - | QSVLTQPPSVSGAPG QRVTISCTGSSSDIGA GYDVHWYQQLPGT APKLLIFANTNRPSG VPDRFSGSKSGASAS LAITGLQADDEADY YCQSYDRSLSHVFG TGTKVTVLG (SEQ ID NO: 20) | - |
| 9 | 2H1 | QVTLKESGGGVVQ SGRSLRLSCAASGF TFSSYTMHWVRQA PGKGLEWVSSITG GSSYVDYSASVKG RFTISRDNAQSSLY LQMNSLRAEDTAV YYCARDDYGSGSY SNWFDPWGQGTL VTVSS (SEQ ID NO: 21) | EVQLVESGGGVV QSGRSLRLSCAA SGFTFSSYTMHW VRQAPGKGLEW VSSITGGSSYVD YSASVKGRFTISR DNAQSSLYLQM NSLRAEDTAVYY CARDDYGSGSYS NWFDPWGQGTL VTVSS (SEQ ID NO: 206) | QSVLTQPPSVSGAPG QRVTISCTGSSSNIGA GYDVHWYQQLPGR APKLLIYANTNRPSG VADRFSGSKSGASAS LAITGLQADDEADY YCQSYDRSLSHVFG TGTKVTVLG (SEQ ID NO: 22) | - |
| 10 | 4G4 | EVQLVESGGGLVK PGGSLRLSCAGSGF TFSSYTMHWVRQA PGKGLEWVSSITG GSSYVDYSASVKG RFTISRDNAQSSLY LQMNSLRAEDTAV YYCARDDYGSGSY SNWFDPWGQGTL VTVSS (SEQ ID NO: 207) | - | QSVLTQPPSVSGAPG QRVTISCTGNSSNLG AGYDVHWYQQLPG TAPKLLIYANTNRPS GVPDRFSGSKSGASA SLAITGLQTEDEADY YCQSYDRSLSHVFG TGTKVTVLG (SEQ ID NO: 208) | - |
| 11 | 12E9 | EVQLVESGGGLVK PGGSLRLSCAASGF TFTSYRMHWVRQ APGKGLEWVSSIT GGGNYIEYADSVK GRFTISRDNAQSSL YLQMNSLRAEDTA VYYCARDDYGSGS YSNWFDPWGQGT LVTVSS(SEQ ID NO: 31) | - | QTVVTQEPSVSGAP GQRVTISCTGSSSNIG AGYDVHWYQQLPG RAPKLLIFANTNRPS GVPDRFSGSKSGASA SLAITGLQADDEAD YYCQSYDRSLSHVF GTGTKVTVLG (SEQ ID NO: 209) | QSVLTQPPSVSG APGQRVTISCTGS SSNIGAGYDVHW YQQLPGRAPKLL IFANTNRPSGVPD RFSGSKSGASAS LAITGLQADDEA DYYCQSYDRSLS HVFGTGTKVTVL G (SEQ ID NO: 24) |
| 12 | 4F11 | EVQLVESGGGLVK PGGSLRLSCAGSGF AFSSYTMHWVRQ APGKGLEWVSSIT | - | QPVLTQPPSVSGAPG QRVTISCTGSSSNIGA GYDVHWYQQVPGT APKLLIFGSTNRPSG | QSVLTQPPSVSG APGQRVTISCTGS SSNIGAGYDVHW YOOVPGTAPKLL |
| | | GGSSYLDYAHSVK GRFTISRDNGQNSL FLQMNSLRTEDTA VYYCARDDYGSGS YSNYFDPWGQGTL VTVSS (SEQ ID NO: 32) | | VPDRFSGSKSGASAS LAITGLQTEDEADYY CQSYDRSLSHVFGT GTKVTVLG (SEQ ID NO: 210) | IFGSTNRPSGVPD RFSGSKSGASAS LAITGLQTEDEA DYYCQSYDRSLS HVFGTGTKVTVA R (SEQ ID NO: 33) |
| 13 | 10E7 | EVQLVESGGGLVK PGGSLRLSCAASGF TFTSYRMHWVRQ APGKGLEWVSSIT GGGNYIEYADSVK GRFTISRDNAKNSL DLQMNSLRAEDTA IYYCARDMYGLGS YYSPNYFDSWGQG TLVTVSS (SEQ ID NO: 23) | - | QSVLTQPPSVSGAPG QRVTISCTGSSSNIGA GYDVHWYQQLPGR APKLLIFANTNRPSG VPDRFSGSKSGASAS LAITGLQADDEADY YCQSYDRSLSHVFG TGTKVTVLG (SEQ ID NO: 24) | - |
| 14 | 2E9 | QVTLKESGGGLVK PGGSLRLSCAGSGF TFSSYTMHWVRQA PGKGLEWVSSITG GSSYVDYSASVKG RFTISRDNAKNSLF LQMNSLRAEDTAV YYCARDDYGSGSY SNWFDPWGQGTL VTVSS(SEQ ID NO: 211) | - | QSVLTQPPSVSGAPG QRVTISCTGSSSNIGA GYDVHWYQQLPGR APKLLIFANTNRPSG VPDRFSGSKSGASAS LAITGLQADDEADY YCQSYDRSLSHVFG TGTKVTVLG (SEQ ID NO: 212) | - |
| 15 | 12B11 | EVQLVESGGGVVQ PGRSLRLACAASG FTFNSYAMHWVR QAPGKGLEWVSSI TGGSSYVDYSASV KGRFTISRDNAQSS LYLQMNSLRAEDT AVYYCARDMYGL GSYYSPNYFDPWG QGTLVTVSS(SEQ ID NO: 213) | - | SYELTQPPSVSGAPG QRVTISCTGSSSNIGA GYDVHWYQQLPGR APKLLIFANTNRPSG VPDRFSGSKSGTSAS LAITGLQAEDEADY YCQSYDSSLSYVFGT GTKVTVLG (SEQ ID NO: 214) | QSVLTQPPSVSG APGQRVTISCTGS SSNIGAGYDVHW YQQLPGRAPKLL IFANTNRPSGVPD RFSGSKSGTSASL AITGLQAEDEAD YYCQSYDSSLSY VFGTGTKVTVLG (SEQ ID NO: 215) |
| 16 | 3H8 | EVQLVESGGGLVK PGGSLRLSCAGSGF TFSSYTMHWVRQA PGKGLEWVSSITG GSSYVDYSASVKG RFTISRDNAQSSLY LQMNSLRAEDTAV YYCARDDYGSGSY SNWFDPWGRGTT VTVSS(SEQ ID NO: 25) | - | QPVLTQPLSASGTPG QRVTISCTGSSSNIGA GYDVHWYQQLPGR APKLLIFANTNRPSG VPDRFSGSKSGTSAS LAITGLQAEDEADY YCQSYDRSLSHVFG TGTKVTVLG (SEQ ID NO: 26) | QSVLTQPLSASG TPGQRVTISCTGS SSNIGAGYDVHW YQQLPGRAPKLL IFANTNRPSGVPD RFSGSKSGTSASL AITGLQAEDEAD YYCQSYDRSLSH VFGTGTKVTVLG (SEQ ID NO: 216) |
| 17 | 4E1 | EVQLVESGGGLVQ PGGSLRLACAASG FTFSSYTMHWVRQ APGKGLEWVSSIT GGSSYVDYSASVK GRFTISRDNAQSSL YLQMNSLRAEDTA VYYCARDDYGSGS YSNWFDPWGQGT LVTVSS(SEQ ID NO: 217) | - | SYELTQPPSVSGAPG QRVTISCTGSSSNIGA GYDVHWYQQLPGR APKLLIFANTNRPSG VPDRFSGSKSGASAS LAITGLQAEDEADY YCQSYDRSLSHVFG TGTKVTVLG (SEQ ID NO: 218) | QSVLTQPPSVSG APGQRVTISCTGS SSNIGAGYDVHW YQQLPGRAPKLL IFANTNRPSGVPD RFSGSKSGASAS LAITGLQAEDEA DYYCQSYDRSLS HVFGTGTKVTVL G (SEQ ID NO: 219) |
| 18 | 2H9 | QVTLKESGGGLVK PGGSLRLSCAASGF | - | SYELTQPLSVSGAPG QRVTISCTGSSSNIGA | QSVLTQPLSVSG APGORVTISCTGS |
| | | TFTSYRMHWVRQ APGKGLEWVSSIT GGGNYIEYADSVK GRFTISRDNAKNSL DLQMNSLRAEDTA IYYCARDMYGLGS YYSPNYFDPWGQG TLVTVSS (SEQ ID NO: 220) | | GYDVHWYQQVPGT APKLLIFGSTNRPSG VPDRFSGSKSGASAS LAITGLQADDEADY YCQSYDRSLSHVFG TGTKVTVLG (SEQ ID NO: 221) | SSNIGAGYDVHW YQQVPGTAPKLL IFGSTNRPSGVPD RFSGSKSGASAS LAITGLQADDEA DYYCQSYDRSLS HVFGTGTKVTVL G (SEQ ID NO: 222) |
| 19 | 12C11 | EVQLVESGGGLVK PGGSLRLSCAASGF TFTSYRMHWVRQ APGKRLEWVSSIT GGGNYIEYADSVK GRFTISRDNAKNSL DLQMNSLRAEDTA IYYCARDMYGLGS YYSPNYFDPWGQG TLVTVSS(SEQ ID NO: 223) | - | QSVLTQPPSVSGAPG QRVTISCTGSSSNIGA GYDVHWYQQVPGT APKLLIFGSTNRPSG VPDRFSGSKSGASAS LAITGLQTEDEADYY CQSYDRSLSHVFGT GTKVTVLG (SEQ ID NO: 224) | - |
| 20 | 3E10 | EVQLVESGGGLVK PGGSLRLSCAASGF TFTSYRMHWVRQ APGKGLEWVSSIT GGGNYIEYADSVK GRFTISRDNAKNSL DLQMNSLRAEDTA IYYCARDMYGLGS YYSPNYFDPWGQG TLVTVSS (SEQ ID NO: 225) | - | QPVLTQPPSVSGAPG QRVTISCTGSSSNIGA GYDVHWYQQVPGT APKLLIFGSTNRPSG VPDRFSGSKSGASAS LAITGLQTEDEADYY CQSYDRSLSHVFGT GTKVTVLG (SEQ ID NO: 226) | QSVLTQPPSVSG APGQRVTISCTGS SSNIGAGYDVHW YQQVPGTAPKLL IFGSTNRPSGVPD RFSGSKSGASAS LAITGLQTEDEA DYYCQSYDRSLS HVFGTGTKVTVL G (SEQ ID NO: 227) |
| 21 | 2G1 | QVTLKESGGGLVK PGGSLRLSCAASGF TFTSYRMHWVRQ APGKGLEWVSSIT GGGNYIEYADSVK GRFTISRDNAKNSL DLQMNSLRAEDTA IYYCARDMYGLGS YYSPNYFDPWGQG TLVTVSS (SEQ ID NO: 228) | - | QSVLTQPPSVSGAPG QRVTISCTGSSSNIGA GYDVHWYQQLPGT APKLLIFGSTNRPSG VPDRFSGSKSGASAS LAITGLQTEDEADYY CQSYDRSLSHVFGT GTKVTVLG (SEQ ID NO: 229) | - |
| 22 | 1E6 | EVQLVESGGGLVK PGGSLRLSCAASGF TFTSYRMHWVRQ APGKGLEWVSSIT GGGNYIEYADSVK GRFTISRDNAKNSL DLQMNSLRAEDTA IYYCARDMYGLGS YYSPNYFDPWGQG TLVTVSS (SEQ ID NO: 230) | - | QSVLTQPPSVSGAPG QRVTISCTGSSSNIGA GYDVHWYQQLPGR APKLLIFANTNRPSG VPDRFSGSKSGASAS LAITGLQADDEADY YCQSYDRSLSHVFG TGTKVTVLG (SEQ ID NO: 231) | - |
| 23 | 2H11 | QVQLVQSGGGLVK PGGSLRLSCAASGF TFTSYRMHWVRQ APGKGLEWVSSIT GGGNYIEYADSVK GRFTISRDNAKNSL DLQMNSLRAEDTA IYYCARDMYGLGS YYSPNYFDPWGQG TTVTVSS(SEQ ID NO: 232) | - | QSVLTQPPSVSGAPG QRVTISCTGSSSNIGA GYDVHWYQQLPGR APKLLIFANTNRPSG VPDRFSGSKSGASAS LAISGLQAEDEAHY YCQSYDRSLNVVFG GGTELTVLG (SEQ ID NO: 233) | - |
| 24 | 2F1 | EVQLVESGGGLVK PGGSLRLSCAASGF TFTSYRMHWVRQ APGKGLEWVSSIT GGGNYIEYADSVK GRFTISRDNAKNSL DLQMNSLRAEDTA IYYCARDMYGLGS YYSPNYFDPWGQG TLVTVSS (SEQ ID NO: 234) | - | QSVLTQPPSVSGAPG QRVTISCTGSSSNIGA GYDVHWYQQVPGT APKLLIFGSTNRPSG VPDRFSGSKSGASAS LAITGLQTEDEADYY CQSYDRSLSAWVFG GGTKLTVLG (SEQ ID NO: 235) | - |
| 25 | 13C2 | EVQLVESGGGLVK PGGSLRLSCAASGF TFTSYRMHWVRQ APGKGLEWVSSIT GGGNYIEYADSVK GRFTISRDNAKNSL DLQMNSLRAEDTA IYYCARDMYGLGS YYSPNYFDPWGQG TLVTVSS (SEQ ID NO: 236) | - | QSVLTQPPSVSGAPG QRVTISCTGSSSNIGA GYDVHWYQQVPGT APKLLIFGSTDRPSG VPDRFSGSKSGNTAS LTISGLQAEDEADYY CQSYDRSLSHVFGT GTKVTVLG (SEQ ID NO: 237) | - |
| 26 | 2H8 | EVQLVESGGGLAK PGGSLRLSCAASGF TFTSYRMHWVRQ APGKGLEWVSSIT GGGNYIEYADSVK GRFTISRDNAKNSL DLQMNSLRAEDTA IYYCARDMYGLGS YYSPNYFDPWGQG TLVTVSS(SEQ ID NO: 238) | - | QSVLTQPPSVSGAPG QRVTISCTGSSSNIGA GYDVHWYQQLPGA APRLLMFGNSNRPS GVPDRFSGSKSGTSA SLAITGLQAEDEADY YCQSYDRSLSHVFG TGTKVTVLG (SEQ ID NO: 239) | - |
| 27 | 10A6 | EVQLVESGGGLVK PGGSLRLSCAASGF TFTSYRMHWVRQ APGKGLEWVSSIT GGGNYIEYADSVK GRFTISRDNAKNSL DLQMNSLRAEDTA IYYCARDMYGLGS YYSPNYFDSWGQG TLVTVSS(SEQ ID NO: 240) | - | SYELTQPPSVSGAPG QRVTISCTGSSSNIGA GYDVHWYQQLPGA APRLLMFGNSNRPS GVPDRFSGSKSGTSA SLAITGLQAEDEADY YCQSYDSSLSYVFGT GTKVTVLG (SEQ ID NO: 241) | QSVLTQPPSVSG APGQRVTISCTGS SSNIGAGYDVHW YQQLPGAAPRLL MFGNSNRPSGVP DRFSGSKSGTSA SLAITGLQAEDE ADYYCQSYDSSL SYVFGTGTKVTV LG (SEQ ID NO: 242) |
| 28 | 4G2 | EVQLVESGGGLVK PGGSLRLSCAASGF TFTSYRMHWVRQ APGKGLEWVSSIT GGGNYIEYADSVK GRFTISRDNAKNSL DLQMNSLRAEDTA IYYCARDMYGLGS YYSPNYFDPWGQG TLVTVSS(SEQ ID NO: 243) | - | QSVLTQPPSVSGAPG QRVTISCTGSSSNIGA GYDVHWYQQLPGA APRLLMFGNSNRPS GVPDRFSGSKSGTSA SLAITGLQAEDEADY YCQSYDSSLSHVFGT GTKVTVLG (SEQ ID NO: 244) | - |
| 29 | 2G11 | EVQLVESGGGLVQ PGGSLRLSCAASGF TFTSYRMHWVRQ APGKGLEWVSSIT GGGNYIEYADSVK GRFTISRDNAKNSL DLQMNSLRAEDTA IYYCARDMYGLGS YYSPNYFDPWGQG | - | QSVLTQPPSVSGAPG QRVTISCTGSSSNIGA GYDVHWYQQLPGA APRLLMFGNSNRPS GVPDRFSGSKSGTSA SLAITGLQAEDEADY YCQSYDSSLSYVFGT GTKVTVLG (SEQ ID NO: 246) | - |
| | | TLVTVSS(SEQ ID NO: 245) | | | |
| 30 | 11H9 | EVQLVESGGGLVQ PGGSLRLSCAASEF TFSMNWVRQAPG KGLEWVSSVRGGG TETYYADSVKGRF TVSRDNSKNTLHL QMNSLRAEDTALY YCAGGPIVEPNIDY FNSWGQGTLVTVS S (SEQ ID NO: 247) | - | QSVLTQPPSVSGAPG QRVTISCTGSSSNIGA GYDVHWYQQLPGR APKLLIFANTNRPSG VPDRFSGSKSGASAS LAITGLQADDEADY YCQSYDSKHHVVFG TGTKLTVLG (SEQ ID NO: 248) | QSVLTQPPSVSG APGQRVTISCTGS SSNIGAGYDVHW YQQLPGRAPKLL IFANTNRPSGVPD RFSGSKSGASAS LAITGLQADDEA DYYCQSYDSKH HVVFGTGTKLTV LG (SEQ ID NO: 249) |
| 31 | 12C9 | EVQLVESGGGLVQ PGGSLRLSCAASGF SFRSYWMSWLRQ APGKGLQWVANIK PDGSVESYVDSVE GRFTISRDNAKNSL FLQMNSLSAEDMA VYYCARTDDGSS WFVSTSSFYGMDV WGQGTTVTVSS (SEQ ID NO: 250) | - | QSVLTQPPSVSGAPG QRVTISCTGSSSNIGA GYDVHWYQQVPGT APKLLIFGSTNRPSG VPDRFSGSKSGASAS LAIIGLQTEDEADYY CQSYDRSLSHVFGT GTQLTDLG (SEQ ID NO: 251) | - |
| 32 | 7A10 | EVQLVESGGGLVK PGGSLRLSCAASGF TFSSYTMHWVRQA PGKGLEWVSSITG GSSYVDYSASVKG RFTISRDNSKNTLY LQMSSLRTDDTAV YFCAKDQRGDSYD SVMNWYFDLWGR GTTVTVSS(SEQ ID NO: 252) | - | QSVLTQPPSVSGAPG QRVTISCTGNSSNLG AGYDVHWYQQVPG TAPKLLIFGSTNRPS GVPDRFSGSKSGASA SLTISGLQAEDEADY YCCSYAASSSRVFGT GTKVTVLG (SEQ ID NO: 253) | - |
| 33 | 4G1 | QVTLKESGGGLVK PGGSLRLSCAASGF RLSSYGMNWVRQ APGKGLEWVSSIS ASSSFINYADSVRD RFTISRDNAKNSLY LQMNSLRAEDTAV YYCARDDYGSGSY SNWFDPWGQGTL VTVSS (SEQ ID NO: 27) | EVQLVESGGGLV KPGGSLRLSCAA SGFRLSSYGMN WVRQAPGKGLE WVSSISASSSFIN YADSVRDRFTIS RDNAKNSLYLQ MNSLRAEDTAV YYCARDDYGSG SYSNWFDPWGQ GTLVTVSS (SEQ ID NO: 254) | QSVLTQPPSVSGAPG QRVTISCTGNSSNLG AGYDVHWYQQLPG RAPKLLIFANTNRPS GVPDRFSGSKSGASA SLAITSLQADDEADY YCQSYDRSLSHVFG TGTKVTVLG (SEQ ID NO: 28) | - |
| 34 | 7C6 | EVQLVESGGGLVK PGGSLRLSCAASGF TFSHYGISWVRQA PGQGLEWMAWIS AYNGNTDSIQKVQ GRVTMTTDTSTNT AYLELRSLRSDDT AVYYCVRDVPATG GAATADYWGQGT LVTVSS (SEQ ID NO: 255) | QVQLVQSGAEV KKPGASVKVSCK ASGFTFSHYGIS WVRQAPGQGLE WMAWISAYNGN TDSIQKVQGRVT MTTDTSTNTAYL ELRSLRSDDTAV YYCVRDVPATG GAATADYWGQG TLVTVSS (SEQ ID NO: 256) | QSVLTQPPSVSGAPG QRVTISCTGSSSNIGA GYDVHWYQQVPGT APKLLIFGSTNRPSG VPDRFSGSKSGASAS LAITGLQAEDEADY YCSSYTSSSTFVVFG GGTQLIILG (SEQ ID NO: 257) | - |
| 35 | 7A7 | QVQLVESGGGVVQ PGRSLRLSCAASGF TFNSYAMHWVRQ APGNGLDWVAGIS YDGGNKYYADSV | - | QSALTQPASVSGSPG QSITISCTGTSSDVGG YNYVSWFQQYPGK APKLMIYDVSKRPSG VSNRFSGSKSGNTAS | - |
| | | KDRFTISRDNSKNT LYLQMSSLRTDDT AVYYCAKDAGWE AWWYFDLWGRGT LVTVSS (SEO ID NO: 258) | | LTISELQAEDEADYY CSSYTSSSTGVFGGG TKLTVLG (SEQ ID NO: 259) | |
| 36 | 6A9 | EVQLVESGGGLVQ PGGSLRLSCAASGT FTKYAMTWVRQA LGKGLEWVSTIGS GSDTHYADSVKGR FTISRDNSRNTLSL QMNSLRAEDTAVY YCAKYLGITVGDT GFRTFDYWGQGTT VTVSS (SEQ ID NO: 260) | - | QSALTQPRSVSGSPG QSVTISCTGTSSDVG SYNLVSWYQQHPGK APKLMIYDVSKRPSG VSNRFSGSKSGNTAS LTISGLQAEDEADYY CSSYAGSGNVVFGG GTQLIILG (SEQ ID NO: 261) | - |
| 37 | 12B8 | QVQLVESGGGVVQ PGRSLRLSCAASGF TFSIYAMHWVRQA PGKGLEWVAVVS YDGSEKYYADSVQ GRFTISRDKSKNTL YLQMNSLTAEDTA VYYCAREPWVGTI GYWGQGTLVTVSS (SEQ ID NO: 262) | - | QSALTQPPSASGSPG QSVTISCTGTSSDVG GYNYVSWYRQHPG KAPKLLIYEVNNRPS GVPSRFSGSKSGNTA SLTISGLQAEDEADY YCSSYTSSSTFVVLG GGTQLIILG (SEQ ID NO: 263) | - |
| 38 | 12F7 | QMQLVQSGPEVKK PGSSVKVSCKASG YTFTDYGLSWVRQ APGHGLEWMGWI TAYNGDTNYAQKF QDRLSVTTDISTST AYMELRSLKSDDT AVYYCARHVFCSG DGCYSGLGSWGQ GTLVTVSS (SEQ ID NO: 264) | QVQLVQSGAEV KKPGASVKVSCK ASGYTFTDYGLS WVRQAPGHGLE WMGWITAYNGD TNYAQKFQDRLS VTTDISTSTAYM ELRSLKSDDTAV YYCARHVFCSGD GCYSGLGSWGQ GTLVTVSS (SEQ ID NO: 265) | QSVLTQPPSASGTPG QRVTISCSGSSSNVG SNTVNWYQQLPGTA PKLLIYDKNERPSGV PDRFSASKSGTSASL AISGLQSEDEADYYC AAWDDSLNGWVFG GGTELTVLG (SEQ ID NO: 266) | QSVLTQPPSASG APGQRVIISCSGS SSNVGSNTVNW YQQLPGTAPKLL IYDKNERPSGVP DRFSASKSGTSA SLAISGLQSEDEA DYYCAAWDDSL NGWVFGGGTKL TVLG (SEQ ID NO: 267) |
| 39 | 7A11 | EVQLVESGGGLVK PGGSLRLSCAASGF RLSSYGMNWVRQ APGKGLEWVSSIS ASSSFINYADSVRG RFTISRDKSKNTLY LQMNSLTAEDTAV YYCAREPWVGTID YWGQGTLVTVSS (SEO ID NO: 268) | - | QSALTQPASVSGSPG QSITISCTGTSSDVGS YNLVSWYQQHPGK APKLLIYEDTKRPSGI PDRFSGSSSGNTASL TITGAQAEDEAEYY CSSRDSSGNHLVFGG GTKLTVLG (SEQ ID NO: 269) | - |
| 40 | 4H11 | QVQLVQSGAEVKK PGASVKVSCKASG YTFTDYGLSWVRQ APGHGLEWMGWI TAYNGDTNYAQKF QDRLSVTTDISTST AYMELRSLKSDDT AVYYCARHVFCSG DGCYSGLGSWGQ GTLVTVSS (SEQ ID NO: 270) | - | SYELTQPPSASGAPG QRVIISCSGSSSNVGS NTVNWYQQLPGRAP KLLIFANTNRPSGVP DRFSGSKSGTSASLA ISGLQSEDEADYYCA AWDDSLNGWVFGG GTKLTVLG (SEQ ID NO: 271) | QSVLTQPPSASG APGQRVIISCSGS SSNVGSNTVNW YQQLPGRAPKLL IFANTNRPSGVPD RFSGSKSGTSASL AISGLQSEDEAD YYCAAWDDSLN GWVFGGGTKLT VLG (SEQ ID NO: 272) |
| 41 | 2G3 | QVQLVQSGAEVKK PGASVKVSCKASG YTFTDYGLSWVRQ APGHGLEWMGWI | - | SYELTQPLSASGTPG QRVTISCSGSSSNIGS GTVNWYQQLSGTAP KLLMHSDNQRPSGV | QSVLTQPLSASG TPGQRVTISCSGS SSNIGSGTVNWY QQLSGTAPKLLM |
| | | TAYNGDTNYAQKF QDRLSVTTDISTST AYMELRSLKSDDT AVYYCARHVFCSG DGCYSGLGSWGQ GTLVTVSS (SEQ ID NO: 273) | | PDRFSGSKSGTSASL AISGLQSEDEADYYC AAWDDSLNGWVFG GGTKLTVLG (SEQ ID NO: 274) | HSDNQRPSGVPD RFSGSKSGTSASL AISGLQSEDEAD YYCAAWDDSLN GWVFGGGTKLT VLG (SEQ ID NO: 275) |
| 42 | 12C10 | QVQLVQSGSEVKK PGASVKVSCKASG YTFTDYGLSWVRQ APGHGLEWMGWI TAYNGDTNYAQKF QDRLSVTTDISTST AYMELRSLKSDDT AVYYCARHVFCSG DGCYSGLGSWGQ GTLVTVSS (SEQ ID NO: 276) | - | QPVLTQPPSVSGTPG QRVTISCSGSSSNIGS DTVNWYQQLSGTAP KLLMHSDNQRPSGV PDRFSGSKSGTSASL AISGLQSEDEADYYC AAWDDSLNGWVFG GGTKLTVLG (SEQ ID NO: 277) | QSVLTQPPSVSG TPGQRVTISCSGS SSNIGSDTVNWY QQLSGTAPKLLM HSDNQRPSGVPD RFSGSKSGTSASL AISGLQSEDEAD YYCAAWDDSLN GWVFGGGTKLT VLG (SEQ ID NO: 278) |
| 43 | 3E8 | QVQLVQSGSEVKK PGASVKVSCKASG YTFTDYGLSWVRQ APGHGLEWMGWI TAYNGDTNYAQKF QDRLSVTTDISTST AYMELRSLKSDDT AVYYCARHVFCSG DGCYSGLGSWGQ GTLVTVSS (SEQ ID NO: 279) | QVQLVQSGSEVK KPGASVKVSCKA SGYTFTDYGLSW VRQAPGHGLEW MGWITAYNGDT NYAQKFQDRLSV TTDISTSTAYMEL RSLKSDDTAVYY CARHVFCSGDGC YSGLGSWGQGT LVTVSS (SEQ ID NO: 280) | QPVLTQPPSASGTPG QRVTISCSGSSSNIGS DTVNWYQQLSGTAP KLLMHSDNQRPSGV PDRFSGSKSGTSASL AISGLQSEDEADYYC AAWDDSLNGWVFG GGTKLTVLG (SEQ ID NO: 281) | QSVLTQPPSASG TPGQRVTISCSGS SSNIGSDTVNWY QQLSGTAPKLLM HSDNQRPSGVPD RFSGSKSGTSASL AISGLQSEDEAD YYCAAWDDSLN GWVFGGGTKLT VLG (SEQ ID NO: 282) |
| 44 | 7D1 | QVQLVQSGAEVKK PGASVKVSCKASG YTFTDYGLSWVRQ APGHGLEWMGWI TAYNGDTNYAQKF QDRLSVTTDISTST AYMELRSLKSDDT AVYYCARHVFCSG DGCYSGLGSWGQ GTLVTVSS (SEQ ID NO: 283) | - | QSVLTQPPSASGTPG QRVTISCSGSSSNIGS NTVNWYQQLPGTAP KLLIYSNNQRPSGVP DRFSGSTSGTSASLAI SGLQSEDEADYYCA AWDDSLNGWVFGG GTKLTVLG (SEQ ID NO: 284) | - |
| 45 | 4G5 | QVQLVQSGSEVKK PGASVKVSCKASG YTFTDYGLSWVRQ APGHGLEWMGWI TAYNGDTNYAQKF QDRLSVTTDISTST AYMELRSLKSDDT AVYYCARHVFCSG DGCYSGLGSWGQ GTLVTVSS(SEQ ID NO: 285) | - | SYELTQPLSASGTPG QRVTISCSGSSSNIGS NTVNWYQQLPGTAP KLLIYSNNQRPSGVP DRFSGSKSGTSASLA ISGLQSEDEADYYCA AWDDSLNGWVFGG GTKLTVLG (SEQ ID NO: 286) | QSVLTQPLSASG TPGQRVTISCSGS SSNIGSNTVNWY QQLPGTAPKLLI YSNNQRPSGVPD RFSGSKSGTSASL AISGLQSEDEAD YYCAAWDDSLN GWVFGGGTKLT VLG (SEQ ID NO: 287) |
| 46 | 2E8 | QVQLVQSGPEVKK PGSSVKVSCKASG YTFTDYGLSWVRQ APGHGLEWMGWI TAYNGDTNYAQKF QDRLSVTTDISTST AYMELRSLKSDDT AVYYCARHVFCSG DGCYSGLGSWGQ GTLVTVSS(SEQ ID NO: 288) | - | SYELTQPPSASGAPG QRVIISCSGSSSNVGS NTVNWYQQLPGTAP KLLIYDKNERPSGVP DRFSASKSGTSASLA ISGLQSEDEADYYCA AWDDSLNGWVFGG GTKLTVLG (SEQ ID NO: 289) | QSVLTQPPSASG APGQRVIISCSGS SSNVGSNTVNW YQQLPGTAPKLL IYDKNERPSGVP DRFSASKSGTSA SLAISGLQSEDEA DYYCAAWDDSL NGWVFGGGTKL TVLG (SEQ ID NO: 290) |
| 47 | 13H9 | QVQLVQSGAEVKK | - | SYELIQPPSASGAPG | QSVLTQPPSASG |
| | | PGASVKVSCKASG YTGYTFTDYGLSW VRQAPGHGLEWM GWITAYNGDTNYA QKFQDRLSVTTDIS TSTAYMELRSLKS DDTAVYYCARHVF CSGDGCYSGLGSW GQGTLVTVSS(SEQ ID NO: 291) | | QRVIISCSGSSSNVGS NTVNWYQQLPGTAP KLLIYDKNERPSGVP DRFSASKSGTSASLA ISGLQSEDEADYYCA AWDDSLNGWVFGG GTELTVLG (SEQ ID NO: 292) | APGQRVIISCSGS SSNVGSNTVNW YQQLPGTAPKLL IYDKNERPSGVP DRFSASKSGTSA SLAISGLQSEDEA DYYCAAWDDSL NGWVFGGGTKL TVLG (SEQ ID NO: 293) |
| 48 | 4E9 | QVQLVQSGAEVKK PGSSVKVSCKASG YTFTDYGLSWVRQ APGHGLEWMGWI TAYNGDTNYAQKF QDRLSVTTDISTST AYMELRSLKSDDT AVYYCARHVFCSG DGCYSGLGSWGQ GTLVTVSS(SEQ ID NO: 294) | - | QSVLTQPPSVSGAPG QRVTISCSGSSSNVG SNTVNWYQQLPGTA PKLLIYDKNERPSGV PDRFSASKSGTSSSL AIGGLRSEDEADYY CGTWDDNLNGWVF GGGTKLTVLG (SEQ ID NO: 295) | - |
| 49 | 4H7 | QVQLVQSGAEVKK PGASVKVSCKASG YTFTDYGLSWVRQ APGHGLEWMGWI TAYNGDTNYAQKF QDRLSVTTDISTST AYMELRSLKSDDT AVYYCARHVFCSG DGCYSGLGSWGQ GTLVTVSS(SEQ ID NO: 296) | - | SYELTQPPSASGAPG QRVIISCSGSSSNVGS NTVNWYQQLPGTAP KLLIYDKNERPSGVP DRFSASKSGTSSSLAI GGLRSEDEADYYCA TWDDNLNGWVFGG GTKLTVLG (SEQ ID NO: 297) | QSVLTQPPSASG APGQRVIISCSGS SSNVGSNTVNW YQQLPGTAPKLL IYDKNERPSGVP DRFSASKSGTSSS LAIGGLRSEDEA DYYCATWDDNL NGWVFGGGTKL TVLG (SEQ ID NO: 298) |
| 50 | 2F8 | QVQLVQSGAEVKK PGASVKVSCKASG YTFTDYGLSWVRQ APGHGLEWMGWI TAYNGDTNYAQKF QDRLSVTTDISTST AYMELRSLKSDDT AVYYCARHVFCSG DGCYSGLGSWGQ GTLVTVSS(SEQ ID NO: 299) | - | SYELTQPPSASGAPG QRVIISCSGSSSNVGS NTVNWYQQLPGTAP KLLIYDKNERPSGVP DRFSASKSGTSSSLAI GGLRSEDEADYYCA TWDDNLNGWVFGG GTKLTVLG (SEQ ID NO: 300) | QSVLTQPPSASG APGQRVIISCSGS SSNVGSNTVNW YQQLPGTAPKLL IYDKNERPSGVP DRFSASKSGTSSS LAIGGLRSEDEA DYYCGTWDDNL NGWVFGGGTKL TVLG (SEQ ID NO: 301) |
| 51 | 3E7 | EVQLVESGTEVKK PGASVKVSCKASG YTFTDYGLSWVRQ APGHGLEWMGWI TAYNGDTNYAQKF QDRLSVTTDISTST AYMELRSLKSDDT AVYYCARHVFCSG DGCYSGLGSWGQ GTLVTVSS (SEQ ID NO: 302) | EVQLVQSGAEVK KPGASVKVSCKA SGYTFTDYGLSW VRQAPGHGLEW MGWITAYNGDT NYAQKFQDRLSV TTDISTSTAYMEL RSLKSDDTAVYY CARHVFCSGDGC YSGLGSWGQGT LVTVSS (SEQ ID NO: 303) | QSVLTQPPSASGAPG QRVIISCSGSSSNVGS NTVNWYQQLPGTAP KLLIYDKNERPSGVP DRFSASKSGTSSSLAI GGLRSEDEADYYCG TWDDNLNGWVFGG GTKLTVLG (SEQ ID NO: 304) | - |
| 52 | 9H4 | QVQLVQSGGGLVQ PGGSLRISCTASGF TFRNYEMNWVRQ APGKGLEWVAYIS SSGSSRYYADSVK GRFTISRDNAKNSL FLQMNSLRAEDMA VYYCARTDDGSS WFVSTSSFYGMDV | EQVQLVESGGGL VQPGGSLRISCTA SGFTFRNYEMN WVRQAPGKGLE WVAYISSSGSSR YYADSVKGRFTI SRDNAKNSLFLQ MNSLRAEDMAV YYCARTDDGSS | EIVLTQSPGIQSLSPG ETATLSCRASESISSS YFAWYQQKPGQAPR LLIYGASSRASGIPDR FSGSGSGTDFTLTISR LEPEDFAVYYCQQY GSSPPITFGQGTRLEI KR (SEQ ID NO: 307) | - |
| | | WGQGTTVTVSS (SEQ ID NO: 305) | WFVSTSSFYGMD VWGQGTTVTVS S (SEQ ID NO: 306) | | |
| 53 | 8B1 | QVQLVQSGGGLVQ PGGSLRISCTASGF TFRNYEMNWVRQ APGKGLEWVAYIS SSGSSRYYADSVK GRFTISRDNAKNTL YLQMSSLRSDDTA VYYCARTDDGSS WFVSTSSFYGMDV WGQGTTVTVSS(SE Q ID NO: 308) | EQVQLVESGGGL VQPGGSLRISCTA SGFTFRNYEMN WVRQAPGKGLE WVAYISSSGSSR YYADSVKGRFTI SRDNAKNTLYLQ MSSLRSDDTAVY YCARTDDGSSWF VSTSSFYGMDV WGQGTTVTVSS (SEQ ID NO: 309) | DVVMTQSPGTLSLSP GERATLSCRASQSVS SSYLAWYQQKPGQA PRLLIYGVSSRATGIP DRFSGSGSGTDFTLT ISRLEPEDFAVYYCQ QYGSSPPITFGQGTK LEIKR (SEQ ID NO: 310) | EIVLTQSPGTLSL SPGERATLSCRA SQSVSSSYLAWY QQKPGQAPRLLI YGVSSRATGIPD RFSGSGSGTDFTL TISRLEPEDFAVY YCQQYGSSPPITF GQGTKLEIKR (SEQ ID NO: 311) |
| 54 | 7G12 | EVQLVESGGGLVQ PGGSLRLSCAASEF TFSMNWVRQAPG KGLEWVSSVRGGG TETYYADSVKGRF TVSRDNSKNTLYL QMNSLRVEDTAVY YCTVCVVMFRGW CNWFDPWGQGTL VTVSS (SEQ ID NO: 312) | - | DIQLTQSPSSVSASV GERVTITRRASQNID RWLAWYQQKPGKA PDLLIFAASSLQSGV PSRFSGSGSGTDFTL TISSLQPEDFATYYC QQGKTFPPTFGQGT KLEIKR (SEQ ID NO: 313) | - |
| 55 | 12F11 | EVQLLESGGGLVQ PGGSLRLSCAASEF TFSMNWVRQAPG KGLEWVSSVRGGG TETYYADSVKGRF TVSRDNSKNTLYL QMNSLRVEDTAVY YCTVCVVMFRGW CNWFDPWGQGTL VTVSS(SEQ ID NO: 314) | - | DVVMTQSPSSLSASV GDRVTITCRASQSLS SYLNWYQQKPGKAP KLLIYATTNLQSGVP SRFSGSGSGTHFTLTI GSLQPEDFATYYCQ QSFQTPLTFGGGTKV EIKR (SEQ ID NO: 315) | DIQMTQSPSSLSA SVGDRVTITCRA SQSLSSYLNWYQ QKPGKAPKLLIY ATTNLQSGVPSR FSGSGSGTHFTLT IGSLQPEDFATYY CQQSFQTPLTFG GGTKLEIKR (SEQ ID NO: 316) |
| 56 | SB12 | QVQLVESGGGVVQ PGRSLRLSCAASGF TFISYVMHWVRQA PGKGLEWVAVVS YDGSEKYYADSVK GRFTISRDNSKNTL YLQMNSLTAEDTA VYYCAREPWVGTI DYWGQGTLVTVSS (SEQ ID NO: 317) | - | DIQMTQSPATLSASV GDRVTITCRASQSIN NNLNWYQQKPGQA PKLLIYAASTLQGGV PSRFSGSGSGTEFTLT ISSLQPEDFATYYCQ QLNGYPLTFGGGTK VEIKR (SEQ ID NO: 318) | - |
| 57 | 8C3 | EVQLLESGGGLVQ PGGSLRLSCAASGF TFSDYAMSWVRQ APGKGLEWVSSIT ANGLMTYYTDSV KGRFTISRDNSKNT LYLQMSSLRTDDT AVYYCAKDAGWE AWWYFDLWGRGT LVTVSS(SEQ ID NO: 319) | - | DIQMTQSPDSLAVSL GERATINCKSSQSVL YSSNNKNYLAWYQ QKPGQPPKMLIYWA SIRETGVPDRFSGSG SETDFTLTISSLQAED VAVYYCQQYYSTLT FGGGTKVEIKR (SEQ ID NO: 320) | - |
| 58 | 2F4 | QVQLVQSGAEVKK PGASVKVSCKASG YTFTSYYMRWLRQ APGQGLEWMGIID PSGGSTTYAQKFQ | - | EIVLTQSPATVSLSPG ERTMLSCRASQNVS NYLGWYQQRRGQPP RLLISDASNRASGVP ARFSGSGSGTDFTLT | - |
| | | GRVSITRDTSTSTV YMELSSLRSEDTA VYYCARGRGVVP AGNPSTGGVGMD VWGQGTTVTVSS( SEQ ID NO: 321) | | ITSLQPEDFAVYFCQ HRSSWPVTFGGGTK VEIKR (SEQ ID NO: 322) | |
| 59 | 1C10 | QVQLVQSGSEVKK PGASVKVSCKASG YTFGHYGISWVRQ APGQGLEWMAWI SAYNGNTDSIQKV QGRVTMTTDTSTN TAYLELRSLRSDDT AVYYCVRDVPATG GAATADYWGQGT LVTVSS (SEQ ID NO: 323) | - | DVVMTQSPLSLPVTL GQPASISCRSSQSLVF SDGNTYLSWFQQRP GQSPRRLIYKVSNRD SGVPDRFSASGSGTD FTLKISRVEADDVGV YYCMQVTHWPRTF GQGTKLEIKR (SEQ ID NO: 324) | - |
| 60 | 5E9 | QVQLVQSGPEVKK PGSSVKVSCKASG YTFGHYGISWVRQ APGQGLEWMAWI SAYNGNTDSIQKV QGRVTMTTDTSTN TAYLELRSLRSDDT AVYYCVRDVPATG GAATADYWGQGT TVTVSS(SEQ ID NO: 325) | - | DVVMTQSPLSLPVTL GQPASISCRSSQSLVF SDGNTYLSWFQQRP GQSPRRLIYKVSNRD SGVPDRFSASGSGTD FTLKISRVEADDVGV YYCMQVTHWPRTF GQGTRLEIKR (SEQ ID NO: 326) | - |
| 61 | 5C9 | QVQLVQSGSEVKK PGASVKVSCKASG YTFGHYGISWVRQ APGQGLEWMAWI SAYNGNTDSIQKV QGRVTMTTDTSTN TAYLELRSLRSDDT AVYYCVRDVPATG GAATADYWGQGT TVTVSS(SEQ ID NO: 327) | - | DVVMTQSPLSLPVTL GQPASISCRSSQSLVF SDGNTYLSWFQQRP GQSPRRLIYKVSNRD SGVPDRFSASGSGTD FTLKISRVEADDVGV YYCMQVTHWPRTF GQGTKLEIKR (SEQ ID NO: 328) | - |
| 62 | 5C7 | QVQLQQSGSEVKK PGASVKVSCKASG YTFGHYGISWVRQ APGQGLEWMAWI SAYNGNTDSIQKV QGRVTMTTDTSTN TAYLELRSLRSDDT AVYYCVRDVPATG GAATADYWGQGT LVTVSS (SEQ ID NO: 329) | QVQLVQSGAEV KKPGASVKVSCK ASGYTFGHYGIS WVRQAPGQGLE WMAWISAYNGN TDSIQKVQGRVT MTTDTSTNTAYL ELRSLRSDDTAV YYCVRDVPATG GAATADYWGQG TLVTVSS (SEQ ID NO: 330) | DVVMTQSPLSLPVPL GQPASISCRSSQSLV HSDGNTYLSWFQQR PGQSPRRLIYKVSDR DSGVPDRFSGSGSGT DFTLKISRVEAEDVG VYYCMQVTHWPRT FGQGTRLEIKR (SEQ ID NO: 331) | - |
| 63 | 8A11 | QVQLVQSGSEVKK PGASVKVSCKASG YTFGHYGISWVRQ APGQGLEWMAWI SAYNGNTDSIQKV QGRVTMTTDTSTN TAYLELRSLRSDDT AVYYCVRDVPATG GAATADYWGQGT LVTVSS (SEQ ID NO: 332) | - | DVVMTQSPLSLPVTL GQPASISCKSSRSLV HSDGNTYLSWFQQR PGQSPRRLIYKVSNR DSGVPDRFSASGSGT DFTLKISRVEADDVG VYYCMQVTHWPRT FGQGTKLEIKR (SEQ ID NO: 333) | - |

### 4. Result of screening anti-RSV neutralizing antibodies

The neutralizing activity against RSV virus for 127 IgG supernatant samples (1^{st}: 88, 2^{nd}: 39) was confirmed by a FRNT test method. As a result, a total of 75 samples (1^{st}: 43, 2^{nd}: 32) exhibiting 50% or more neutralizing activity at a 1:10 dilution were confirmed. Although there was a difference in neutralizing activity according to 1:100 and 1:1000 dilutions, it is considered to be a result that can appear due to the difference in the expression level and antibody concentration of each sample.

**[Table 20]**

| Samples with 50% or more neutralizing activity according to dilution | | |
|---|---|---|
| Dilution | Neutralizing activity > 50% | |
| | 1^{st} (88 samples) | 2^{nd} (39 samples) |
| 1:10 | 43 | 32 |
| 1:100 | 36 | 31 |
| 1:1000 | 6 | 26 |

### Example 5. Analysis result for selected RSV neutralizing antibodies

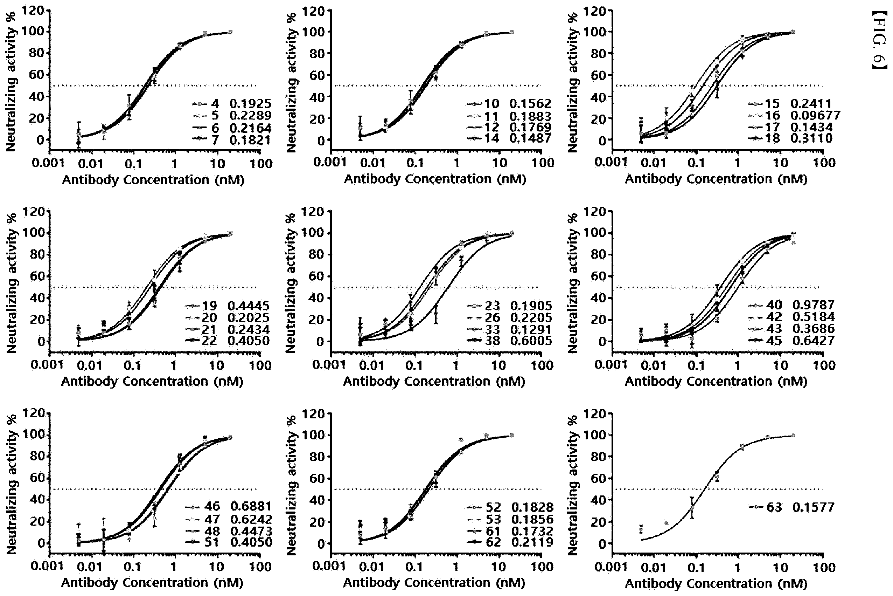

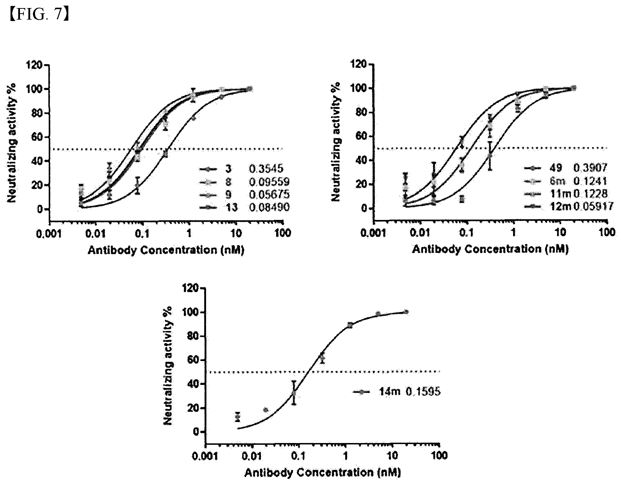

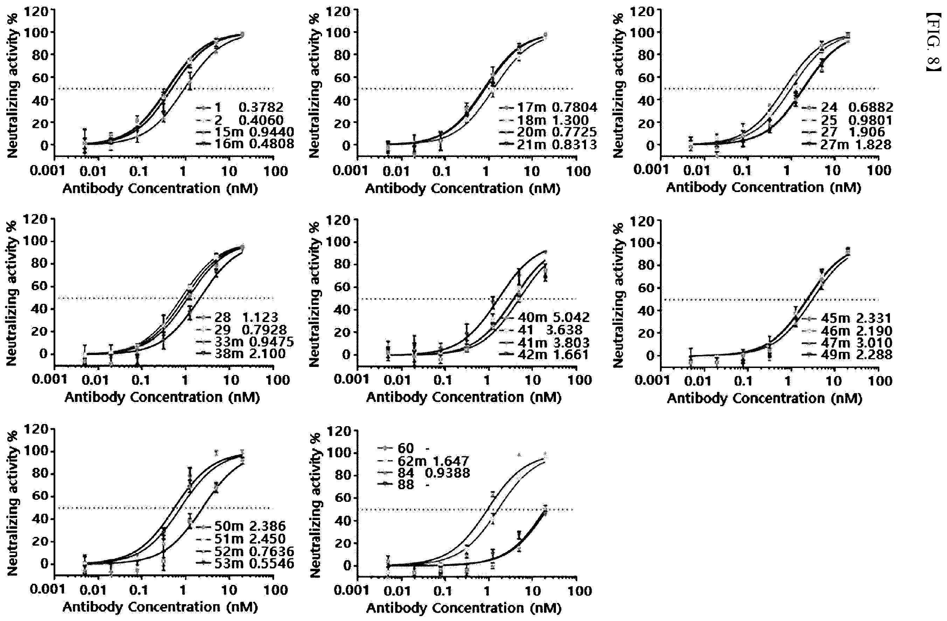

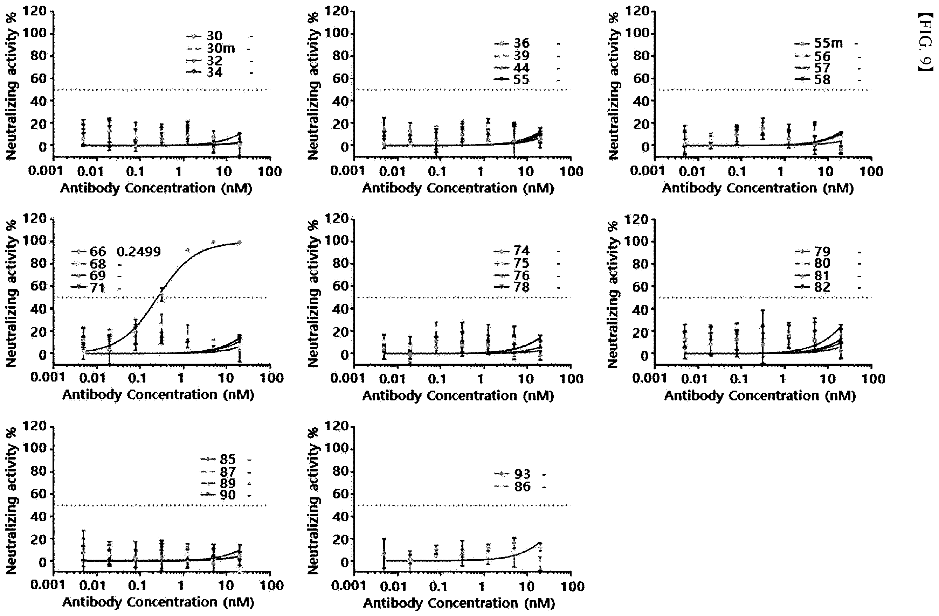

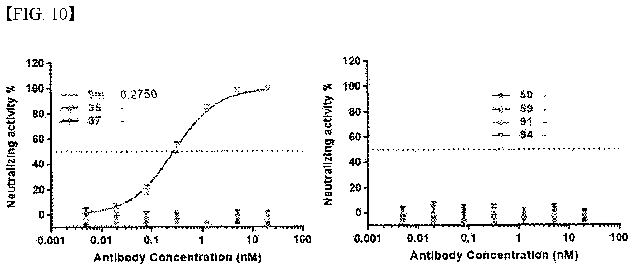

The result of the neutralizing activity test for the selected RSV antibodies are shown in FIGS. 6 to 10, and the IC₅₀ and range (confidence interval: 95%) of each antibody are shown in Table 21 below.

**[Table 21]**

| Sample Name | IC₅₀ (pM) | Range (95% CI) | | IC50 (ng/mL) |
|---|---|---|---|---|
| 1 | 378.20 | 334.4 - 427.8 | | 49.17 |
| 2 | 406.00 | 331.1 - 497.8 | | 52.78 |
| 3 | 354.50 | 304.8 - 412.3 | | 46.09 |
| 4 | 192.50 | 158.6 - 233.8 | | 25.03 |
| 5 | 228.90 | 199.8 - 262.1 | | 29.76 |
| 6 | 216.40 | 193.4 - 242.1 | | 28.13 |
| 7 | 182.10 | 167.5 - 198.0 | | 23.67 |
| **8** | **95.59** | **76.34 - 119.7** | | **12.43** |
| **9** | **56.75** | **47.29 - 68.11** | | **7.38** |
| 10 | 156.20 | 125.7 - 194.1 | | 20.31 |
| 11 | 188.30 | 162.1 - 218.9 | | 24.48 |
| 12 | 176.90 | 151.8 - 206.1 | | 23.00 |
| **13** | **84.90** | **66.47 - 108.5** | | **11.04** |
| 14 | 148.70 | 130.7 - 169.3 | | 19.33 |
| 15 | 241.10 | 210.4 - 276.2 | | 31.34 |
| **16** | **96.77** | **80.97 - 115.7** | | **12.58** |
| 17 | 143.40 | 110.0 - 187.1 | | 18.64 |
| 18 | 311.00 | 251.4 - 384.7 | | 40.43 |
| 19 | 444.50 | 371.7 - 531.5 | | 57.79 |
| 20 | 202.50 | 168.8 - 242.8 | | 26.33 |
| 21 | 243.40 | 202.5 - 292.4 | | 31.64 |
| 22 | 405.00 | 323.0 - 507.8 | | 52.65 |
| 23 | 190.50 | 153.9 - 235.9 | | 24.77 |
| 24 | 688.20 | 565.6 - 837.3 | | 89.47 |
| 24 | 980.10 | 828.3 - 1160 | | 127.41 |
| 26 | 220.50 | 185.4 - 262.2 | | 28.67 |
| 27 | 1906.00 | 1606 - 2263 | | 247.78 |
| 28 | 1123.00 | 901.8 - 1398 | | 145.99 |
| 29 | 792.80 | 693.0 - 906.9 | | 103.06 |
| 30 | ND | ND | | ND |
| 32 | ND | ND | | ND |
| **33** | **129.10** | **104.0 - 160.4** | | **16.78** |
| 34 | ND | ND | | ND |
| 35 | ND | ND | | ND |
| 36 | ND | ND | | ND |
| 37 | ND | ND | | ND |
| 38 | 600.50 | 471.9 - 764.2 | | 78.07 |
| 39 | ND | ND | | ND |
| 40 | 978.70 | 791.1 - 1211 | | 127.23 |
| 41 | 3638.00 | 2935 -4510 | | 472.94 |
| 42 | 518.40 | 416.9 - 644.6 | | 67.39 |
| 43 | 368.60 | 310.9 - 437.1 | | 47.92 |
| 44 | ND | ND | | ND |
| 45 | 642.70 | 573.6 - 720.2 | | 83.55 |
| 46 | 688.10 | 537.0 - 881.7 | | 89.45 |
| 47 | 624.20 | 478.8 - 813.8 | | 81.15 |
| 48 | 447.30 | 363.3 - 550.8 | | 58.15 |
| 49 | 390.70 | 311.2 - 490.5 | | 50.79 |
| 50 | ND | ND | | ND |
| 51 | 405.00 | 340.2 - 482.0 | | 52.65 |
| 52 | 182.80 | 150.6 - 221.8 | | 23.76 |
| 53 | 185.60 | 146.2 - 235.6 | | 24.13 |
| 55 | ND | ND | | ND |
| 56 | ND | ND | | ND |
| 57 | ND | ND | | ND |
| 58 | ND | ND | | ND |
| 59 | ND | ND | | ND |
| 60 | ND | ND | | ND |
| 61 | 173.20 | 145.3 - 206.5 | | 22.52 |
| 62 | 211.90 | 166.6 - 269.6 | | 27.55 |
| 63 | 157.70 | 126.2 - 197.1 | | 20.50 |
| 66 | 249.9 | 184.8 - 338.1 | | 32.49 |
| 68 | ND | ND | | ND |
| 69 | ND | ND | | ND |
| 71 | ND | ND | | ND |
| 74 | ND | ND | | ND |
| 75 | ND | ND | | ND |
| 76 | ND | ND | | ND |
| 78 | ND | ND | | ND |
| 79 | ND | ND | | ND |
| 80 | ND | ND | | ND |
| 81 | ND | ND | | ND |
| 82 | ND | ND | | ND |
| 84 | 938.80 | 602.6 - 1463 | | 122.04 |
| 85 | ND | ND | | ND |
| 86 | ND | ND | | ND |
| 87 | ND | ND | | ND |
| 88 | ND | ND | | ND |
| 89 | ND | ND | | ND |
| 90 | ND | ND | | ND |
| 91 | ND | ND | | ND |
| 93 | ND | ND | | ND |
| 94 | ND | ND | | ND |
| **11m** | **122.80** | **92.80 - 162.5** | | **15.96** |
| **12m** | **59.17** | **44.05 - 79.49** | | **7.69** |
| 14m | 159.50 | 128.1 - 198.7 | | 20.74 |
| 15m | 944.00 | 816.0 - 1092 | | 122.72 |
| 16m | 480.80 | 405.9 - 569.6 | | 62.50 |
| 17m | 780.40 | 613.2 - 993.2 | | 101.45 |
| 18m | 1300.00 | 1094 - 1543 | | 169.00 |
| 20m | 772.50 | 654.7 - 911.4 | | 100.43 |
| 21m | 831.30 | 707.5 - 976.9 | | 108.07 |
| 27m | 1828.00 | 1550 - 2156 | | 237.64 |
| 30m | ND | ND | | ND |
| 33m | 947.50 | 794.5 - 1130 | | 123.18 |
| 38m | 2100.00 | 1661 - 2655 | | 273.00 |
| 40m | 5042.00 | 4253 - 5978 | | 655.46 |
| 41m | 3803.00 | 2766 - 5228 | | 494.39 |
| 42m | 1661.00 | 1341 - 2059 | | 215.93 |
| 45m | 2331.00 | 1878 - 2892 | | 303.03 |
| 46m | 2190.00 | 1834 - 2615 | | 284.70 |
| 47m | 3010.00 | 2348 - 3857 | | 391.30 |
| 49m | 2288.00 | 1748 - 2996 | | 297.44 |
| 50m | 2386.00 | 1813 - 3140 | | 310.18 |
| 51m | 2450.00 | 1965 - 3054 | | 318.50 |
| 52m | 763.60 | 533.2 - 1094 | | 99.27 |
| 53m | 554.60 | 424.1 - 725.3 | | 72.10 |
| 55m | ND | ND | | ND |
| 5C4 | 91.42 | 76.90 - 108.7 | | 11.88 |
| 62m | 1647.00 | 1359 - 1995 | | 214.11 |
| **6m** | **124.10** | **95.44 - 161.4** | | **16.13** |
| 9m | 275 | 235.2 - 321.6 | | 35.75 |

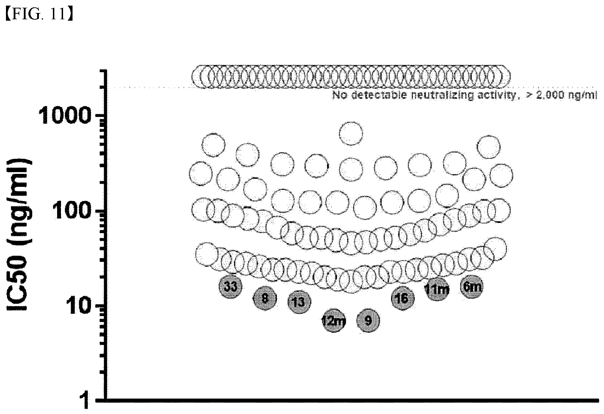

The IC₅₀ values of an RSV A2 neutralizing antibody for 112 RSV screening antibody samples were confirmed by an FRNT test. As a result, 8 clones (8, 9, 13, 16, 33, 6m, 11m, and 12m) were confirmed as antibodies exhibiting excellent RSV neutralizing activity (FIG. 11).

**[Table 22]**

| IC₅₀ of RSV neutralizing antibody against selected antibodies | | | | |
|---|---|---|---|---|
| Clone | IC₅₀ (ng/mL) | Range | Name | Library |
| 8 | 12.43 | 9.92 - 15.56 | 12A1 | Chromium |
| 9 | 7.38 | 6.15 - 8.85 | 2H1 | Chromium |
| 13 | 11.04 | 8.64 - 14.11 | 10E7 | Manual |
| 16 | 12.58 | 10.53 - 15.04 | 3H8 | Manual |
| 33 | 16.78 | 13.52 - 20.85 | 4G1 | Chromium |
| 6m | 16.13 | 12.41 - 20.98 | 2E7 | Chromium |
| 11m | 15.96 | 12.06 - 23.13 | 12E9 | Chromium |
| 12m | 7.69 | 5.73 - 10.33 | 4F11 | Chromium |

### Example 6. Quantitative measurement result for binding ability of F-protein

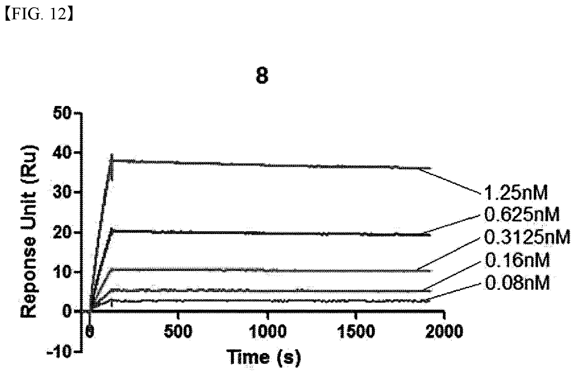

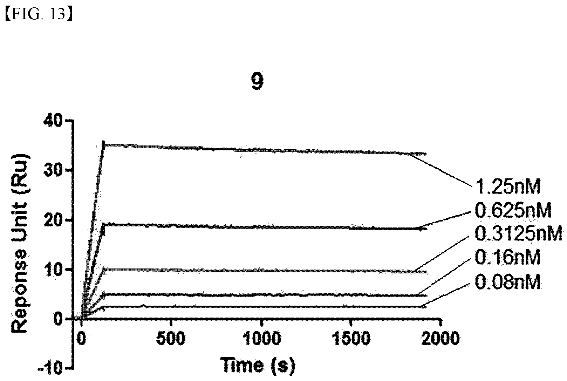

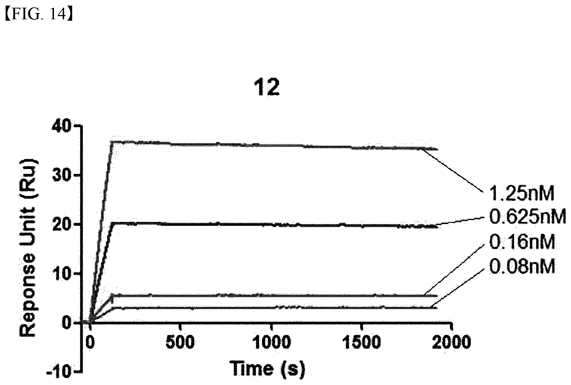

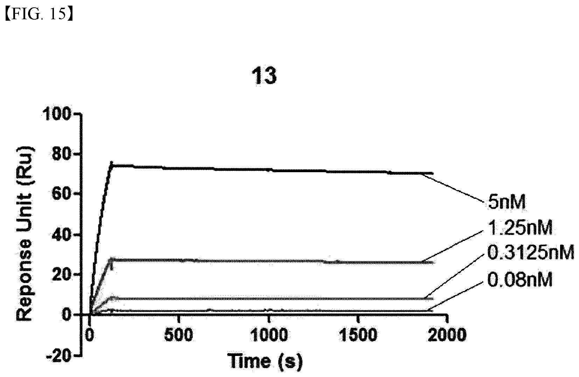

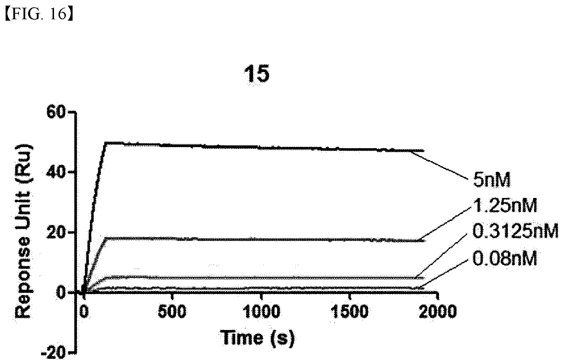

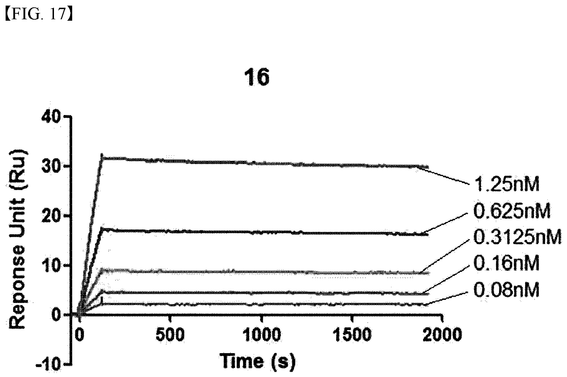

Table 23 below shows the affinity measurement results for the anti-F antibodies selected from the results of the neutralizing antibody analysis as the kinetic rate constants (Kₒₙ and K_{off}) and the equilibrium dissociation constant (K_{D}) (FIGS. 12 to 17).

**[Table 23]**

| Equilibrium dissociation constant of anti-F antibody | | | |
|---|---|---|---|
| Clone | Kₒₙ | K_{off} | K_{D} |
| 8 | 2.80X10⁶ | 3.18X10⁻⁵ | 1.14X10⁻¹¹ |
| 9 | 1.50X10⁷ | 5.74X10⁻⁵ | 3.84X10⁻¹² |
| 12m | 3.06X10⁶ | 2.07X10⁻⁵ | 6.77X10⁻¹² |
| 13 | 2.03X10⁶ | 2.81X10⁻⁵ | 1.38X10⁻¹¹ |
| 15 | 2.52X10⁶ | 2.89X10⁻⁵ | 1.15X10⁻¹¹ |
| 16 | 1.53X10⁷ | 6.06X10⁻⁵ | 3.97X10⁻¹² |
| 33 | 1.77X10⁶ | 6.02X10⁻⁵ | 3.41X10⁻¹¹ |

### Example 7. Confirmation of sequences of finally-selected 8 types of anti-RSV antibodies

As described above, 8 types of antibodies having an excellent effect were finally selected by measuring IC₅₀ values, and the sequence information on heavy chains CDR1 to CDR3 and light chains CDR1 to CDR3, and heavy chain variable domains and light chain variable domains of the antibodies are shown in Tables 24 to 26.

**[Table 24]**

| to CDR3 Amino for acid sequences 8 types of antibodies of heavy chain CDR1 to CDR3 and light chain CDR1 | | | | | | | |
|---|---|---|---|---|---|---|---|
| Clone | Variab le doma in | CDR1 | SE Q ID NO: | CDR2 | SE Q ID NO: | CDR3 | SE Q ID NO: |
| 8 | Heavy chain chain | SSYTMH | 1 | SITGGSSYVDYSASVK G | 2 | DDYGSGSYSNWF | 3 |
| | Light chain | SSSDIGAGYDV H | 4 | ANTNRPS | 5 | QSYDRSLS | 6 |
| 9 | Heavy chain | SSYTMH | 1 | SITGGSSYVDYSASVK G | 2 | DDYGSGSYSNWF | 3 |
| | Light chain | SSSNIGAGYDV H | 7 | ANTNRPS | 5 | QSYDRSLS | 6 |
| 13 | Heavy chain | TSYRMH | 8 | SITGGGNYIEYADSVK G | 9 | DMYGLGSYYSPNY F | 10 |
| | Light chain | SSSNIGAGYDV H | 7 | ANTNRPS | 5 | QSYDRSLS | 6 |
| 16 | Heavy chain | SSYTMH | 1 | SITGGSSYVDYSASVK G | 2 | DDYGSGSYSNWF | 3 |
| | Light chain | SSSNIGAGYDV H | 7 | ANTNRPS | 5 | QSYDRSLS | 6 |
| 33 | Heavy chain | SSYGMN | 11 | SISASSSFINYADSVRD | 12 | DDYGSGSYSNWF | 3 |
| | Light chain | SSNLGAGYDV H | 13 | ANTNRPS | 5 | QSYDRSLS | 6 |
| 6m | Heavy chain | SSYTMH | 1 | SITGGSSYVDYSASVK G | 2 | DDYGSGSYSNWF | 3 |
| | Light chain | SSNIGAGYDVH | 14 | GNSNRPS | 15 | QSYDRSLS | 6 |
| 11m | Heavy chain | TSYRMH | 8 | SITGGGNYIEYADSVK G | 9 | DDYGSGSYSNWF | 3 |
| | Light chain | SSNIGAGYDVH | 14 | ANTNRPS | 5 | QSYDRSLS | 6 |
| 12m | Heavy chain | SSYTMH | 1 | SITGGSSYLDYAHSVK G | 16 | DDYGSGSYSNYF | 17 |
| | Light chain | SSNIGAGYDVH | 14 | GSTNRPS | 18 | QSYDRSLS | 6 |

**[Table 25]**

| variable Amino acid domain for 8 sequences of heavy chain variable domain and light types of antibodies (each CDR region is underlined) chain | | | |
|---|---|---|---|
| Clone | Variable domain | Amino acid sequence | SEQ ID NO: |
| 8 | Heavy chain | EVQLVESGGGLIQPGGSLRLSCAGSGFTFSSYTMHWVRQA PGKGLEWVSSITGGSSYVDYSASVKGRFTISRDNAQSSLY LQMNSLRAEDTAVYYCARDDYGSGSYSNWFDPWGQGTL VTVSS | 19 |
| | Light chain | QSVLTQPPSVSGAPGQRVTISCTGSSSDIGAGYDVHWYQQ LPGTAPKLLIFANTNRPSGVPDRFSGSKSGASASLAITGLQ ADDEADYYCQSYDRSLSHVFGTGTKVTVLG | 20 |
| 9 | Heavy chain | QVTLKESGGGVVQSGRSLRLSCAASGFTFSSYTMHWVRQ APGKGLEWVSSITGGSSYVDYSASVKGRFTISRDNAQSSL YLQMNSLRAEDTAVYYCARDDYGSGSYSNWFDPWGQGT LVTVSS | 21 |
| | Light chain | QSVLTQPPSVSGAPGQRVTISCTGSSSNIGAGYDVHWYQQ LPGRAPKLLIYANTNRPSGVADRFSGSKSGASASLAITGLQ ADDEADYYCQSYDRSLSHVFGTGTKVTVLG | 22 |
| 13 | Heavy chain | EVQLVESGGGLVKPGGSLRLSCAASGFTFTSYRMHWVRQ APGKGLEWVSSITGGGNYIEYADSVKGRFTISRDNAKNSL DLQMNSLRAEDTAIYYCARDMYGLGSYYSPNYFDSWGQ | 23 |
| | | GTLVTVSS | |
| | Light chain | QSVLTQPPSVSGAPGQRVTISCTGSSSNIGAGYDVHWYQQ LPGRAPKLLIFANTNRPSGVPDRFSGSKSGASASLAITGLQ ADDEADYYCQSYDRSLSHVFGTGTKVTVLG | 24 |
| 16 | Heavy chain | EVQLVESGGGLVKPGGSLRLSCAGSGFTFSSYTMHWVRQ APGKGLEWVSSITGGSSYVDYSASVKGRFTISRDNAQSSL YLQMNSLRAEDTAVYYCARDDYGSGSYSNWFDPWGRGT TVTVSS | 25 |
| | Light chain | QPVLTQPLSASGTPGQRVTISCTGSSSNIGAGYDVHWYQQ LPGRAPKLLIFANTNRPSGVPDRFSGSKSGTSASLAITGLQ AEDEADYYCQSYDRSLSHVFGTGTKVTVLG | 26 |
| 33 | Heavy chain | QVTLKESGGGLVKPGGSLRLSCAASGFRLSSYGMNWVRQ APGKGLEWVSSISASSSFINYADSVRDRFTISRDNAKNSLY LQMNSLRAEDTAVYYCARDDYGSGSYSNWFDPWGQGTL VTVSS | 27 |
| | Light chain | QSVLTQPPSVSGAPGQRVTISCTGNSSNLGAGYDVHWYQ QLPGRAPKLLIFANTNRPSGVPDRFSGSKSGASASLAITSL QADDEADYYCQSYDRSLSHVFGTGTKVTVLG | 28 |
| 6m | Heavy chain | EVQLVESGGGLVKPGGSLRLSCAGSGFTFSSYTMHWVRQ APGKGLEWVSSITGGSSYVDYSASVKGRFTISRDNAQSSL YLQMNSLRAEDTAVYYCARDDYGSGSYSNWFDPWGQGT LVTVSS | 29 |
| | Light chain | QSVLTQPPSASGTPGQRVTISCTGSSSNIGAGYDVHWYQQ LPGAAPRLLMFGNSNRPSGVPDRFSGSKSGTSASLAITGLQ ADDEADYYCQSYDRSLSHVFGTGTKVTVLG | 30 |
| 11m | Heavy chain | EVQLVESGGGLVKPGGSLRLSCAASGFTFTSYRMHWVRQ APGKGLEWVSSITGGGNYIEYADSVKGRFTISRDNAQSSL YLQMNSLRAEDTAVYYCARDDYGSGSYSNWFDPWGQGT LVTVSS | 31 |
| | Light chain | QSVLTQPPSVSGAPGQRVTISCTGSSSNIGAGYDVHWYQQ LPGRAPKLLIFANTNRPSGVPDRFSGSKSGASASLAITGLQ ADDEADYYCQSYDRSLSHVFGTGTKVTVLG | 24 |
| 12m | Heavy chain | EVQLVESGGGLVKPGGSLRLSCAGSGFAFSSYTMHWVRQ APGKGLEWVSSITGGSSYLDYAHSVKGRFTISRDNGQNSL FLQMNSLRTEDTAVYYCARDDYGSGSYSNYFDPWGQGT LVTVSS | 32 |
| | Light chain | QSVLTQPPSVSGAPGQRVTISCTGSSSNIGAGYDVHWYQQ VPGTAPKLLIFGSTNRPSGVPDRFSGSKSGASASLAITGLQ TEDEADYYCQSYDRSLSHVFGTGTKVTVLG | 33 |

**[Table 26]**

| variable Polynucleotide domain for 8 sequences of heavy chain variable domain and light types of antibodies chain | | | |
|---|---|---|---|
| Clone | Variable domain | DNA sequence | SEQ ID NO: |
| 8 | Heavy chain | GAGGTGCAGCTGGTGGAGTCTGGGGGAGGCCTGATACA GCCTGGGGGGTCCCTGAGACTCTCCTGTGCAGGCTCTG GATTCACCTTCAGTAGCTATACCATGCACTGGGTCCGCC AGGCTCCAGGGAAGGGGCTGGAGTGGGTCTCGTCCATA ACTGGTGGCAGTAGTTATGTCGACTACTCAGCCTCAGT GAAGGGCCGATTCACCATCTCCAGAGACAACGCCCAGA | 34 |
| | | GCTCACTTTATCTGCAAATGAACAGCCTGAGAGCCGAG GACACGGCCGTGTATTACTGTGCGAGAGATGATTATGG TTCGGGGAGTTATTCCAACTGGTTCGACCCCTGGGGCC AGGGAACCCTGGTCACCGTCTCCTCA | |
| | Light chain | CAGTCTGTCCTGACGCAGCCGCCCTCAGTGTCTGGGGC CCCAGGGCAGAGGGTCACCATCTCCTGCACTGGGAGCA GCTCCGACATCGGGGCAGGTTATGATGTACACTGGTAC CAGCAACTTCCAGGAACAGCCCCCAAACTCCTCATCTT TGCTAACACCAATCGGCCCTCAGGGGTCCCTGATCGAT TCTCTGGCTCCAAGTCTGGCGCCTCTGCCTCCCTGGCCA TCACTGGCCTCCAGGCTGACGATGAGGCTGATTATTAC TGCCAGTCCTATGACCGCAGCCTGAGTCATGTCTTCGG AACTGGGACCAAGGTCACCGTCCTAGGC | 35 |
| 9 | Heavy chain | CAGGTCACCTTGAAGGAGTCTGGGGGAGGCGTGGTCCA GTCTGGGAGGTCCCTGAGACTCTCCTGTGCAGCCTCTG GATTCACCTTCAGTAGCTATACCATGCACTGGGTCCGCC AGGCTCCAGGGAAGGGGCTGGAGTGGGTCTCGTCCATA ACTGGTGGCAGTAGTTATGTCGACTACTCAGCCTCAGT GAAGGGCCGATTCACCATCTCCAGAGACAACGCCCAGA GCTCACTTTATCTGCAAATGAACAGCCTGAGAGCCGAG GACACGGCTGTGTATTACTGTGCGAGAGATGATTATGG TTCGGGGAGTTATTCCAACTGGTTCGACCCCTGGGGCC AGGGAACCCTGGTCACCGTCTCCTCA | 36 |
| | Light chain | CAGTCTGTGCTGACGCAGCCGCCCTCAGTGTCTGGGGC CCCAGGGCAGAGGGTCACCATCTCCTGCACTGGGAGCA GCTCCAACATCGGGGCAGGTTATGATGTACACTGGTAC CAGCAGCTTCCAGGAAGAGCCCCCAAACTCCTCATCTA TGCTAACACCAATCGGCCCTCAGGGGTCGCTGACCGAT TCTCTGGCTCCAAGTCTGGCGCCTCTGCCTCCCTGGCCA TCACTGGCCTCCAGGCTGACGATGAGGCTGATTATTAC TGCCAGTCCTATGACCGCAGCCTGAGTCATGTCTTCGG AACTGGGACCAAGGTCACCGTCCTAGGC | 37 |
| 13 | Heavy chain | GAGGTGCAGCTGGTGGAGTCTGGGGGAGGCCTGGTCAA GCCTGGGGGGTCCCTGAGACTCTCCTGTGCAGCCTCTG GATTCACCTTCACTAGTTATAGGATGCATTGGGTCCGCC AGGCTCCAGGGAAGGGGCTGGAGTGGGTCTCATCAATT ACTGGTGGTGGTAATTATATAGAGTACGCAGACTCAGT GAAGGGCCGGTTCACCATCTCCAGAGACAACGCCAAGA ACTCACTGGATCTGCAAATGAACAGCCTGAGAGCCGAG GACACGGCTATTTATTACTGTGCGAGAGATATGTATGG TTTGGGGAGTTATTATTCGCCTAACTACTTCGACTCCTG GGGCCAGGGAACCCTGGTCACCGTCTCCTCA | 38 |
| | Light chain | CAGTCTGTGCTGACGCAGCCGCCCTCAGTGTCTGGGGC CCCAGGGCAGAGGGTCACCATCTCCTGCACTGGGAGCA GCTCCAACATCGGGGCAGGTTATGATGTACACTGGTAC CAGCAGCTTCCAGGAAGAGCCCCCAAACTCCTCATCTT TGCTAACACCAATCGGCCCTCAGGGGTCCCTGACCGAT TCTCTGGCTCCAAGTCTGGCGCCTCTGCCTCCCTGGCCA TCACTGGCCTCCAGGCTGACGATGAGGCTGATTATTAC TGCCAGTCCTATGACCGCAGCCTGAGTCATGTCTTCGG AACTGGGACCAAGGTCACCGTCCTAGGC | 39 |
| 16 | Heavy chain | GAGGTGCAGCTGGTGGAGTCTGGGGGAGGCCTGGTCAA GCCTGGGGGGTCCCTGAGACTCTCCTGTGCAGCCTCTG GATTCACCTTCAGTAGCTATACCATGCACTGGGTCCGCC AGGCTCCAGGGAAGGGGCTGGAGTGGGTCTCGTCCATA ACTGGTGGCAGTAGTTATGTCGACTACTCAGCCTCAGT GAAGGGCCGATTCACCATCTCCAGAGATAACGCCCAGA | 40 |
| | | GCTCACTTTATCTGCAAATGAACAGCCTGAGAGCCGAG GACACGGCTGTGTATTACTGTGCGAGAGATGATTATGG TTCGGGGAGTTATTCCAACTGGTTCGACCCCTGGGGCC GGGGAACCACGGTCACCGTCTCCTCA | |
| | Light chain | CAGCCTGTGCTGACTCAGCCACTCTCAGCGTCTGGGAC CCCCGGGCAGAGGGTCACCATCTCCTGCACTGGGAGCA GCTCCAACATCGGGGCAGGTTATGATGTACACTGGTAC CAGCAGCTTCCAGGAAGAGCCCCCAAACTCCTCATCTT TGCTAACACCAATCGGCCCTCAGGGGTCCCTGACCGAT TCTCTGGCTCCAAGTCGGGCACCTCAGCCTCCCTGGCCA TCACTGGGCTCCAGGCTGAGGATGAGGCTGATTATTAC TGCCAGTCCTATGACCGCAGCCTGAGTCATGTCTTCGG AACTGGGACCAAGGTCACCGTCCTAGGC | 41 |
| 33 | Heavy chain | CAGGTCACCTTGAAGGAGTCTGGGGGCGGCCTGGTTAA GCCTGGGGGGTCCCTGAGACTCTCGTGTGCAGCCTCTG GGTTCAGGCTCAGTAGCTATGGCATGAACTGGGTCCGC CAGGCTCCAGGGAAGGGGCTGGAGTGGGTCTCATCCAT TTCTGCTAGTAGTAGTTTTATAAACTATGCAGACTCAGT GAGGGACCGATTCACCATCTCCAGAGACAACGCCAAGA ACTCACTGTATCTGCAAATGAACAGCCTGAGAGCCGAG GACACGGCTGTGTATTACTGTGCGAGAGATGATTATGG TTCGGGGAGTTATTCCAACTGGTTCGACCCCTGGGGCC AGGGAACCCTGGTCACCGTCTCCTCA | 42 |
| | Light chain | CAGTCTGTGCTGACGCAGCCGCCCTCAGTGTCTGGGGC CCCAGGGCAGAGGGTCACCATTTCCTGCACTGGGAACA GCTCCAACCTCGGGGCAGGTTATGATGTACACTGGTAC CAGCAGCTTCCAGGAAGAGCCCCCAAACTCCTCATCTT TGCTAACACCAATCGGCCCTCAGGGGTCCCTGACCGAT TCTCTGGCTCCAAGTCTGGCGCCTCTGCCTCCCTGGCCA TCACTAGCCTCCAGGCTGACGATGAGGCTGATTATTAC TGCCAGTCCTATGACCGCAGCCTGAGTCATGTCTTCGG AACTGGGACCAAGGTCACCGTCCTAGGC | 43 |
| 6m | Heavy chain | GAGGTGCAGCTGGTGGAGTCTGGGGGAGGCCTGGTCAA GCCTGGGGGGTCCCTGAGACTCTCCTGTGCAGCCTCTG GATTCACCTTCAGTAGCTATACCATGCACTGGGTCCGCC AGGCTCCAGGGAAGGGGCTGGAGTGGGTCTCGTCCATA ACTGGTGGCAGTAGTTATGTCGACTACTCAGCCTCAGT GAAGGGCCGATTCACCATCTCCAGAGACAACGCCCAGA GCTCACTTTATCTGCAAATGAACAGCCTGAGAGCCGAG GACACGGCTGTATATTACTGTGCGAGAGATGATTATGG TTCGGGGAGTTATTCCAACTGGTTCGACCCCTGGGGCC AGGGAACCCTGGTCACCGTCTCCTCA | 44 |
| | Light chain | CAGTCTGTGCTGACGCAGCCGCCCTCAGCGTCTGGGAC CCCCGGGCAGAGGGTCACCATCTCCTGCACTGGGAGCA GCTCCAACATCGGGGCAGGTTATGATGTACACTGGTAT CAGCAGCTTCCGGGAGCAGCCCCCAGACTCCTCATGTT TGGTAACAGCAATCGGCCCTCGGGGGTACCTGACCGCT TCTCTGGCTCCAAGTCTGGCACCTCCGCCTCCCTGGCCA TCACTGGCCTCCAGGCTGACGATGAGGCTGATTATTAC TGCCAGTCCTATGACCGCAGCCTGAGTCATGTCTTCGG AACTGGGACCAAGGTCACCGTCCTAGGC | 45 |
| 11m | Heavy chain | GAGGTGCAGCTGGTGGAGTCTGGGGGAGGCCTGGTCAA GCCTGGGGGGTCCCTGAGACTCTCCTGTGCAGCCTCTG GATTCACCTTCACTAGTTATAGGATGCATTGGGTCCGCC AGGCTCCAGGGAAGGGGCTGGAGTGGGTCTCATCAATT ACTGGTGGTGGTAATTATATAGAGTACGCAGACTCAGT GAAGGGCCGATTCACCATCTCCAGAGACAACGCCCAGA | 46 |
| | | GCTCACTTTATCTGCAAATGAACAGCCTGAGAGCCGAG GACACGGCTGTGTATTACTGTGCGAGAGATGATTATGG TTCGGGGAGTTATTCCAACTGGTTCGACCCCTGGGGCC AGGGAACCCTGGTCACCGTCTCCTCA | |
| | Light chain | CAGTCTGTGCTGACGCAGCCGCCCTCAGTGTCTGGGGC CCCAGGGCAGAGGGTCACCATCTCCTGCACTGGGAGCA GCTCCAACATCGGGGCAGGTTATGATGTACACTGGTAC CAGCAGCTTCCAGGAAGAGCCCCCAAACTCCTCATCTT TGCTAACACCAATCGGCCCTCAGGGGTCCCTGACCGAT TCTCTGGCTCCAAGTCTGGCGCCTCTGCCTCCCTGGCCA TCACTGGCCTCCAGGCTGACGATGAGGCTGATTATTAC TGCCAGTCCTATGACCGCAGCCTGAGTCATGTCTTCGG AACTGGGACCAAGGTCACCGTCCTAGGC | 39 |
| 12m | Heavy chain | GAGGTGCAGCTGGTGGAGTCTGGGGGAGGCCTGGTCAA GCCTGGGGGGTCCCTGAGACTTTCCTGTGCTGGCTCTGG ATTCGCCTTCAGTAGTTACACTATGCACTGGGTGCGCCA GGCTCCAGGGAAGGGGCTGGAGTGGGTCTCATCCATCA CTGGCGGCAGTAGTTACCTAGACTACGCACACTCAGTG AAGGGCCGATTCACCATCTCCAGAGACAATGGCCAGAA CTCACTGTTTCTGCAAATGAACAGCCTGAGGACCGAGG ACACGGCTGTATATTACTGTGCGAGAGATGACTATGGT TCGGGGAGTTATTCCAACTACTTCGACCCCTGGGGCCA GGGAACCCTGGTCACCGTCTCCTCA | 47 |
| | Light chain | CAGTCTGTGCTGACGCAGCCGCCCTCAGTGTCTGGGGC CCCAGGGCAGAGGGTCACCATCTCCTGCACTGGGAGCA GCTCCAACATCGGGGCAGGTTATGATGTACACTGGTAC CAGCAGGTTCCAGGAACGGCCCCCAAACTCCTCATCTT TGGTAGCACCAATCGGCCCTCAGGGGTCCCTGACCGAT TCTCTGGCTCCAAGTCTGGCGCCTCAGCCTCCCTGGCCA TCACTGGGCTCCAGACTGAGGATGAGGCTGATTATTAC TGCCAGTCCTATGACCGCAGCCTGAGTCATGTCTTCGG AACTGGGACCAAGGTCACCGTCCTAGGC | 48 |

## Claims

1. An antibody or an antigen-binding fragment thereof specifically binding to respiratory syncytial virus (RSV) selected from the group consisting of the following (i) to (vii):
(i) an antibody specifically binding to RSV, comprising a heavy chain variable domain which comprises heavy chain CDR1 comprising the amino acid sequence of SEQ ID NO: 1, heavy chain CDR2 comprising the amino acid sequence of SEQ ID NO: 2, and heavy chain CDR3 comprising the amino acid sequence of SEQ ID NO: 3, and a light chain variable domain which comprises light chain CDR1 comprising the amino acid sequence of SEQ ID NO: 4, light chain CDR2 comprising the amino acid sequence of SEQ ID NO: 5, and light chain CDR3 comprising the amino acid sequence of SEQ ID NO: 6;
(ii) an antibody specifically binding to RSV, comprising a heavy chain variable domain which comprises heavy chain CDR1 comprising the amino acid sequence of SEQ ID NO: 1, heavy chain CDR2 comprising the amino acid sequence of SEQ ID NO: 2, and heavy chain CDR3 comprising the amino acid sequence of SEQ ID NO: 3, and a light chain variable domain which comprises light chain CDR1 comprising the amino acid sequence of SEQ ID NO: 7, light chain CDR2 comprising the amino acid sequence of SEQ ID NO: 5, and light chain CDR3 comprising the amino acid sequence of SEQ ID NO: 6;
(iii) an antibody specifically binding to RSV, comprising a heavy chain variable domain which comprises heavy chain CDR1 comprising the amino acid sequence of SEQ ID NO: 8, heavy chain CDR2 comprising the amino acid sequence of SEQ ID NO: 9, and heavy chain CDR3 comprising the amino acid sequence of SEQ ID NO: 10, and a light chain variable domain which comprises light chain CDR1 comprising the amino acid sequence of SEQ ID NO: 7, light chain CDR2 comprising the amino acid sequence of SEQ ID NO: 5, and light chain CDR3 comprising the amino acid sequence of SEQ ID NO: 6;
(iv) an antibody specifically binding to RSV, comprising a heavy chain variable domain which comprises heavy chain CDR1 comprising the amino acid sequence of SEQ ID NO: 11, heavy chain CDR2 comprising the amino acid sequence of SEQ ID NO: 12, and heavy chain CDR3 comprising the amino acid sequence of SEQ ID NO: 3, and a light chain variable domain which comprises light chain CDR1 comprising the amino acid sequence of SEQ ID NO: 13, light chain CDR2 comprising the amino acid sequence of SEQ ID NO: 5, and light chain CDR3 comprising the amino acid sequence of SEQ ID NO: 6;
(v) an antibody specifically binding to RSV, comprising a heavy chain variable domain which comprises heavy chain CDR1 comprising the amino acid sequence of SEQ ID NO: 1, heavy chain CDR2 comprising the amino acid sequence of SEQ ID NO: 2, and heavy chain CDR3 comprising the amino acid sequence of SEQ ID NO: 3, and a light chain variable domain which comprises light chain CDR1 comprising the amino acid sequence of SEQ ID NO: 14, light chain CDR2 comprising the amino acid sequence of SEQ ID NO: 15, and light chain CDR3 comprising the amino acid sequence of SEQ ID NO: 6;
(vi) an antibody specifically binding to RSV, comprising a heavy chain variable domain which comprises heavy chain CDR1 comprising the amino acid sequence of SEQ ID NO: 8, heavy chain CDR2 comprising the amino acid sequence of SEQ ID NO: 9, and heavy chain CDR3 comprising the amino acid sequence of SEQ ID NO: 3, and a light chain variable domain which comprises light chain CDR1 comprising the amino acid sequence of SEQ ID NO: 14, light chain CDR2 comprising the amino acid sequence of SEQ ID NO: 5, and light chain CDR3 comprising the amino acid sequence of SEQ ID NO: 6; and
(vii) an antibody specifically binding to RSV, comprising a heavy chain variable domain which comprises heavy chain CDR1 comprising the amino acid sequence of SEQ ID NO: 1, heavy chain CDR2 comprising the amino acid sequence of SEQ ID NO: 16, and heavy chain CDR3 comprising the amino acid sequence of SEQ ID NO: 17, and a light chain variable domain which comprises light chain CDR1 comprising the amino acid sequence of SEQ ID NO: 14, light chain CDR2 comprising the amino acid sequence of SEQ ID NO: 18, and light chain CDR3 comprising the amino acid sequence of SEQ ID NO: 6.

2. The antibody or an antigen-binding fragment thereof of claim 1, wherein the antibody is an antibody specifically binding to respiratory syncytial virus (RSV) selected from the group consisting of the following (viii) to (xv):
(viii) an antibody specifically binding to RSV, comprising a heavy chain variable domain comprising the amino acid sequence of SEQ ID NO: 19 and a light chain variable domain comprising the amino acid sequence of SEQ ID NO: 20;
(ix) an antibody specifically binding to RSV, comprising a heavy chain variable domain comprising the amino acid sequence of SEQ ID NO: 21 and a light chain variable domain comprising the amino acid sequence of SEQ ID NO: 22;
(x) an antibody specifically binding to RSV, comprising a heavy chain variable domain comprising the amino acid sequence of SEQ ID NO: 23 and a light chain variable domain comprising the amino acid sequence of SEQ ID NO: 24;
(xi) an antibody specifically binding to RSV, comprising a heavy chain variable domain comprising the amino acid sequence of SEQ ID NO: 25 and a light chain variable domain comprising the amino acid sequence of SEQ ID NO: 26;
(xii) an antibody specifically binding to RSV, comprising a heavy chain variable domain comprising the amino acid sequence of SEQ ID NO: 27 and a light chain variable domain comprising the amino acid sequence of SEQ ID NO: 28;
(xiii) an antibody specifically binding to RSV, comprising a heavy chain variable domain comprising the amino acid sequence of SEQ ID NO: 29 and a light chain variable domain comprising the amino acid sequence of SEQ ID NO: 30;
(xiv) an antibody specifically binding to RSV, comprising a heavy chain variable domain comprising the amino acid sequence of SEQ ID NO: 31 and a light chain variable domain comprising the amino acid sequence of SEQ ID NO: 24; and
(xv) an antibody specifically binding to RSV, comprising a heavy chain variable domain comprising the amino acid sequence of SEQ ID NO: 32 and a light chain variable domain comprising the amino acid sequence of SEQ ID NO: 33.

3. The antibody or an antigen-binding fragment thereof of claim 1, wherein the antibody is an antibody specifically binding to respiratory syncytial virus (RSV), selected from the group consisting of the following (xvi) to (xxiii):
(xvi) an antibody specifically binding to RSV, comprising a heavy chain variable domain encoded by the nucleotide sequence of SEQ ID NO: 34 and a light chain variable domain encoded by the nucleotide sequence of SEQ ID NO: 35;
(xvii) an antibody specifically binding to RSV, comprising a heavy chain variable domain encoded by the nucleotide sequence of SEQ ID NO: 36 and a light chain variable domain encoded by the nucleotide sequence of SEQ ID NO: 37;
(xviii) an antibody specifically binding to RSV, comprising a heavy chain variable domain encoded by the nucleotide sequence of SEQ ID NO: 38 and a light chain variable domain encoded by the nucleotide sequence of SEQ ID NO: 39;
(xix) an antibody specifically binding to RSV, comprising a heavy chain variable domain encoded by the nucleotide sequence of SEQ ID NO: 40 and a light chain variable domain encoded by the nucleotide sequence of SEQ ID NO: 41;
(xx) an antibody specifically binding to RSV, comprising a heavy chain variable domain encoded by the nucleotide sequence of SEQ ID NO: 42 and a light chain variable domain encoded by the nucleotide sequence of SEQ ID NO: 43;
(xxi) an antibody specifically binding to RSV, comprising a heavy chain variable domain encoded by the nucleotide sequence of SEQ ID NO: 44 and a light chain variable domain encoded by the nucleotide sequence of SEQ ID NO: 45;
(xxii) an antibody specifically binding to RSV, comprising a heavy chain variable domain encoded by the nucleotide sequence of SEQ ID NO: 46 and a light chain variable domain encoded by the nucleotide sequence of SEQ ID NO: 39; and
(xxiii) an antibody specifically binding to RSV, comprising a heavy chain variable domain encoded by the nucleotide sequence of SEQ ID NO: 47 and a light chain variable domain encoded by the nucleotide sequence of SEQ ID NO: 48.

4. The antibody or an antigen-binding fragment thereof of any one of claims 1 to 3, wherein the antibody specifically binds to the F-protein of RSV.

5. The antibody or an antigen-binding fragment thereof of any one of claims 1 to 3, wherein the antibody is a humanized antibody.

6. The antibody or an antigen-binding fragment thereof of any one of claims 1 to 3, wherein the antibody is a neutralizing antibody.

7. The antibody or an antigen-binding fragment thereof of any one of claims 1 to 3, wherein the antigen-binding fragment of the antibody is a Fab fragment, a Fab' fragment, a F(ab')₂ fragment, or a scFv fragment.

8. A polynucleotide encoding a light chain variable domain and a light chain variable domain of the antibody specifically bind to respiratory syncytial virus (RSV) according to any one of claims 1 to 3.

9. An expression vector comprising the polynucleotide of claim 8.

10. A host cell transformed with the expression vector of claim 9.

11. A method of preparing an antibody or an antigen-binding fragment thereof specifically binding to respiratory syncytial virus (RSV), comprising the step of culturing the host cell of claim 10.

12. A pharmaceutical composition for use in preventing or treating respiratory syncytial virus (RSV) infection, comprising the antibody or antigen-binding fragment thereof of any one of claims 1 to 3.
